(19) Europäisches Patentamt
European Patent Office
Office européen des brevets

(11) EP 2 406 228 B1

(12) **EUROPÄISCHE PATENTSCHRIFT**

(45) Veröffentlichungstag und Bekanntmachung des Hinweises auf die Patenterteilung:
**18.09.2013 Patentblatt 2013/38**

(21) Anmeldenummer: **10708503.7**

(22) Anmeldetag: **09.03.2010**

(51) Int Cl.:
***C07D 215/38*** (2006.01) ***C07D 409/12*** (2006.01)
***A61K 31/4709*** (2006.01) ***A61K 31/47*** (2006.01)
***A61P 25/00*** (2006.01)

(86) Internationale Anmeldenummer:
**PCT/EP2010/001449**

(87) Internationale Veröffentlichungsnummer:
**WO 2010/102779 (16.09.2010 Gazette 2010/37)**

(54) **SUBSTITUIERTE 3-AMINO-2-MERCAPTOCHINOLINE ALS KCNQ2/3 MODULATOREN**

Substituted 3-amino-2-mercaptochinolins as KCNQ2/3 modulators

3-amino-2-mercaptochinolines substituées en tant que modulateurs de KCNQ2/3

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **10.03.2009 EP 09003431**

(43) Veröffentlichungstag der Anmeldung:
**18.01.2012 Patentblatt 2012/03**

(73) Patentinhaber: **Grünenthal GmbH
52078 Aachen (DE)**

(72) Erfinder:
- **KÜHNERT, Sven
52355 Düren (DE)**
- **BAHRENBERG, Gregor
52156 Monschau-Konzen (DE)**
- **SCHRÖDER, Wolfgang
52074 Aachen (DE)**

(56) Entgegenhaltungen:
**WO-A-2007/057447**

- **KWANG HO YOO, EUN BOK CHOI, HYEON KYU LEE, GUY HWAN YEON, HEE CHEOL YANG, CHWANG SIEK PAK: "Beckmann Rearrangement Using Indium(III) Chloride: Synthesis of Substituted Oxazoloquinolines from the Corresponding Ketoximes of 3-Acyl-1H-quinolin-4-ones" SYNTHESIS, Nr. 10, 27. April 2006 (2006-04-27), Seiten 1599-1612, XP002580722 DOI: 10.1055/s-2006-926463**
- **WERMUTH C G: "MOLECULAR VARIATIONS BASED ON ISOSTERIC REPLACEMENTS" PRACTICE OF MEDICINAL CHEMISTRY, XX, XX, 1. Januar 1996 (1996-01-01), Seiten 203-237, XP002190259**

EP 2 406 228 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft substituierte 3- Amino- 2- mercaptochinoline, Verfahren zu ihrer Herstellung, Arzneimittel enthaltend diese Verbindungen sowie die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Ein pathophysiologisches Merkmal von chronischen Schmerzen besteht in der Übererregbarkeit von Neuronen. Die neuronale Erregbarkeit wird entscheidend von der Aktivität von $K^+$ Kanälen beeinflusst, da diese das Ruhemembranpotential der Zelle und somit die Erregbarkeitsschwelle maßgeblich bestimmen. Heteromere $K^+$ Kanäle vom molekularen Subtyp KCNQ2/3 (Kv7.2/7.3) sind in Neuronen verschiedener Regionen des zentralen (Hippocampus, Amygdala) und peripheren (Hinterwurzelganglien) Nervensystems exprimiert und regulieren deren Erregbarkeit. Die Aktivierung von KCNQ2/3 $K^+$ Kanälen führt zu einer Hyperpolarisation der Zellmembran und damit einhergehend zu einer Abnahme der elektrischen Erregbarkeit dieser Neurone. KCNQ2/3 exprimierende Neurone der Hinterwurzelganglien sind an der Übertragung nociceptiver Erregungen von der Peripherie ins Rückenmark beteiligt (Passmore et al., J Neurosci. 2003; 23 (18) : 7227- 36) .

Dementsprechend konnte für den KCNQ2/3 Agonisten Retigabine eine analgetische Wirksamkeit in präklinischen Neuropathie- und Entzündungsschmerzmodellen nachgewiesen werden (Blackburn- Munro and Jensen, Eur J Pharmacol. 2003; 460 (2- 3) : 109- 16; Dost et al., Naunyn Schmiedebergs Arch Pharmacol 2004; 369 (4) : 382- 390) .

Der KCNQ2/3 $K^+$ Kanal stellt somit einen geeigneten Ansatzpunkt zur Behandlung von Schmerz; insbesondere von Schmerz ausgewählt aus der Gruppe bestehend aus chronischem Schmerz, neuropathischem Schmerz, inflammatorischem Schmerz und muskulärem Schmerz (Nielsen et al., Eur J Pharmacol. 2004; 487(1-3): 93-103), insbesondere von neuropathischem und inflammatorischem Schmerz dar.

Darüber hinaus ist der KCNQ2/3 $K^+$ Kanal ein geeignetes Target für die Therapie einer Vielzahl weiterer Erkrankungen wie beispielsweise Migräne (US2002/0128277) , kognitive Erkrankungen (Gribkoff, Expert Opin Ther Targets 2003; 7 (6) : 737- 748) , Angstzuständen (Korsgaard et al., J Pharmacol Exp Ther. 2005, 14 (1) : 282- 92) , Epilepsie (Wickenden et al., Expert Opin Ther Pat 2004, 14 (4) : 457- 469; Gribkoff, Expert Opin Ther Targets 2008, 12 (5) : 565- 81; Miceli et al., Curr Opin Pharmacol 2008, 8 (1) : 65- 74) , Harninkontinenz (Streng et al., J Urol 2004; 172: 2054- 2058) , Abhängigkeit (Hansen et al., Eur J Pharmacol 2007, 570 (1- 3) : 77- 88) , Manie/ bipolare Störungen (Dencker et al., Epilepsy Behav 2008, 12 (1) : 49- 53) , Dystonie- assoziierte Dyskinesien (Richter et al., Br J Pharmacol 2006, 149 (6) : 747- 53) .

Aus dem Stand der Technik sind substituierte Tetrahydropyrrolopyrazine bekannt, die eine Affinität zum KCNQ2/3 $K^+$ Kanal aufweisen (WO 2008/046582).

WO 2007/057 447 beschreibt Chinazolin derivate, die eine Affinität zum KCNQ2-, KCNQ3-, KCNQ4-, und/oder KCNQ5-$K^+$ Kanal aufweisen.

Es besteht ein Bedarf an weiteren Verbindungen mit vergleichbaren oder besseren Eigenschaften, nicht nur im Hinblick auf die Affinität an KCNQ2/3 als solche (*potency*, *efficacy*).

So kann es vorteilhaft sein, die metabolische Stabilität, die Löslichkeit in wässrigen Medien oder die Permeabilität der Verbindungen zu verbessern. Diese Faktoren können sich günstig auf die orale Bioverfügbarkeit auswirken oder können das- PK/PD (Pharmakokinetik/ Pharmakodynamik)- Profil verändern, was z.B. zu einer günstigeren Wirkdauer führen kann.

Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Ferner kann es von Vorteil sein, wenn die Verbindungen eine hohe Selektivität gegenüber anderen Rezeptoren der KCNQ- Familie zeigen (Spezifität) , beispielsweise gegenüber KCNQ1, KCNQ3/5 oder KCNQ4. Eine hohe Selektivität kann sich günstig auf das Nebenwirkungsprofil auswirken. So ist beispielsweise bekannt, dass Verbindungen, welche (auch) an KCNQ1 binden, ein hohes Risiko kardialer Nebenwirkungen mit sich bringen, weshalb eine hohe Selektivität gegenüber KCNQ1 wünschenswert sein kann. Eine hohe Selektivität kann jedoch auch gegenüber anderen Rezeptoren von Vorteil sein. Eine geringe Affinität zum hERG- Ionenkanal oder zum L- Typ Calcium- Ionenkanal (Phenylalkylamin-, Benzothiazepin-, Dihydropyridin- Bindungsstellen) kann vorteilhaft sein, da diese Rezeptoren in Zusammenhang mit dem Auftreten kardialer Nebenwirkungen gebracht werden. Insgesamt kann eine verbesserte Selektivität bezüglich der Bindung an andere endogene Proteine (d.h. z.B. Rezeptoren oder Enzyme) zu einer Verbesserung des Nebenwirkungsprofils, und dadurch zu einer verbesserten Verträglichkeit führen.

Eine Aufgabe der Erfindung bestand daher darin, neue Verbindungen zur Verfügung zu stellen, welche Vorteile gegenüber den Verbindungen des Standes der Technik aufweisen. Die Verbindungen sollten sich insbesondere als pharmakologische Wirkstoffe in Arzneimitteln eignen, vorzugsweise in Arzneimitteln zur Behandlung von Störungen oder Krankheiten, welche zumindest teilweise durch KCNQ2/3 $K^+$ Kanäle vermittelt werden.

Diese Aufgabe wird durch den Gegenstand der Patentansprüche gelöst.

Es wurde überraschend gefunden, dass sich substituierte 3- Amino- 2- mercaptochinoline der nachstehend angegebenen allgemeinen Formel (1) zur Behandlung von Schmerzen eignen. Es wurde ferner überraschend gefunden, dass substituierte 3- Amino- 2- mercaptochinoline der nachstehend angegebenen allgemeinen Formel (1) auch eine ausgezeichnete Affinität zum KCNQ2/3 $K^+$ Kanal aufweisen und sich daher zur Behandlung von Störungen oder Krankheiten eignen, die zumindest teilweise durch KCNQ2/3 $K^+$ Kanäle vermittelt werden. Dabei wirken die substituierten 3-Amino- 2- mercaptochinoline als Modulatoren, d.h. Agonisten oder Antagonisten, des KCNQ2/3 $K^+$ Kanals.

Ein Gegenstand der Erfindung sind substituierte 3- Amino- 2- mercaptochinoline der allgemeinen Formel (1)

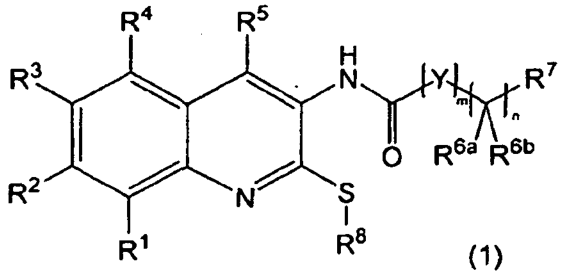

(1) ,

worin

m für 0 oder 1 und

n für eine ganze Zahl von 0 bis 4 steht,

$R^1$, $R^2$, $R^3$, $R^4$, $R^{6a}$, $R^{6b}$ jeweils unabhängig voneinander für H; F; Cl; Br; I; $NO_2$; $CF_3$; CN; OH; $OCF_3$; SH; $SCF_3$; $NH_2$; $C_{1-10}$- Alkyl, O- $C_{1-10}$- Alkyl, O- C (=O)- $C_{1-10}$- Alkyl, S- $C_{1-10}$- Alkyl, NH ($C_{1-10}$- Alkyl) , N ($C_{1-10}$- Alkyl)$_2$, NH-C (=O)- $C_{1-10}$- Alkyl, N (C (=O)- $C_{1-10}$- Alkyl)$_2$ oder C (=O)- $C_{1-10}$- Alkyl, jeweils gesättigt oder ungesättigt, verzweigt oder unverzweigt, unsubstituiert oder einfach oder mehrfach substituiert; $C_{3-8}$- Cycloalkyl oder- Heterocyclyl, jeweils gesättigt oder ungesättigt, unsubstituiert oder einfach oder mehrfach substituiert; stehen,

$R^5$ für H; F; Cl; Br; I; $NO_2$; $CF_3$; CN; $OCF_3$; SH; $SCF_3$; $NH_2$; $C_{1-10}$- Alkyl, O- $C_{1-10}$- Alkyl, O- C (=O)- $C_{1-10}$- Alkyl, S-$C_{1-10}$- Alkyl, NH ($C_{1-10}$- Alkyl) , N ($C_{1-10}$- Alkyl)$_2$, NH- C (=O)- $C_{1-10}$- Alkyl, N (C (=O)- $C_{1-10}$- Alkyl)$_2$ oder C (=O)-$C_{1-10}$- Alkyl, jeweils gesättigt oder ungesättigt, verzweigt oder unverzweigt, unsubstituiert oder einfach oder mehrfach substituiert; $C_{3-8}$- Cycloalkyl oder- Heterocyclyl, jeweils gesättigt oder ungesättigt, unsubstituiert oder einfach oder mehrfach substituiert; steht,

Y für O oder $NR^9$ steht, wobei $R^9$ für H oder $C_{1-4}$-Alkyl gesättigt oder ungesättigt, verzweigt oder unverzweigt, unsubstituiert oder einfach oder mehrfach substituiert steht,

$R^7$ für $C_{1-10}$- Alkyl oder $C_{2-10}$-Heteroalkyl, gesättigt oder ungesättigt, verzweigt oder unverzweigt, unsubstituiert oder einfach oder mehrfach substituiert; $C_{3-10}$- Cycloalkyl oder- Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert steht;
mit der Maßgabe, dass, wenn $R^7$ Heterocyclyl bedeutet, die Bindung des Heterocyclyls an die übergeordnete allgemeine Struktur über ein Kohlenstoffatom des Heterocyclyls erfolgen kann, bevorzugt erfolgt die Bindung des Heterocyclyls an die übergeordnete allgemeine Struktur über ein Kohlenstoffatom des Heterocyclyls; und
mit der Maßgabe, dass, wenn $R^7$ Aryl oder Heteroaryl bedeutet, die Summe von n und m größer oder gleich 1 beträgt;

$R^8$ ausgewählt ist aus der Gruppe bestehend aus $C_{1-10}$- Alkyl oder $C_{2-10}$- Heteroalkyl, jeweils gesättigt oder ungesättigt, verzweigt oder unverzweigt, unsubstituiert oder einfach oder mehrfach substituiert; $C_{3-10}$- Cycloalkyl oder-Heterocyclyl, jeweils gesättigt oder ungesättigt, unsubstituiert oder einfach oder mehrfach substituiert; Aryl oder

Heteroaryl, jeweils unsubstituiert oder einfach oder mehrfach substituiert; über $C_{1-8}$- Alkyl oder $C_{2-8}$- Heteroalkyl verbrücktes $C_{3-10}$- Cycloalkyl oder- Heterocyclyl, jeweils gesättigt oder ungesättigt, unsubstituiert oder einfach oder mehrfach substituiert; oder über $C_{1-8}$- Alkyl oder $C_{2-8}$- Heteroalkyl verbrücktes Aryl oder Heteroaryl, jeweils unsubstituiert oder einfach oder mehrfach substituiert, wobei die Alkyl- oder Heteroalkylkette jeweils verzweigt oder unverzweigt, gesättigt oder ungesättigt, unsubstituiert sein kann;

worin "Alkyl substituiert", "Heteroalkyl substituiert", "Heterocyclyl substituiert" und "Cycloalkyl substituiert" für die Substitution eines oder mehrerer Wasserstoffatome jeweils unabhängig voneinander durch F; Cl; Br; I; CN; $CF_3$; =O; =NH; =C $(NH_2)_2$; $NO_2$; $R^0$; C (=O) H; C (=O) $R^0$ $CO_2H$; C (=O) $OR^0$; $CONH_2$; C (=O) $NHR^0$; C (=O) N $(R^0)_2$; OH; $OR^0$;- O- ($C_{1-8}$- Alkyl)- O- ; O- C (=O)- $R^0$; O- C (=O)- O- $R^0$; O- (C=O)- NH- $R^0$; O- C (=O)- N $(R^0)_2$; O- S $(=O)_2$- $R^0$; O- S $(=O)_2OH$; O- S $(=O)_2OR^0$; O- S $(=O)_2NH_2$; O- S $(=O)_2NHR^0$; O- S $(=O)_2N$ $(R^0)_2$; $NH_2$; NH- $R^0$; N $(R^0)_2$; NH- C (=O)- $R^0$; NH- C (=O)- O- $R^0$; NH- C (=O)- $NH_2$; NH- C (=O)- NH- $R^0$; NH- C (=O)- N $(R^0)_2$; $NR^0$- C (=O)- $R^0$; $NR^0$- C (=O)- O- $R^0$; $NR^0$- C (=O)- $NH_2$; $NR^0$- C (=O)- NH- $R^0$; $NR^0$- C (=O)- N $(R^0)_2$; NH- S $(=O)_2OH$; NH- S $(=O)_2R^0$; NH- S $(=O)_2OR^0$; NH- S $(=O)_2NH_2$; NH- S $(=O)_2NHR^0$; NH- S $(=O)_2N$ $(R^0)_2$; $NR^0$- S $(=O)_2OH$; $NR^0$- S $(=O)_2R^0$; $NR^0$- S $(=O)_2OR^0$; $NR^0$- S $(=O)_2NH_2$; $NR^0$- S $(=O)_2NHR^0$; $NR^0$- S $(=O)_2N$ $(R^0)_2$; SH; $SR^0$; S (=O) $R^0$; S $(=O)_2R^0$; S $(=O)_2H$; S $(=O)_2OH$; S $(=O)_2OR^0$; S $(=O)_2NH_2$; S $(=O)_2NHR^0$; S $(=O)_2N$ $(R^0)_2$; steht;

worin "Aryl substituiert" und "Heteroaryl substituiert" für die Substitution eines oder mehrerer Wasserstoffatome jeweils unabhängig voneinander durch F; Cl; Br; I; $NO_2$; $CF_3$; CN; $R^0$; C (=O) H; C (=O) $R^0$; $CO_2H$; C (=O) $OR^0$; $CONH_2$; C (=O) $NHR^0$; C (=O) N $(R^0)_2$ OH; $OR^0$;- O- ($C_{1-8}$- Alkyl)- O- ; O- C (=O)- $R^0$; O- C (=O)- O- $R^0$; O- (C=O)- NH- $R^0$; O- C (=O)- N $(R^0)_2$; O- S $(=O)_2$- $R^0$; O- S $(=O)_2OH$; O- S $(=O)_2OR^0$; O- S $(=O)_2NH_2$; 0- S $(=O)_2NHR^0$; O- S $(=O)_2N$ $(R^0)_2$; $NH_2$; NH- $R^0$; N $(R^0)_2$; NH- C (=O)- $R^0$; NH- C (=O)- O- $R^0$; NH- C (=O)- $NH_2$; NH- C (=O)- NH- $R^0$; NH- C (=O)- N $(R^0)_2$; $NR^0$- C (=O)- $R^0$; $NR^0$- C (=O)- O- $R^0$; $NR^0$- C (=O)- $NH_2$; $NR^0$- C (=O)- NH- $R^0$; $NR^0$- C (=O)- N $(R^0)_2$; NH- S $(=O)_2OH$; NH- S $(=O)_2R^0$; NH- S $(=O)_2OR^0$; NH- S $(=O)_2NH_2$; NH- S $(=O)_2NHR^0$; NH- S $(=O)_2N$ $(R^0)_2$; $NR^0$- S $(=O)_2OH$; $NR^0$- S $(=O)_2R^0$; $NR^0$- S $(=O)_2OR^0$; $NR^0$- S $(=O)_2NH_2$; $NR^0$- S $(=O)_2NHR^0$; $NR^0$- S $(=O)_2N$ $(R^0)_2$ SH; $SR^0$ S (=O) $R^0$; S $(=O)_2R^0$; S $(=O)_2OH$; S $(=O)_2OR^0$; S $(=O)_2NH_2$; S $(=O)_2NHR^0$; S $(=O)_2N$ $(R^0)_2$; steht; und

$R^0$ für $C_{1-10}$- Alkyl oder $C_{2-10}$- Heteroalkyl, jeweils gesättigt oder ungesättigt, verzweigt oder unverzweigt, unsubstituiert oder einfach oder mehrfach substituiert; $C_{3-10}$- Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$- Alkyl oder $C_{2-8}$- Heteroalkyl verbrücktes $C_{3-10}$- Cycloalkyl oder Heterocyclyl, jeweils gesättigt oder ungesättigt, unsubstituiert oder einfach oder mehrfach substituiert, wobei die Alkyl- oder Heteroalkylkette jeweils verzweigt oder unverzweigt, gesättigt oder ungesättigt, unsubstituiert, einfach oder mehrfach substituiert sein kann; oder über $C_{1-8}$- Alkyl oder $C_{2-8}$- Heteroalkyl verbrücktes Aryl oder Heteroaryl, jeweils unsubstituiert oder einfach oder mehrfach substituiert, wobei die Alkyl- oder Heteroalkylkette jeweils verzweigt oder unverzweigt, gesättigt oder ungesättigt, unsubstituiert, einfach oder mehrfach substituiert sein kann; steht;

in Form der freien Verbindungen oder Salze physiologisch verträglicher Säuren oder Basen.

Die Ausdrücke "Alkyl" bzw. "$C_{1-10}$- Alkyl", "$C_{1-8}$- Alkyl", "$C_{1-4}$- Alkyl" und "$C_{2-10}$- Alkyl" umfassen im Sinne dieser Erfindung acyclische gesättigte oder ungesättigte aliphatische Kohlenwasserstoffreste, die verzweigt oder unverzweigt sowie unsubstituiert oder ein- oder mehrfach substituiert sein können, mit 1 bis 10 bzw. 1 bis 8 bzw. 1 bis 4 bzw. 2 bis 10 C- Atomen, d.h. $C_{1-10}$- Alkanyle, $C_{2-10}$- Alkenyle und $C_{2-10}$- Alkinyle bzw. $C_{1-8}$- Alkanyle, $C_{2-8}$- Alkenyle und $C_{2-8}$- Alkinyle bzw. $C_{1-4}$- Alkanyle, $C_{2-4}$- Alkenyle und $C_{2-4}$- Alkinyle bzw. $C_{2-10}$- Alkanyle, $C_{2-10}$- Alkenyle und $C_{2-10}$- Alkinyle Dabei weisen Alkenyle mindestens eine C- C- Doppelbindung und Alkinyle mindestens eine C- C- Dreifachbindung auf. Bevorzugt ist Alkyl aus der Gruppe ausgewählt, die Methyl, Ethyl, n- Propyl, 2- Propyl, n- Butyl, iso- Butyl, sec.- Butyl, tert.- Butyl, n- Pentyl, iso- Pentyl, neo- Pentyl, n- Hexyl, n- Heptyl, n- Octyl, n- Nonyl, n- Decyl, Ethenyl (Vinyl) , Ethinyl, Propenyl (- $CH_2CH=CH_2$, -CH=CH- $CH_3$, -C $(=CH_2)$- $CH_3$) , Propinyl (- CH- C=CH, -C=C- $CH_3$) , Butenyl, Butinyl, Pentenyl, Pentinyl, Hexenyl und Hexinyl, Heptenyl, Heptinyl, Octenyl, Octinyl, Nonenyl, Noninyl, Decenyl und Decinyl umfasst.

Die Ausdrücke "Heteroalkyl" bzw. "$C_{2-10}$- Heteroalkyl", "$C_{2-8}$- Heteroalkyl" und "$C_{2-4}$- Heteroalkyl" umfassen im Sinne dieser Erfindung acyclische aliphatische gesättigte oder ungesättigte Kohlenwasserstoffreste mit 2 bis 10 C- Atomen, d.h. $C_{2-10}$- Heteroalkanyle, $C_{2-10}$- Heteroalkenyle und $C_{2-10}$- Heteroalkinyle bzw. mit 2 bis 8 C- Atomen, d.h. $C_{2-8}$- Heteroalkanyle, $C_{2-8}$- Heteroalkenyle und $C_{2-8}$- Heteroalkinyle bzw. mit 2 bis 4 C- Atomen, d.h. $C_{2-4}$- Heteroalkanyle, $C_{2-4}$- Heteroalkenyle und $C_{2-4}$- Heteroalkinyle, die jeweils verzweigt oder unverzweigt sowie unsubstituiert oder ein- oder mehrfach substituiert sein können und in denen mindestens ein, gegebenenfalls auch zwei oder drei Kohlenstoffatome durch ein Heteroatom oder eine Heteroatomgruppe jeweils unabhängig voneinander ausgewählt aus der Gruppe bestehend aus O, S, S (=O) , S $(=O)_2$, N, NH, und N ($C_»$- Alkyl) , vorzugsweise N $(CH_3)$ ersetzt sind, wobei das

anfangsständige Kohlenstoffatom eines $C_{2-10}$- Heteroalkyls oder eines $C_{2-8}$- Heteroalkyls oder eines $C_{2-4}$- Heteroalkyls, über welches das $C_{2-10}$- Heteroalkyl oder das $C_{2-8}$- Heteroalkyl oder das $C_{2-4}$- Heteroalkyl an die jeweilige übergeordnete allgemeine Struktur gebunden wird, nicht durch ein Heteroatom oder eine Heteroatomgruppe ersetzt werden kann und einander benachbarte Kohlenstoffatome nicht gleichzeitig durch ein Heteroatom oder eine Heteroatomgruppe ersetzt werden können. Gegebenenfalls können auch die Heteroatomgruppen NH und N ($C_{1-8}$- Alkyl) des Heteroalkyls einfach oder mehrfach substituiert sein. $C_{2-10}$- Heteroalkenyle, $C_{2-8}$- Heteroalkenyle und $C_{2-4}$- Heteroalkenyle weisen mindestens eine C- C- oder eine C- N- Doppelbindung und $C_{2-10}$- Heteroalkinyle, $C_{2-8}$- Heteroalkinyle und $C_{2-4}$- Heteroalkinyle mindestens eine C- C- Dreifachbindung auf. Bevorzugt ist Heteroalkyl aus der Gruppe ausgewählt, die- $CH_2$- O- $CH_3$, -$CH_2$- $CH_2$- O- $CH_3$, -$CH_2$- $CH_2$- O- $CH_2$- $CH_3$, -$CH_2$- $CH_2$- O- $CH_2$- $CH_2$- O- $CH_3$, -CH=CH- O- $CH_3$, -CH=CH- O- $CH_2$- $CH_3$, =CH- O- $CH_3$, =CH- O- $CH_2$- $CH_3$, =CH- $CH_2$- O- $CH_2$- $CH_3$, =CH- $CH_2$- O- $CH_3$, -$CH_2$- NH- $CH_3$, -$CH_2$- $CH_2$- NH- $CH_3$, -$CH_2$- $CH_2$- NH- $CH_2$- $CH_3$, -$CH_2$- $CH_2$- NH- $CH_2$- $CH_2$- NH- $CH_3$, -CH=CH- NH- $CH_3$, -CH=CH- NH- $CH_2$- $CH_3$, -CH=CH- N ($CH_3$)- $CH_2$- $CH_3$, =CH- NH- $CH_3$, =CH- NH- $CH_2$- $CH_3$, =CH- $CH_2$- NH- $CH_2$- $CH_3$, =CH- $CH_2$- NH- $CH_3$, -$CH_2$- N ($CH_3$)- $CH_3$, -$CH_2$- $CH_2$- N ($CH_3$)- $CH_3$, -$CH_2$- $CH_2$- N ($CH_3$)- $CH_2$- $CH_3$, -$CH_2$- $CH_2$- N ($CH_3$)- $CH_2$- $CH_2$- N ($CH_3$)- $CH_3$, $CH_2$- $CH_2$- NH- $CH_2$- $CH_2$- O- $CH_3$, $CH_2$- $CH_2$- O- $CH_2$- $CH_2$- NH- $CH_3$, $CH_2$- $CH_2$- N ($CH_3$)- $CH_2$- $CH_2$- O- $CH_3$, $CH_2$- $CH_2$- O- $CH_2$- $CH_2$- N ($CH_3$)- $CH_3$, $CH_2$- NH- $CH_2$- O- $CH_3$, $CH_2$- O- $CH_2$- NH- $CH_3$, $CH_2$- N ($CH_3$)- $CH_2$- O- $CH_3$, $CH_2$- O- $CH_2$- N ($CH_3$)- $CH_3$, -CH=CH- N ($CH_3$)- $CH_3$, =CH- N ($CH_3$)- $CH_3$, =CH- N ($CH_3$)- $CH_2$- $CH_3$, =CH- $CH_2$- N ($CH_3$)- $CH_2$- $CH_3$, =CH- $CH_2$- N ($CH_3$)- $CH_3$, -$CH_2$- $CH_2$=N ($CH_3$) und- $CH_2$=N ($CH_3$) umfasst.

Der Ausdruck "Cycloalkyl" oder "$C_{3-10}$- Cycloalkyl" bedeutet für die Zwecke dieser Erfindung cyclische aliphatische Kohlenwasserstoffe mit 3, 4, 5, 6, 7, 8, 9 oder 10 Kohlenstoffatomen, wobei die Kohlenwasserstoffe gesättigt oder ungesättigt (aber nicht aromatisch) , unsubstituiert oder ein- oder mehrfach substituiert sein können. Die Bindung des Cycloalkyls an die jeweilige übergeordnete allgemeine Struktur kann über jedes beliebige und mögliche Ringglied des Cycloalkyl- Restes erfolgen. Die Cycloalkyl- Reste können auch mit weiteren gesättigten, (partiell) ungesättigten, (hetero) cyclischen, aromatischen oder heteroaromatischen Ringsystemen , d.h. mit Cycloalkyl, Heterocyclyl, Aryl oder Heteroaryl kondensiert sein, die wiederum unsubstituiert oder einfach oder mehrfach substituiert sein können. Die Cycloalkyl- Reste können weiterhin einfach oder mehrfach verbrückt sein wie bspw. im Fall von Adamantyl, Bicyclo [2.2.1] heptyl oder Bicyclo [2.2.2] octyl. Bevorzugt ist Cycloalkyl aus der Gruppe ausgewählt, die Cyclopropyl, Cyclobutyl, Cyclopentyl, Cyclohexyl, Cycloheptyl, Cyclooctyl, Cyclononyl, Cyclodecyl, Adamantyl,

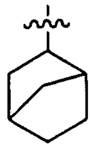 , 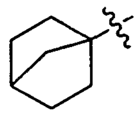 , 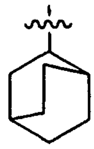 , 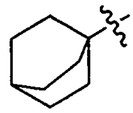 ,

Cyclopentenyl, Cyclohexenyl, Cycloheptenyl und Cyclooctenyl umfasst.

Der Begriff "Heterocyclyl" oder "Heterocycloalkyl" umfasst aliphatische gesättigte oder ungesättigte (aber nicht aromatische) Cycloalkyle mit drei bis zehn, d.h. 3, 4, 5, 6, 7, 8, 9 oder 10 Ringgliedern, in denen mindestens ein, ggf. auch zwei oder drei Kohlenstoffatome durch ein Heteroatom oder eine Heteroatomgruppe jeweils unabhängig voneinander ausgewählt aus der Gruppe bestehend aus O, S, N, NH und N ($C_{1-8}$- Alkyl) , vorzugsweise N ($CH_3$) ersetzt sind, wobei die Ringglieder unsubstituiert oder ein- oder mehrfach substituiert sein können. Die Bindung des Heterocyclyls an die übergeordnete allgemeine Struktur kann über jedes beliebige und mögliche Ringglied des Heterocyclyl- Restes erfolgen. Die Heterocyclyl- Reste können auch mit weiteren gesättigten, (partiell) ungesättigten (hetero) cyclischen oder aromatischen oder heteroaromatischen Ringsystemen, d.h. mit Cycloalkyl, Heterocyclyl, Aryl oder Heteroaryl kondensiert sein, die wiederum unsubstituiert oder einfach oder mehrfach substituiert sein können. Bevorzugt sind Heterocyclyl-Reste aus der Gruppe umfassend Azetidinyl, Aziridinyl, Azepanyl, Azocanyl, Diazepanyl, Dithiolanyl, Dihydrochinolinyl, Dihydropyrrolyl, Dioxanyl, Dioxolanyl, Dihydroindenyl Dihydropyridinyl, Dihydrofuranyl, Dihydroisochinolinyl, Dihydroindolinyl, Dihydroisoindolyl, Imidazolidinyl, Isoxazolidinyl, Morpholinyl, Oxiranyl, Oxetanyl, Pyrrolidinyl, Piperazinyl, Piperidinyl, Pyrazolidinyl, Pyranyl, Tetrahydropyrrolyl, Tetrahydropyranyl, Tetrahydrochinolinyl, Tetrahydroisochinolinyl, Tetrahydroindolinyl, Tetrahydrofuranyl, Tetrahydropyridinyl, Tetrahydrothiophenyl, Tetrahydropyridoindolyl, Tetrahydronaphthyl, Tetrahydrocarbolinyl, Tetrahydroisoxazolopyridinyl, Thiazolidinyl und Thiomorpholinyl.

Der Begriff "Aryl" bedeutet im Sinne dieser Erfindung aromatische Kohlenwasserstoffe mit bis zu 14 Ringgliedern, u.a. Phenyle und Naphthyle. Jeder Aryl- Rest kann unsubstituiert oder einfach oder mehrfach substituiert vorliegen, wobei die ArylSubstituenten gleich oder verschieden und in jeder beliebigen und möglichen Position des Aryls sein können. Die Bindung des Aryls an die übergeordnete allgemeine Struktur kann über jedes beliebige und mögliche Ringglied des ArylRestes erfolgen. Die Aryl- Reste können auch mit weiteren gesättigten, (partiell) ungesättigten, (hetero) cyclischen, aromatischen oder heteroaromatischen Ringsystemen kondensiert sein, d.h. mit Cycloalkyl, Heterocyclyl, Aryl oder Heteroaryl, die wiederum unsubstituiert oder einfach oder mehrfach substituiert sein können. Bevorzugte

kondensierte Aryl- Reste sind Benzodioxolanyl und Benzodioxanyl. Bevorzugt ist Aryl aus der Gruppe ausgewählt, die Phenyl, 1- Naphthyl und 2- Naphthyl enthält, welche jeweils unsubstituiert oder ein- oder mehrfach substituiert sein können. Ein besonders bevorzugtes Aryl ist Phenyl, unsubstituiert oder einfach oder mehrfach substituiert.

Der Begriff "Heteroaryl" steht für einen 5- oder 6- gliedrigen cyclischen aromatischen Rest, der mindestens 1, ggf. auch 2, 3, 4 oder 5 Heteroatome, enthält, wobei die Heteroatome jeweils unabhängig voneinander ausgewählt sind aus der Gruppe S, N und O und der Heteroaryl- Rest unsubstituiert oder ein- oder mehrfach substituiert sein kann; im Falle der Substitution am Heteroaryl können die Substituenten gleich oder verschieden sein und in jeder beliebigen und möglichen Position des Heteroaryls sein. Die Bindung an die übergeordnete allgemeine Struktur kann über jedes beliebige und mögliche Ringglied des Heteroaryl- Restes erfolgen. Das Heteroaryl kann auch Teil eines bi- oder polycyclischen Systems mit bis zu 14 Ringgliedern sein, wobei das Ringsystem mit weiteren gesättigten, (partiell) ungesättigten, (hetero) cyclischen oder aromatischen oder heteroaromatischen Ringen gebildet werden kann, d.h. mit Cycloalkyl, Heterocyclyl, Aryl oder Heteroaryl, die wiederum unsubstituiert oder einfach oder mehrfach substituiert sein können. Es ist bevorzugt, dass der Heteroaryl- Rest ausgewählt ist aus der Gruppe, die Benzofuranyl, Benzoimidazolyl, Benzothienyl, Benzothiadiazolyl, Benzothiazolyl, Benzotriazolyl, Benzooxazolyl, Benzooxadiazolyl, Chinazolinyl, Chinoxalinyl, Carbazolyl, Chinolinyl, Dibenzofuranyl, Dibenzothienyl, Furyl (Furanyl) , Imidazolyl, Imidazothiazolyl, Indazolyl, Indolizinyl, Indolyl, Isochinolinyl, Isoxazoyl, Isothiazolyl, Indolyl, Naphthyridinyl, Oxazolyl, Oxadiazolyl, Phenazinyl, Phenothiazinyl, Phtalazinyl, Pyrazolyl, Pyridyl (2- Pyridyl, 3- Pyridyl, 4- Pyridyl) , Pyrrolyl, Pyridazinyl, Pyrimidinyl, Pyrazinyl, Purinyl, Phenazinyl, Thienyl (Thiophenyl) , Triazolyl, Tetrazolyl, Thiazolyl, Thiadiazolyl oder Triazinyl umfasst. Besonders bevorzugt sind Furyl, Pyridyl und Thienyl.

Die Ausdrücke "über $C_{1-4}$- Alkyl oder $C_{1-8}$- Alkyl verbrücktes Aryl, Heteroaryl, Heterocyclyl oder Cycloalkyl" bedeuten im Sinne der Erfindung, dass $C_{1-4}$- Alkyl oder $C_{1-8}$- Alkyl und Aryl bzw. Heteroaryl bzw. Heterocyclyl bzw. Cycloalkyl die oben definierten Bedeutungen haben und der Aryl- bzw. Heteroaryl- bzw. Heterocyclyl- bzw. Cycloalkyl- Rest über eine $C_{1-4}$- Alkyl- oder eine $C_{1-8}$- Alkyl- Gruppe an die jeweilige übergeordnete allgemeine Struktur gebunden ist. Die Alkylkette kann in allen Fällen gesättigt oder ungesättigt, verzweigt oder unverzweigt, unsubstituiert oder einfach oder mehrfach substituiert sein. Vorzugsweise sind $C_{1-4}$- Alkyl oder $C_{1-8}$- Alkyl ausgewählt aus der Gruppe umfassend- $CH_2$-, -$CH_2$- $CH_2$-, -CH ($CH_3$)-, -$CH_2$- $CH_2$- $CH_2$-, -CH ($CH_3$)- $CH_2$-, -CH ($CH_2CH_3$)-, -$CH_2$- ($CH_2$)$_2$- $CH_2$-, -CH ($CH_3$)- $CH_2$- $CH_2$-, -$CH_2$- CH ($CH_3$)- $CH_2$-, -CH ($CH_3$)- CH ($CH_3$)-, -CH ($CH_2CH_3$)- $CH_2$-, -C ($CH_3$)$_2$- $CH_2$-, -CH ($CH_2CH_2CH_3$)-, -C ($CH_3$) ($CH_2CH_3$)-, -$CH_2$- ($CH_2$)$_3$- $CH_2$-, -CH ($CH_3$)- $CH_2$- $CH_2$- $CH_2$-, -$CH_2$- CH ($CH_3$)- $CH_2$- $CH_2$-, -CH ($CH_3$)- $CH_2$- CH ($CH_3$)-, -CH ($CH_3$)- CH ($CH_3$)- $CH_2$-, -C ($CH_3$)$_2$- $CH_2$- $CH_2$-, -$CH_2$- C ($CH_3$)$_2$- $CH_2$-, -CH ($CH_2CH_3$)- $CH_2$- $CH_2$-, -$CH_2$- CH ($CH_2CH_3$)- $CH_2$-, -C ($CH_3$)$_2$- CH ($CH_3$)-, -CH ($CH_2CH_3$)- CH ($CH_3$)-, -C ($CH_3$) ($CH_2CH_3$)- $CH_2$-, -CH ($CH_2CH_2CH_3$)- $CH_2$-, -C ($CH_2CH_2CH_3$)- $CH_2$-, -CH ($CH_2CH_2CH_2CH_3$)-, -C ($CH_3$) ($CH_2CH_2CH_3$)-, -C ($CH_2CH_3$)$_2$-, -$CH_2$- ($CH_2$)$_4$- $CH_2$-, -CH=CH-, -CH=CH- $CH_2$-, -C ($CH_3$) =$CH_2$-, -CH=CH- $CH_2$- $CH_2$-, -$CH_2$- CH=CH- $CH_2$-, -CH=CH- CH=CH-, -C ($CH_3$) =CH- $CH_2$-, -CH=C ($CH_3$)- $CH_2$-, -C ($CH_3$) =C ($CH_3$)-, -C ($CH_2CH_3$) =CH-, -CH=CH- $CH_2$- $CH_2$- $CH_2$-, -$CH_2$- CH=$CH_2$- $CH_2$- $CH_2$-, -CH=CH=CH- $CH_2$- $CH_2$-, -CH=$CH_2$- CH- CH=$CH_2$-, -C≡C-, -C≡C- $CH_2$-, -C≡C- $CH_2$- $CH_2$-, -C≡C- CH ($CH_3$)-, -$CH_2$- C≡C- $CH_2$-, -C≡C- C≡C-, -C≡C- C ($CH_3$)$_2$-, -C≡C- $CH_2$- $CH_2$- $CH_2$-, -$CH_2$- C≡C- $CH_2$- $CH_2$-, -C≡C- C≡C- $CH_2$- und- C≡C- $CH_2$- C≡C- .

Die Ausdrücke "über $C_{2-8}$- Heteroalkyl verbrücktes Aryl, Heteroaryl, Heterocyclyl oder Cycloalkyl" und "über $C_{2-4}$- Heteroalkyl verbrücktes Aryl, Heteroaryl, Heterocyclyl oder Cycloalkyl" bedeuten im Sinne der Erfindung, dass $C_{2-8}$- Heteroalkyl oder $C_{2-4}$- Heteroalkyl und Aryl bzw. Heteroaryl bzw. Heterocyclyl bzw. Cycloalkyl die oben definierten Bedeutungen haben und der Aryl- bzw. Heteroaryl- bzw. Heterocyclyl- bzw. Cycloalkyl- Rest über eine $C_{2-8}$- Heteroalkyl- Gruppe oder eine $C_{2-4}$- Heteroalkyl- Gruppe an die jeweilige übergeordnete allgemeine Struktur gebunden ist. Die Heteroalkylkette kann in allen Fällen gesättigt oder ungesättigt, verzweigt oder unverzweigt, unsubstituiert oder einfach oder mehrfach substituiert sein. Ist ein endständiges Kohlenstoffatom der $C_{2-8}$- Heteroalkyl- Gruppe oder der $C_{2-4}$- Heteroalkyl- Gruppe durch ein Heteroatom oder eine Heteroatomgruppe ersetzt, so erfolgt die Bindung eines Heteroaryls oder eines Heterocyclyls an das Heteroatom oder die Heteroatomgruppe des $C_{2-8}$- Heteroalkyls oder des $C_{2-4}$- Heteroalkyls stets über ein Kohlenstoffatom des Heteroaryls oder Heterocyclyls. Unter dem endständigen Kohlenstoffatom wird das Kohlenstoffatom innerhalb des $C_{2-8}$- Heteroalkyls oder des $C_{2-4}$- Heteroalkyls verstanden, welches innerhalb der jeweiligen Kette am weitesten von der allgemeinen übergeordneten Struktur entfernt ist. Ist das endständige Kohlenstoffatom eines $C_{2-8}$- Heteroalkyls bspw. durch eine N ($CH_3$)- Gruppe ersetzt, ist diese innerhalb des $C_{2-8}$- Heteroalkyls am weitesten von der allgemeinen übergeordneten Struktur entfernt und an den Aryl- bzw. Heteroaryl- bzw. Heterocyclyl- bzw. Cycloalkyl- Rest gebunden. Vorzugsweise ist $C_{2-8}$- Heteroalkyl oder $C_{2-4}$- Heteroalkyl ausgewählt aus der Gruppe umfassend- $CH_2$- NH-, -$CH_2$- N ($CH_3$)-, -$CH_2$- O-, -$CH_2$- $CH_2$- NH-, -$CH_2$- $CH_2$- N ($CH_3$)-, -$CH_2$- $CH_2$- O-, -$CH_2$- $CH_2$- $CH_2$- NH-, -$CH_2$- $CH_2$- $C_{H2}$- N ($CH_3$)-, -$CH_2$- $CH_2$- $CH_2$- O-, -$CH_2$- O- $CH_2$-, -$CH_2$- $CH_2$- O- $CH_2$-, -$CH_2$- $CH_2$- O- $CH_2$- $CH_2$-, -$CH_2$- $CH_2$- O- $CH_2$- $CH_2$- O- $CH_2$-, -CH=CH- O- $CH_2$-, -CH=CH- O- $CH_2$- $CH_2$-, =CH- O- $CH_2$-, =CH- O- $CH_2$- $CH_2$-, =CH- $CH_2$- O- $CH_2$- $CH_2$-, =CH- $CH_2$- O- $CH_2$-, -$CH_2$- NH- $CH_2$-, -$CH_2$- $CH_2$- NH- $CH_2$-, -$CH_2$- $CH_2$- NH- $CH_2$- $CH_2$-, -$CH_2$- $CH_2$- NH- $CH_2$- $CH_2$- NH- $CH_2$, -CH=CH- NH- $CH_2$-, -CH=CH- NH- $CH_2$- $CH_2$-, -CH=CH- N ($CH_3$)- $CH_2$- $CH_2$-, =CH- NH- $CH_2$-, =CH- NH- $CH_2$- $CH_2$-, =CH- $CH_2$- NH- $CH_2$- $CH_2$-, =CH- $CH_2$- NH- $CH_2$-, -$CH_2$- N ($CH_3$)- $CH_2$-, -$CH_2$- $CH_2$- N ($CH_3$)- $CH_2$-, -$CH_2$- $CH_2$- N ($CH_3$)- $CH_2$- $CH_2$-, -$CH_2$- $CH_2$- N ($CH_3$)- $CH_2$- $CH_2$- N ($CH_3$)- $CH_2$-, -$CH_2$-

$CH_2$- NH- $CH_2$- $CH_2$- O- $CH_2$-, -$CH_2$- $CH_2$- O- $CH_2$- $CH_2$- NH- $CH_2$-, -$CH_2$- $CH_2$- N ($CH_3$)- $CH_2$- $CH_2$- O- $CH_2$-, -$CH_2$- $CH_2$- O- $CH_2$- $CH_2$- N ($CH_3$)- $CH_2$-, -$CH_2$- NH- $CH_2$- O- $CH_2$-, -$CH_2$- O- $CH_2$- NH- $CH_2$-, -$CH_2$- N ($CH_3$)- $CH_2$- O- $CH_2$-, -$CH_2$- O- $CH_2$- N ($CH_3$)- $CH_2$-, -CH=CH- N ($CH_3$)- $CH_2$-, =CH- N ($CH_3$)- $CH_2$-, =CH- N ($CH_3$)- $CH_2$- $CH_2$-, =CH- $CH_2$- N ($CH_3$)- $CH_2$- $CH_2$-, =CH- $CH_2$- N ($CH_3$)- $CH_2$-, -$CH_2$- S-, -$CH_2$- $CH_2$- S-, -$CH_2$- $CH_2$- $CH_2$- S-, -$CH_2$- $CH_2$- $CH_2$- $CH_2$- S-, -CH2- S $(=O)_2$-, -$CH_2$- $CH_2$- S $(=O)_2$-, -$CH_2$- $CH_2$- $CH_2$- S $(=O)_2$- und- $CH_2$- $CH_2$- $CH_2$- $CH_2$- S $(=O)_2$- .

Im Zusammenhang mit "Alkyl", "Heteroalkyl", "Heterocyclyl" und "Cycloalkyl" versteht man unter dem Begriff "ein- oder mehrfach substituiert" im Sinne dieser Erfindung die ein- oder mehrfache, z.B. zwei-, drei- oder vierfache Substitution eines oder mehrerer Wasserstoffatome jeweils unabhängig voneinander durch Substituenten ausgewählt aus der Gruppe aus F; Ci; Br; I; CN; $CF_3$; =O; =NH; =C $(NH_2)_2$; $NO_2$; $R^0$; C (=O) H; C (=O) $R^0$; $CO_2H$; C (=O) $OR^0$; $CONH_2$; C (=O) $NHR^0$; C (=O) N $(R^0)_2$ OH; $OR^0$;- O- ($C_{1-8}$- Alkyl)- O- ; O- C (=O)- $R^0$; O- C (=O)- O- $R^0$; O- (C=O)- NH- $R^0$; O- C (=O)- N $(R^0)_2$; O- S $(=O)_2$- $R^0$; O- S $(=O)_2$OH; O- S $(=O)_2OR^0$; O- S $(=O)_2NH_2$; O- S $(=O)_2NHR^0$; O- S $(=O)_2$N $(R^0)_Z$; $NH_2$; NH- $R^0$; N $(R^0)_2$; NH- C (=O)- $R^0$; NH- C (=O)- O- $R^0$; NH- C (=O)- $NH_2$; NH- C (=O)- NH- $R^0$; NH- C (=O)- N $(R^0)_2$; $NR^0$- C (=O)- $R^0$; $NR^0$- C (=O)- O- $R^0$; $NR^0$- C (=O)- $NH_2$; $NR^0$- C (=O)- NH- $R^0$; $NR^0$- C (=O)- N $(R^0)_2$; NH- S $(=O)_2$OH; NH- S $(=O)_2R^0$; NH- S $(=O)_2OR^0$; NH- S $(=O)_2NH_2$; NH- S $(=O)_2NHR^0$; NH- S $(=O)_2$N $(R^0)_2$-, $NR^0$- S $(=O)_2$OH; $NR^0$- S $(=O)_2R^0$; $NR^0$- S $(=O)_2OR^0$; $NR^0$- S $(=O)_2NH_2$; $NR^0$- S $(=O)_2NHR^0$; $NR^0$- S $(=O)_2$N $(R^0)_2$, SH; $SR^0$; S (=O) $R^0$; S $(=O)_2R^0$; S $(=O)_2$OH; S $(=O)_2OR^0$; S $(=O)_2NH_2$; S $(=O)_2NHR^0$; S $(=O)_2$N $(R^0)_2$, wobei unter mehrfach substituierten Resten solche Reste zu verstehen sind, die entweder an verschiedenen oder an gleichen Atomen mehrfach, z.B. zwei-, drei- oder vierfach substituiert sind, beispielsweise dreifach am gleichen C- Atom wie im Falle von $CF_3$ oder $CH_2CF_3$ oder an verschiedenen Stellen wie im Falle von CH (OH)- CH=CH- $CHCl_2$. Ein Substituent kann gegebenenfalls seinerseits wiederum einfach oder mehrfach substituiert sein. Die Mehrfachsubstitution kann mit dem gleichen oder mit verschiedenen Substituenten erfolgen.

Bevorzugte "Alkyl", "Heteroalkyl- ", "Heterocyclyl- " und "Cycloalkyl- " Substituenten sind ausgewählt aus der Gruppe aus F; Cl; Br; I; $NO_2$; $CF_3$; CN; =O; =NH; $R^0$; C (=O) ($R^0$ oder H) ; C (=O) O ($R^0$ oder H) ; C (=O) N ($R^0$ oder $H)_2$; OH; $OR^0$; O- C (=O)- $R^0$; O- ($C_{1-8}$- Alkyl)- OH;- O- ($C_{1-8}$- Alkyl)- O- ; O- ($C_{1-8}$- Alkyl)- O- $C_{1-8}$- Alkyl; $OCF_3$; N ($R^0$ oder $H)_2$; N ($R^0$ oder H)- C (=O)- $R^0$; N ($R^0$ oder H)- C (=O)- N ($R^0$ oder $H)_2$; SH; $SCF_3$; $SR^0$; S $(=O)_2R^0$; S $(=O)_2$O ($R^0$ oder H) und S $(=O)_2$- N ($R^0$ oder $H)_2$.

Besonders bevorzugte "Alkyl", "Heteroalkyl- ", "Heterocyclyl- " und "Cycloalkyl- " Substituenten sind ausgewählt aus der Gruppe bestehend aus F; Cl; Br; I; $NO_2$; $CF_3$; CN; =O; $C_{1-8}$- Alkyl; Aryl; Heteroaryl; $C_{3-10}$- Cycloalkyl; Heterocyclyl; über $C_{1-8}$- Alkyl verbrücktes Aryl, Heteroaryl, $C_{3-10}$- Cycloalkyl oder Heterocyclyl; CHO; C (=O) $C_{1-8}$- Alkyl; C (=O) Aryl; C (=O) Heteroaryl; $CO_2H$; C (=O) O- $C_{1-8}$- Alkyl; C (=O) O- Aryl; C (=O) O- Heteroaryl; $CONH_2$; C (=O) NH- $C_{1-8}$- Alkyl; C (=O) N ($C_{1-8}$- $Alkyl)_2$; C (=O) NH- Aryl; C (=O) N $(Aryl)_2$; C (=O) NH- Heteroaryl; C (=O) N $(Heteroaryl)_2$; C (=O) N ($C_{1-8}$- Alkyl) (Aryl) ; C (=O) N ($C_{1-8}$- Alkyl) (Heteroaryl) ; C (=O) N (Heteroaryl) (Aryl) ; OH; O- $C_{1-8}$- Alkyl; $OCF_3$;- O- ($C_{1-8}$- Alkyl)- O- ; O- ($C_{1-8}$- Alkyl)- OH; O- ($C_{1-8}$- Alkyl)- O- $C_{1-8}$- Alkyl; 0- Benzyl; O- Aryl; O- Heteroaryl; O- C (=O) $C_{1-8}$- Alkyl; O- C (=O) Aryl; O- C (=O) Heteroaryl; $NH_2$; NH- $C_{1-8}$- Alkyl; N ($C_{1-8}$- $Alkyl)_2$; NH- C (=O) $C_{1-8}$- Alkyl; NH- C (=O)- Aryl; NH- C (=O)- Heteroaryl; SH; S- $C_{1-8}$- Alkyl; $SCF_3$; S- Benzyl; S- Aryl; S- Heteroaryl; S $(=O)_2C_{1-8}$- Alkyl; S $(=O)_2$Aryl; S $(=O)_2$Heteroaryl; S $(=O)_2$OH; S $(=O)_2$O- $C_{1-8}$- Alkyl; S $(=O)_2$O- Aryl; S $(=O)_2$O- Heteroaryl; S $(=O)_2$- NH- $C_{1-8}$- Alkyl; S $(=O)_2$- NH- Aryl; und S $(=O)_2$- NH- $C_{1-8}$- Heteroaryl.

Im Zusammenhang mit "Aryl" und "Heteroaryl" versteht man im Sinne dieser Erfindung unter "ein- oder mehrfach substituiert" die ein- oder mehrfache, z.B. zwei-, drei- oder vierfache Substitution eines oder mehrerer Wasserstoffatome des Ringsystems jeweils unabhängig voneinander durch Substituenten ausgewählt aus der Gruppe aus F; Cl; Br; I; $NO_2$; $CF_3$; CN; $R^0$; C (=O) H; C (=O) $R^0$; $CO_2H$; C (=O) $OR^0$; $CONH_2$; C (=O) $NHR^0$; C (=O) N $(R^0)_2$; OH; $OR^0$;- O- ($C_{1-8}$- Alkyl)- O- ; O- C (=O)- $R^0$; 0- C (=O)- O- $R^0$; O- (C=O)- NH- $R^0$; O- C (=O)- N $(R^0)_2$; O- S $(=O)_2$- $R^0$; O- S $(=O)_2$OH; 0- S $(=O)_2OR^0$; O- S $(=O)_2NH_2$; O- S $(=O)_2NHR^0$; O- S $(=O)_2$N $(R^0)_2$; $NH_2$; NH- $R^0$; N $(R^0)_2$; NH- C (=O)- $R^0$; NH- C (=O)- O- $R^0$; NH- C (=O)- $NH_2$; NH- C (=O)- NH- $R^0$; NH- C (=O)- N $(R^0)_2$; $NR^0$- C (=O)- $R^0$; $NR^0$- C (=O)- O- $R^0$) ; $NR^0$- C (=O)- $NH_2$; $NR^0$- C (=O)- NH- $R^0$; $NR^0$- C (=O)- N $(R^0)_2$; NH- S $(=O)_2$OH; NH- S $(=O)_2R^0$; NH- S $(=O)_2OR^0$; NH- S $(=O)_2NH_2$; NH- S $(=O)_2NHR^0$; NH- S $(=O)_2$N $(R^0)_2$; $NR^0$- S $(=O)_2$OH; $NR^0$- S $(=O)_2R^0$; $NR^0$- S $(=O)_2OR^0$; $NR^0$- S $(=O)_2NH_2$; $NR^0$- S $(=O)_2NHR^0$; $NR^0$- S $(=O)_2$N $(R^0)_2$; SH; $SR^0$; S (=O) $R^0$; S $(=O)_2R^0$; S $(=O)_2$OH; S $(=O)_2OR^0$; S $(=O)_2NH_2$; S $(=O)_2NHR^0$; S $(=O)_2$N $(R^0)_2$, an einem oder ggf. verschiedenen Atomen, wobei ein Substituent ggf. seinerseits wiederum einfach oder mehrfach substituiert sein kann. Die Mehrfachsubstitution erfolgt mit dem gleichen oder mit unterschiedlichen Substituenten.

Bevorzugte "Aryl- " und "Heteroaryl- " Substituenten sind F; Cl; Br; I; $NO_2$; $CF_3$; CN; $R^0$; C (=O) ($R^0$ oder H) ; C (=O) O ($R^0$ oder H) ; C (=O) N ($R^0$ oder $H)_2$; OH; $OR^0$; O- C (=O)- $R^0$;- O- ($C_{1-8}$- Alkyl)- O- ; O- ($C_{1-8}$- Alkyl)- O- $C_{1-8}$- Alkyl; $OCF_3$; N ($R^0$ oder $H)_2$; N ($R^0$ oder H)- C (=O)- $R^0$; N ($R^0$ oder H)- C (=O)- N ($R^0$ oder $H)_2$; SH; $SCF_3$; $SR^0$; S $(=O)_2R^0$; S $(=O)_2$O ($R^0$ oder H) ; S $(=O)_2$- N ($R^0$ oder $H)_2$.

Besonders bevorzugte "Aryl- " und "Heteroaryl- " Substituenten sind ausgewählt aus der Gruppe bestehend aus F; Cl; Br; I; $NO_2$; $CF_3$; CN; $C_{1-8}$- Alkyl; Aryl; Heteroaryl; $C_{3-10}$- Cycloalkyl; Heterocyclyl; $C_{1-8}$- Alkyl verbrücktes Aryl, Heteroaryl, $C_{3-10}$- Cycloalkyl oder Heterocyclyl; CHO; C (=O) $C_{1-8}$- Alkyl; C (=O) Aryl; C (=O) Heteroaryl; $CO_2H$; C (=O) O- $C_{1-8}$- Alkyl; C (=O) O- Aryl; C (=O) O- Heteroaryl; $CONH_2$; C (=O) NH- $C_{1-8}$- Alkyl; C (=O) N ($C_{1-8}$- $Alkyl)_2$; C (=O)NH- Aryl; C (=O) N $(Aryl)_2$; C (=O) NH- Heteroaryl; C (=O) N $(Heteroaryl)_2$; C (=O) N ($C_{1-8}$- Alkyl) (Aryl) ; C (=O) N ($C_{1-8}$- Alkyl) (Heteroaryl) ; C (=O) N (Heteroaryl) (Aryl) ; OH; O- $C_{1-8}$- Alkyl; $OCF_3$;- O- ($C_{1-8}$- Alkyl)- O- ; O- ($C_{1-8}$- Alkyl)- OH; O- ($C_{1-8}$- Alkyl)- O- $C_{1-8}$- Alkyl; O- Benzyl; O- Aryl; O- Heteroaryl; O- C (=O) $C_{1-8}$- Alkyl; O- C (=O) Aryl; O- C (=O) Heteroaryl; $NH_2$; NH- $C_{1-8}$- Alkyl; N ($C_{1-8}$- $Alkyl)_2$; NH- C (=O) $C_{1-8}$- Alkyl; NH- C (=O)- Aryl; NH- C (=O)- Heteroaryl; SH; S- $C_{1-8}$- Alkyl; $SCF_3$; S- Benzyl; S- Aryl; S- Heteroaryl; S $(=O)_2C_{1-8}$- Alkyl; S $(=O)_2$Aryl; S $(=O)_2$Heteroaryl; S $(=O)_2$OH; S $(=O)_2$O- $C_{1-8}$- Alkyl; S $(=O)_2$O- Aryl; S $(=O)_2$O- Heteroaryl; S $(=O)_2$- NH- $C_{1-8}$- Alkyl; S $(=O)_2$- NH- Aryl; S $(=O)_2$- NH- $C_{1-8}$- Heteroaryl.

Die erfindungsgemäßen Verbindungen sind durch Substituenten definiert, beispielsweise durch $R^1$, $R^2$ und $R^3$ (Substituenten der 1. Generation) , welche ihrerseits ggf. substituiert sind (Substituenten der 2. Generation) . Je nach Definition können diese Substituenten der Substituenten ihrerseits erneut substituiert sein (Substituenten der 3. Generation) . Ist beispielsweise $R^3 = R^0$ wobei $R^0$ = Aryl (Substituent der 1. Generation) , so kann Aryl seinerseits substituiert sein, z.B. mit $NHR^0$, wobei $R^0$ = $C_{1-10}$- Alkyl (Substituent der 2. Generation) . Es ergibt sich daraus die funktionelle Gruppe Aryl- $NHC_{1-10}$- Alkyl. $C_{1-10}$- Alkyl kann dann seinerseits erneut substituiert sein, z.B. mit Cl (Substituent der 3. Generation) . Es ergibt sich daraus dann insgesamt die funktionelle Gruppe Aryl- $NHC_{1-10}$- Alkyl- Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform bspw. im Fall der allgemeinen Formel (1) die funktionellen Gruppen für $R^0$ bis $R^8$ jeweils ggf. substituiert sein, die jeweiligen Substituenten können dann ihrerseits jedoch nicht erneut substituiert sein.

In einigen Fällen sind die erfindungsgemäßen Verbindungen durch Substituenten definiert, die einen Aryl- oder Heteroaryl- Rest darstellen oder tragen, jeweils unsubstituiert oder einfach oder mehrfach substituiert oder die zusammen mit dem oder den sie verbindenden Kohlenstoff- oder Heteroatom (en) als Ringglied oder als Ringgliedern einen Ring bilden, beispielsweise ein Aryl oder Heteroaryl, jeweils unsubstituiert oder einfach oder mehrfach substituiert. Sowohl diese Aryl- oder Heteroaryl- Reste als auch die so gebildeten aromatischen Ringsysteme können ggf. mit $C_{3-10}$- Cycloalkyl oder Heterocyclyl, jeweils gesättigt oder ungesättigt, kondensiert sein, d.h. mit einem $C_{3-10}$- Cycloalkyl wie Cyclopentyl oder einem Heterocyclyl wie Morpholinyl, wobei die so kondensierten $C_{3-10}$- Cycloalkyl- oder Heterocyclyl- Reste ihrerseits jeweils unsubstituiert oder einfach oder mehrfach substituiert sein können.

In einigen Fällen sind die erfindungsgemäßen Verbindungen durch Substituenten definiert, die einen $C_{3-10}$- Cycloalkyl- oder Heterocyclyl- Rest darstellen oder tragen, jeweils unsubstituiert oder einfach oder mehrfach substituiert oder die zusammen mit dem oder den sie verbindenden Kohlenstoff- oder Heteroatom (en) als Ringglied oder als Ringgliedern einen Ring bilden, beispielsweise ein $C_{3-10}$- Cycloalkyl oder Heterocyclyl, jeweils unsubstituiert oder einfach oder mehrfach substituiert. Sowohl diese $C_{3-10}$- Cycloalkyl oder Heterocyclyl- Reste als auch die gebildeten aliphatischen Ringsysteme können ggf. mit Aryl oder Heteroaryl kondensiert sein, d.h. mit einem Aryl wie Phenyl oder einem Heteroaryl wie Pyridyl, wobei die so kondensierten Aryl- oder Heteroaryl- Reste ihrerseits jeweils unsubstituiert oder einfach oder mehrfach substituiert sein können. Vorzugsweise sind die so gebildeten Ringsysteme mit einem Aryl kondensiert, besonders bevorzugt mit Phenyl. Bilden die Substituenten $R^9$ und $R^{10}$ beispielsweise mit dem sie verbindenden Stickstoffatom ein Piperidinyl, so kann dieser Piperidinyl- Ring mit Phenyl zu Tetrahydroisochinolinyl kondensiert sein. Im Rahmen der vorliegenden Erfindung bezeichnet das in Formeln verwendete Symbol

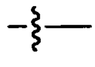

eine Verknüpfung eines entsprechenden Restes an die jeweilige übergeordnete allgemeine Struktur.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch- insbesondere bei Anwendung im Menschen und/ oder Säugetier- verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, Saccharinsäure, Monomethylsebacinsäure, 5- Oxo- prolin, Hexan- 1- sulfonsäure, Nicotinsäure, 2-, 3- oder 4- Aminobenzoesäure, 2, 4, 6- Trimethyl- benzoesäure, $\alpha$- Liponsäure, Acetylglycin, Hippursäure, Phosphorsäure und/ oder Asparaginsäure. Besonders bevorzugt sind die Zitronensäure und die Salzsäure.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calcium-Salze.

In einer bevorzugten Ausführungsform der Erfindung steht m für 0.

In einer ebenfalls bevorzugten Ausführungsform der Erfindung steht n für 1, 2 oder 3.

In einer weiteren bevorzugten Ausführungsform sind die Substituenten $R^1$, $R^2$, $R^3$ und $R^4$ jeweils unabhängig voneinander ausgewählt aus der Gruppe bestehend aus H; F; Cl; Br, I; $NO_2$; $CF_3$; CN; OH; $OCF_3$; SH; $SCF_3$; $NH_2$; $C_{1-8}$- Alkyl, O- $C_{1-8}$- Alkyl, O- C (=O)- $C_{1-8}$- Alkyl, S- $C_{1-8}$- Alkyl, NH ($C_{1-8}$- Alkyl) , N ($C_{1-8}$- Alkyl)$_2$, NH- C (=O)- $C_{1-8}$- Alkyl, N (C (=O)- $C_{1-8}$- Alkyl)$_2$ oder C (=O)- $C_{1-8}$- Alkyl, jeweils gesättigt oder ungesättigt, verzweigt oder unverzweigt, unsubstituiert oder einfach oder mehrfach substituiert; $C_{3-7}$- Cycloalkyl oder Heterocyclyl, jeweils gesättigt oder ungesättigt, unsubstituiert oder einfach oder mehrfach substituiert,
und $R^5$ ist ausgewählt aus der Gruppe bestehend aus H; F; Cl; Br; I; $NO_2$; $CF_3$; CN; $OCF_3$; SH; $SCF_3$; $NH_2$; $C_{1-8}$- Alkyl, O- $C_{1-8}$- Alkyl, O- C (=O)- $C_{1-8}$- Alkyl, S- $C_{1-8}$- Alkyl, NH ($C_{1-8}$- Alkyl) , N ($C_{1-8}$- Alkyl)$_2$, NH- C (=O)- $C_{1-8}$- Alkyl, N (C (=O)- $C_{1-8}$- Alkyl)$_2$ oder C (=O)- $C_{1-8}$- Alkyl, jeweils gesättigt oder ungesättigt, verzweigt oder unverzweigt, unsubstituiert oder einfach oder mehrfach substituiert; $C_{3-7}$- Cycloalkyl oder Heterocyclyl, jeweils gesättigt oder ungesättigt, unsubstituiert oder einfach oder mehrfach substituiert.

Vorzugsweise bedeuten $R^1$, $R^2$, $R^3$, $R^4$ jeweils unabhängig voneinander ausgewählt aus der Gruppe bestehend aus H; F; Cl; Br; I; $NO_2$; $CF_3$; CN; OH; $OCF_3$: SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl. N (C (=O)- $C_{1-6}$- Alkyl)$_2$ oder C (=O)- $C_{1-6}$- Alkyl, jeweils gesättigt oder ungesättigt, verzweigt oder unverzweigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl; $C_{3-6}$- Cycloalkyl oder- Heterocyclyl jeweils gesättigt oder ungesättigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl, und $R^5$ steht für H; F; Cl; Br; I; $NO_2$; $CF_3$; CN; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl, jeweils gesättigt oder ungesättigt, verzweigt oder unverzweigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$: $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl; $C_{3-6}$- Cycloalkyl oder- Heterocyclyl jeweils gesättigt oder ungesättigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-8}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl.

Besonders bevorzugt stehen $R^1$, $R^2$, $R^3$ und $R^4$ jeweils unabhängig voneinander für H; F; Cl; CN; $OCF_3$; $SCF_3$; $CF_3$; $CH_3$ oder $OCH_3$;
und $R^5$ bedeutet H, F, Cl, $OCF_3$, $SCF_3$, $C_{1-6}$- Alkyl, O- $C_{1-6}$- Alkyl, S- $C_{1-6}$- Alkyl, insbesondere H, $C_{1-4}$- Alkyl, O- $C_{1-4}$- Alkyl, ganz besonders bevorzugt H, $CH_3$, $OCH_3$.

Vorzugsweise bedeuten $R^{6a}$ und $R^{6b}$ jeweils unabhängig voneinander für H; F; Cl; Br; I; Methyl; Ethyl; n-Propyl; iso-Propyl; n-Butyl; sec.-Butyl; tert.-Butyl; OH; O-Methyl oder O-Ethyl, besonders bevorzugt H, $CH_3$ oder $OCH_3$.

Vorzugsweise bedeutet $R^7$ für $C_{1-8}$- Alkyl oder $C_{2-8}$- Heteroalkyl, gesättigt oder ungesättigt; verzweigt oder unverzweigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl; $C_{3-8}$- Cycloalkyl oder- Heterocyclyl gesättigt oder ungesättigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl; Aryl oder Heteroaryl, jeweils unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; $NO_2$; OH; $OCF_3$; SH; $SCF_3$; $NH_2$; $C_{1-6}$- Alkyl, O- $C_{1-6}$- Alkyl, O- C (=O)- $C_1$- 6- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{16}$- Alkyl)$_2$ oder C (=O)- $C_{1-6}$- Alkyl.

Besonders bevorzugt steht $R^7$ für $C_{2-6}$- Alkyl oder $C_{2-6}$- Heteroalkyl, gesättigt oder ungesättigt; verzweigt oder unverzweigt, unsubstituiert; $C_{3-8}$- Cycloalkyl oder- Heterocyclyl gesättigt oder ungesättigt, unsubstituiert; Phenyl, Furyl, Thienyl oder Pyridyl, jeweils unsubstituiert oder einfach oder mehrfach substituiert mit einem oder mehreren Substituenten jeweils unabhängig voneinander ausgewählt aus der Gruppe bestehend aus F, Cl, Br, I, CN; $CF_3$, $OCF_3$, $SCF_3$, $CH_3$ und $OCH_3$.

In einer bevorzugten Ausführungsform der Erfindung steht m für 0, n für 1,2 oder 3 und $R^7$ für Aryl oder Heteroaryl

jeweils unsubstituiert oder einfach oder mehrfach substituiert.

In einer besonders bevorzugten Ausführungsform der Erfindung steht m für 0, n für 1 und $R^7$ für Aryl oder Heteroaryl jeweils unsubstituiert oder einfach oder mehrfach substituiert.

In einer ganz besonders bevorzugten Ausführungsform der Erfindung steht m für 0, n für 1 und $R^7$ für Phenyl, Thienyl oder Pyridyl jeweils unsubstituiert oder einfach oder mehrfach substituiert mit einem oder mehreren Substituenten jeweils unabhängig voneinander ausgewählt aus der Gruppe bestehend aus F, Cl, Br, I, CN, $CF_3$, $OCF_3$, $SCF_3$, $CH_3$ und $OCH_3$.

In einer anderen bevorzugten Ausführungsform der Erfindung steht m für 0, n 1 oder 2 und $R^7$ für $C_{1-10}$- Alkyl oder $C_{2-10}$- Heteroalkyl, gesättigt oder ungesättigt; verzweigt oder unverzweigt, unsubstituiert oder einfach oder mehrfach substituiert; $C_{3-10}$- Cycloalkyl oder- Heterocyclyl gesättigt oder ungesättigt, unsubstituiert oder einfach oder mehrfach substituiert.

In einer anderen bevorzugten Ausführungsform der Erfindung steht m für 0, n 1 oder 2 und $R^7$ für $C_{1-8}$- Alkyl oder $C_{2-8}$- Heteroalkyl, gesättigt oder ungesättigt; verzweigt oder unverzweigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl; $C_{3-8}$- Cycloalkyl oder- Heterocyclyl gesättigt oder ungesättigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl.

In einer anderen bevorzugten Ausführungsform der Erfindung steht m für 0, n 1 oder 2 und $R^7$ für $C_{1-6}$- Alkyl oder $C_{2-6}$- Heteroalkyl, gesättigt oder ungesättigt; verzweigt oder unverzweigt, unsubstituiert oder einfach oder mehrfach substituiert mit einem oder mehreren Substituenten unabhängig voneinander ausgewählt aus der Gruppe bestehend aus F, Cl, $OCH_3$ und $CF_3$; $C_{3-8}$- Cycloalkyl oder- Heterocyclyl gesättigt oder ungesättigt, unsubstituiert oder einfach oder mehrfach substituiert mit einem oder mehreren Substituenten unabhängig voneinander ausgewählt aus der Gruppe bestehend aus F, Cl, $OCH_3$ und $CF_3$;

Vorzugsweise ist $R^8$ ausgewählt ist aus der Gruppe bestehend aus $C_{1-10}$- Alkyl oder $C_{2-10}$- Heteroalkyl, jeweils gesättigt oder ungesättigt, verzweigt oder unverzweigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl; $C_{3-10}$- Cycloalkyl oder- Heterocyclyl, jeweils gesättigt oder ungesättigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl; Aryl oder Heteroaryl, jeweils unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl. O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl; über $C_{1-8}$- Alkyl oder $C_{28}$- Heteroalkyl verbrücktes $C_{3-10}$- Cycloalkyl oder- Heterocyclyl, jeweils gesättigt oder ungesättigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl, wobei die Alkyl- oder Heteroalkylkette jeweils verzweigt oder unverzweigt, gesättigt oder ungesättigt, unsubstituiert sein kann; oder über $C_{1-8}$- Alkyl oder $C_{2-8}$- Heteroalkyl verbrücktes Aryl oder Heteroaryl, jeweils unsubstituiert oder einfach oder mehrfach substituiert mit einem oder mehreren Substituenten unabhängig voneinander ausgewählt aus der Gruppe bestehend aus H; F; Cl; Br; I; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$, NH- C (=O)- $C_{1-6}$- Alkyl, N (C (=O)- $C_{1-6}$- Alkyl)$_2$ oder C (=O)- $C_{1-6}$- Alkyl, wobei die Alkyl- oder Heteroalkylkette jeweils verzweigt oder unverzweigt, gesättigt oder ungesättigt, unsubstituiert sein kann;

Besonders bevorzugt ist $R^8$ ausgewählt aus der Gruppe bestehend aus $C_{1-8}$- Alkyl oder $C_{2-8}$- Heteroalkyl, jeweils gesättigt oder ungesättigt, verzweigt oder unverzweigt, unsubstituiert oder einfach oder mehrfach substituiert mit einem oder mehreren Substituenten unabhängig voneinander ausgewählt aus der Gruppe bestehend aus F, Cl, Br, I, OH, =O, $OCF_3$, $SCF_3$, $CF_3$, $C_{1-4}$- Alkyl und $OC_{1-4}$- Alkyl; $C_{3-10}$- Cycloalkyl oder- Heterocyclyl, jeweils gesättigt oder ungesättigt, unsubstituiert oder einfach oder mehrfach substituiert mit einem oder mehreren Substituenten unabhängig voneinander ausgewählt aus der Gruppe bestehend aus F, Cl, Br, I, OH, =O, $OCF_3$, $SCF_3$, $CF_3$, $C_{1-4}$- Alkyl und $OC_{1-4}$- Alkyl; Aryl oder Heteroaryl, jeweils unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- Alkyl)$_2$,

NH- C (=O)- $C_{1-6}$- Alkyl, N (C (=O)- $C_{1-6}$- $Alkyl)_2$ oder C (=O)- $C_{1-6}$- Alkyl; Aryl oder Heteroaryl, jeweils unsubstituiert oder einfach oder mehrfach substituiert mit einem oder mehreren Substituenten unabhängig voneinander ausgewählt aus der Gruppe bestehend aus H; F; Cl; Br; I; $CF_3$; CN; OH; $OCF_3$; SH; $SCF_3$; $NH_2$; $C_{1-6}$- Alkyl, O- $C_{1-6}$- Alkyl, O- C (=O)- $C_{1-6}$- Alkyl, S- $C_{1-6}$- Alkyl, NH ($C_{1-6}$- Alkyl) , N ($C_{1-6}$- $Alkyl)_2$, NH- C (=O)- $C_{1-6}$- Alkyl, N (C (=O)- $C_{1-6}$- $Alkyl)_2$ oder C (=O)- $C_{1-6}$- Alkyl; über $C_{1-8}$- Alkyl oder $C_{2-8}$- Heteroalkyl verbrücktes $C_{3-10}$- Cycloalkyl, gesättigt oder ungesättigt, unsubstituiert oder einfach oder mehrfach substituiert mit einem oder mehreren Substituenten unabhängig voneinander ausgewählt aus der Gruppe bestehend aus F, Cl, Br, I, OH, =O, $OCF_3$, $SCF_3$, $CF_3$, $C_{1-8}$- Alkyl und $OC_{1-8}$- Alkyl, wobei die Alkyl- oder Heteroalkylkette jeweils verzweigt oder unverzweigt, gesättigt oder ungesättigt, unsubstituiert sein kann; oder über $C_{1-8}$- Alkyl oder $C_{2-8}$- Heteroalkyl verbrücktes Aryl oder Heteroaryl, jeweils unsubstituiert oder einfach oder mehrfach substituiert mit einem oder mehreren Substituenten unabhängig voneinander ausgewählt aus der Gruppe bestehend aus F, Cl, Br, I, OH, $NH_2$, $OCF_3$, $SCF_3$, $CF_3$, $C_{1-8}$- Alkyl und $OC_{1-8}$- Alkyl, wobei die Alkyl- oder Heteroalkylkette jeweils verzweigt oder unverzweigt, gesättigt oder ungesättigt, unsubstituiert sein kann.

Ganz besonders bevorzugt ist $R^8$ ausgewählt aus der Gruppe bestehend aus Methyl, Ethyl, n- Propyl, Isopropyl, n- Butyl, 2- Butyl, tert.- Butyl, n- Pentyl, Isopentyl, Neopentyl, n- Hexyl, Cyclopropyl, Cycobutyl, Cyclopentyl, Cyclohexyl, Methyl- cyclopropyl, Methyl- cyclobutyl, Methyl- cyclopentyl, Methyl- cylohexyl, Ethyl- cyclopropyl, Ethyl- cyclobutyl, Ethyl- cyclopentyl, Ethyl- cyclohexyl jeweils unsubstituiert oder ein oder mehrfach substituiert mit einem oder mehreren Substituenten ausgewählt aus der Gruppe bestehend aus F, Cl, Br, I, $OCF_3$, $SCF_3$, $CF_3$, und $OC_{1-8}$- Alkyl; oder Phenyl, Benzyl oder Phenethyl jeweils unsubstituiert oder ein oder mehrfach substituiert mit einem oder mehreren Substituenten ausgewählt aus der Gruppe bestehend aus F, Cl, Br, I, $OCF_3$, $SCF_3$, $CF_3$, $C_{1-8}$- Alkyl, $OC_{1-8}$- Alkyl und CN;

Insbesondere bevorzugt sind Verbindungen aus der Gruppe

**1** 2- Cyclohexyl- N- (2- (2- (phenylsulfonyl) ethylthio) chinolin- 3- yl) acetamid;
**2** N- (2- (2- (Phenylsulfonyl) ethylthio) chinolin- 3- yl)- 2- (thiophen- 2- yl) acetamid;
**4** N- (2- (Pentylthio) chinolin- 3- yl)- 2- (thiophen- 2- yl) acetamid;
**6** N- (2- (2- (Phenylthio) ethylthio) chinolin- 3- yl)- 2- (thiophen- 2- yl) acetamid;
**7** N- (2- (Ethylthio) chinolin- 3- yl)- 2- (thiophen- 2- yl) acetamid;
**9** N- (2- (Ethylthio) chinolin- 3- yl)- 3, 3- dimethylbutanamid;
**11** N- [2- Ethylsulfanyl- 7- (trifluormethyl)- chinolin- 3- yl]- 3, 3- dimethyl- butyramid
**12** 3- Cyclopentyl- N- [2- ethylsulfanyl- 7- (trifluormethyl)- chinolin- 3- yl]- propionamid
**14** 2- (5- Bicyclo [2.2.1] heptanyl)- N- (2- ethylsulfanyl- chinolin- 3- yl)- acetamid
**15** 3- Cyclopentyl- N- (2- ethylsulfanyl- chinolin- 3- yl)- propionamid
**16** 2- (5- Bicyclo [2.2.1] heptanyl)- N- [2- ethylsulfanyl- 7- (trifluormethyl)- chinolin- 3- yl]- acetamid
**17** 3- Cyclopentyl- N- [2- ethylsulfanyl- 4- methyl- 7- (trifluormethyl)- chinolin- 3- yl]- propionamid
**18** N- [2- Ethylsulfanyl- 4- methyl- 7- (trifluormethyl)- chinolin- 3- yl]- 2- thiophen- 2- yl- acetamid

oder deren physiologisch verträgliche Salze.

Die erfindungsgemäßen substituierten 3- Amino- 2- mercaptochinoline sowie jeweils die entsprechenden Säuren, Basen, Salze und Solvate eignen sich als pharmazeutische Wirkstoffe in Arzneimitteln.

Ein weiterer Gegenstand der Erfindung ist daher ein Arzneimittel enthaltend wenigstens ein erfindungsgemäßes substituiertes 3- Amino- 2- mercaptochinolin der allgemeinen Formel (1) , worin die Reste $R^1$- $R^8$ die oben angegebene Bedeutung haben sowie ggf. einen oder mehrere pharmazeutisch verträgliche Hilfsstoffe.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfindungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Diese erfindungsgemäßen Arzneimittel eignen sich zur Beeinflussung von KCNQ2/3-Kanälen und üben eine agonistische oder antagonistische, insbesondere eine agonistische Wirkung aus.

Bevorzugt eignen sich die erfindungsgemäßen Arzneimittel zur Behandlung von Störungen oder Krankheiten, die zumindest teilweise durch KCNQ2/3-Kanäle vermittelt werden.

Bevorzugt eignen sich die erfindungsgemäßen Arzneimittel zur Behandlung von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz, muskuiärem Schmerz und inflammatorischen Schmerz; Epilepsie, Harninkontinenz, Angstzuständen, Abhängigkeit, Manie, bipolaren Störungen, Migräne, kognitiven Erkrankungen, Dystonie-assoziierten Dyskinesien und/oder Harninkontinenz.

Besonders bevorzugt eignen sich die erfindungsgemäßen Arzneimittel zur Behandlung von Schmerz, ganz besonders bevorzugt von chronischem Schmerz, neuropathischem Schmerz, inflammatorischem Schmerz und muskulärem Schmerz.

Weiterhin eignen sich die erfindungsgemäßen Arzneimittel besonders bevorzugt zur Behandlung von Epilepsie.

Ein weiterer Gegenstand der Erfindung ist die Verwendung wenigstens eines erfindungsgemäßen substituierten 3- Amino- 2- mercaptochinolins sowie ggf. eines oder mehrerer pharmazeutisch verträglicher Hilfsstoffe zur Herstellung eines Arzneimittels zur Behandlung von Störungen oder Krankheiten, die zumindest teilweise durch KCNQ2/3- Kanäle vermittelt werden.

Bevorzugt ist die Verwendung wenigstens eines erfindungsgemäßen substituierten 3- Amino- 2- mercaptochinolins sowie ggf. eines oder mehrerer pharmazeutisch verträglicher Hilfsstoffe zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Epilepsie, Harninkontinenz, Angstzuständen, Abhängigkeit, Manie, bipolaren Störungen, Migräne, kognitiven Erkrankungen, Dystonie- assoziierten Dyskinesien und/ oder Harninkontinenz.

Besonders bevorzugt ist die Verwendung wenigstens eines erfindungsgemäßen substituierten 3- Amino- 2- mercaptochinolins sowie ggf. eines oder mehrerer pharmazeutisch verträglicher Hilfsstoffe zur Herstellung eines Arzneimittels zur Behandlung von Schmerz, ganz besonders bevorzugt von chronischem Schmerz, neuropathischem Schmerz, inflammatorischem Schmerz und muskulärem Schmerz.

Weiterhin besonders bevorzugt ist die Verwendung wenigstens eines erfindungsgemäßen substituierten 3- Amino- 2- mercaptochinoiins sowie ggf. eines oder mehrerer pharmazeutisch verträglicher Hilfsstoffe zur Herstellung eines Arzneimittels zur Behandlung von Epilepsie.

Ein weiterer Gegenstand der Erfindung ist wenigstens ein erfindungsgemäßes substituiertes 3- Amino- 2- mercaptochinolin sowie ggf. ein oder mehrere pharmazeutisch verträgliche Hilfsstoffe zur Behandlung von Störungen oder Krankheiten, die zumindest teilweise durch KCNQ2/3- Kanäle vermittelt werden.

Ein weiterer Gegenstand der Erfindung ist wenigstens ein erfindungsgemäßes substituiertes 3- Amino- 2- mercaptochinolin sowie ggf. ein oder mehrere pharmazeutisch verträgliche Hilfsstoffe zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Epilepsie, Harninkontinenz, Angstzuständen, Abhängigkeit, Manie, bipolaren Störungen, Migräne, kognitiven Erkrankungen, Dystonie- assoziierten Dyskinesien und/ oder Harninkontinenz.

Besonders bevorzugt ist wenigstens ein erfindungsgemäßes substituiertes 3- Amino- 2- mercaptochinolins sowie ggf. ein oder mehrere pharmazeutisch verträgliche Hilfsstoffe zur Behandlung von Schmerz, ganz besonders bevorzugt von chronischem Schmerz, neuropathischem Schmerz, inflammatorischem Schmerz und muskulärem Schmerz.

Besonders bevorzugt ist auch wenigstens ein erfindungsgemäßes substituiertes 3- Amino- 2- mercaptochinolin sowie ggf. ein oder mehrere pharmazeutisch verträgliche Hilfsstoffe zur Behandlung von Epilepsie.

Die Wirksamkeit gegen Schmerz kann beispielsweise im Bennett- bzw. Chung-Modell gezeigt werden (Bennett, G.J. and Xie, Y.K., A peripheral mononeuropathy in rat that produces disorders of pain sensation like those seen in man, Pain 1988, 33(1), 87-107; Kim, S.H. and Chung, J.M., An experimental model for peripheral neuropathy produced by segmental spinal nerve ligation in the rat, Pain 1992, 50(3), 355-363). Die Wirksamkeit gegen Epilepsie kann beispielsweise im DBA/2 Maus Modell (De Sarro et al., Naunyn-Schmiedeberg's Arch. Pharmacoi. 2001, 363, 330-336) nachgewiesen werden.

Bevorzugt weisen die erfindungsgemäßen substituierten 3- Amino- 2- mercaptochinoline einen $EC_{50}$- Wert von höchstens 10 μM oder höchstens 5 μM auf, bevorzugter höchstens 3 μM oder höchstens 2 μM, noch bevorzugter höchstens 1, 5 μM oder höchstens 1 μM, am bevorzugtesten höchstens 0, 8 μM oder höchstens 0, 4 μM und insbesondere höchstens 0, 3 μM oder höchstens 0, 2 μM. Methoden zur Bestimmung des $EC_{50}$- Wertes sind dem Fachmann bekannt. Bevorzugt erfolgt die Bestimmung des $EC_{50}$- Wertes fluorimetrisch, besonders bevorzugt wie unter "Pharmakologische Experimente" beschrieben.

Ein weiterer Gegenstand der Erfindung sind Verfahren zur Herstellung der erfindungsgemäßen substituierten 3- Amino- 2- mercaptochinoline.

Die in den nachstehend beschriebenen Umsetzungen zum Einsatz kommenden Chemikalien und Reaktionskomponenten sind käuflich erhältlich oder können jeweils nach üblichen, dem Fachmann bekannten Methoden hergestellt werden.

**Allgemeine Herstellungsverfahren**

Schema 1:

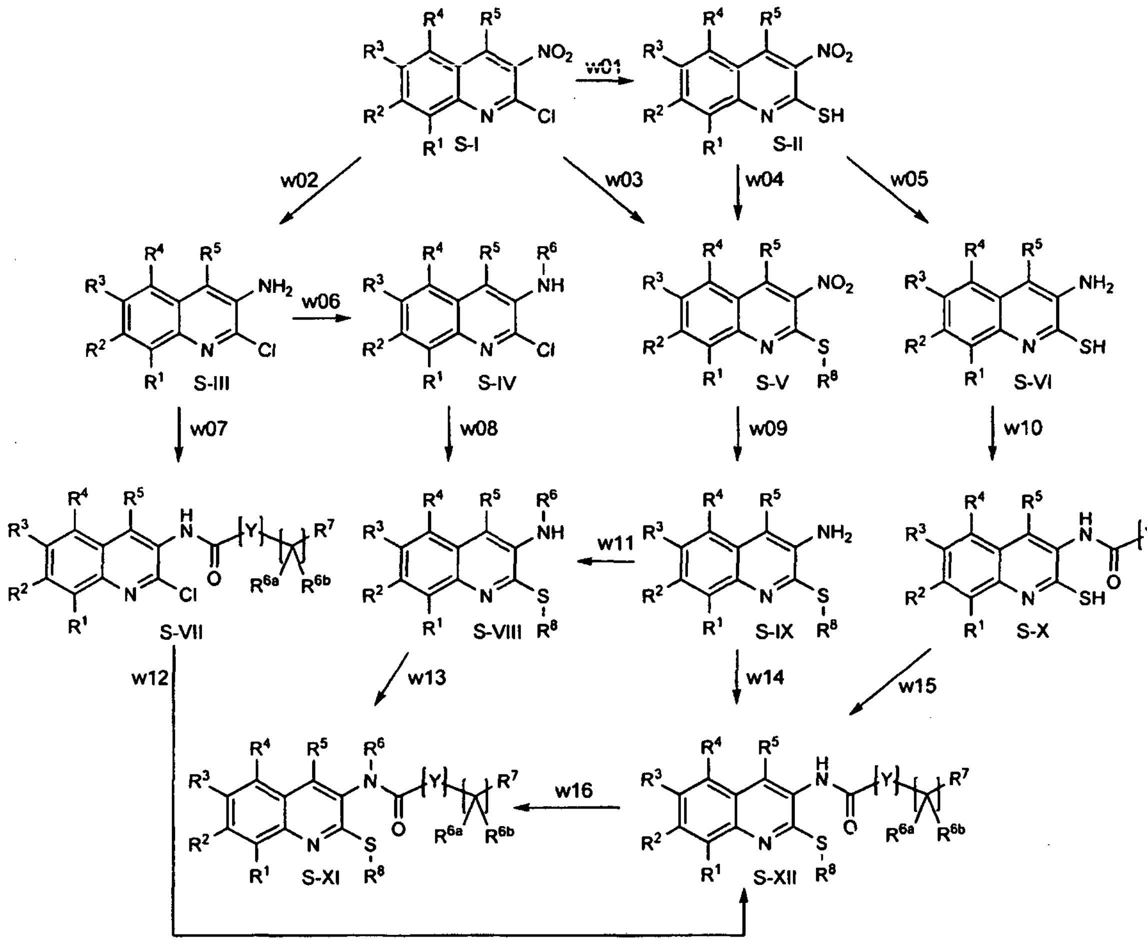


Im Schritt **w01** kann das 2-Halogen-chinolin **S-I**, worin X bevorzugt für F oder Chlor steht, mittels dem Fachmann bekannten Methoden wie bspw. durch Substitution mit einem Thiol, beispielsweise 3-Mercaptopropansäureethylester, zunächst in den entsprechenden Thioether überführt werden, welcher im Anschluss zum Thiol **S-II**, ggf. in Gegenwart einer Säure oder einer Base gespalten werden kann.

In den Schritten **w02**, **w05** und **w09** können die Nitro-Gruppen der Verbindungen **S-I**, **S-II** und **S-V** mittels dem Fachmann geläufigen Reduktionsmethoden wie bspw. in Gegenwart von Metallen in saurer Lösung oder durch katalytische Hydrierung in die entsprechenden Amine **S-III, S-VI** und **S-IX** überführt werden.

In den Schritten **w07, w10, w13** und **w14** können die Amine **S- III, S- VI, S- VIII** und **S- IX** in die entsprechenden Amide **S- VII, S- IX, S- XI** und **S- XII** überführt werden. Dies kann bspw. jeweils durch Reaktion mit einem Säurechlorid $R^7$- C (=O)- Cl mittels dem Fachmann geläufigen Methoden, ggf. in Gegenwart einer Base oder durch Reaktion mit einer Säure R'- C (=O)- OH in Gegenwart eines geeigneten Kopplungsreagenz, beispielsweise HATU oder CDI, ggf. unter Zusatz einer Base, erreicht werden.

In den Schritten **w04** und **w15** können die Thiole **S-II** und **S-X** in die entsprechenden Thioether **S-V** und **S-XII** mittels dem Fachmann geläufigen Methoden überführt werden. Hierbei können die Thiole **S-II** und **S-X** bspw. jeweils durch Einsatz des Alkylhalogenids $R^8$-Hal alkyliert werden, ggf. in Gegenwart einer Base.

In den Schritten **w03, w08** und **w12** können die Thioether **S-V, S-VIII** und **S-XII** ausgehend von den 2-Halogen-chinolinen **S-I, S-IV** und **S-VII,** worin X jeweils für Halogen, bevorzugt für Fluor oder Chlor steht, mittels dem Fachmann geläufigen Methoden gebildet werden, z.B. durch jeweils durch Alkylierung mit dem entsprechenden Thiol $R^8$-SH in

einer ipso-Substitution, ggf. in Gegenwart einer Base.

In den Schritten **w06** und **w11** können die primären Amine **S-III, S-IX** mittels dem Fachmann bekannten Methoden, wie bspw. reduktive Aminierung mit den entsprechenden Aldehyden oder Ketonen unter Zusatz eines geeigneten Reduktionsmittels, in die Verbindungen **S-IV** und **S-VIII** überführt werden.

Im Schritt **w16** kann das Amid **S-XII** zur Verbindung **S-XI** mittels dem Fachmann bekannten Methoden unter Verwendung geeigneter Alkylierungsmittel, ggf. in Gegenwart einer Base, N-alkyliert werden.

Die dem Fachmann geläufigen Methoden zur Durchführung der Reaktionsschritte **w01** bis **w16** sind den Standardwerken der Organischen Chemie zu entnehmen wie bspw. J. March, Advanced Organic Chemistry, Wiley & Sons, 6th edition, 2007; F. A. Carey, R. J. Sundberg, Advanced Organic Chemistry, Parts A and B, Springer, 5th edition, 2007); Autorenkollektiv, Compendium of Organic Synthetic Methods, Wiley & Sons. Darüber hinaus können weitere Methoden sowie Literaturverweise von den gängigen Datenbanken wie der Reaxys® Datenbank der Firma Elsevier, Amsterdam, NL oder der SciFinder® Datenbank der American Chemical Society, Washington, US, ausgegeben werden.

**<u>Beschreibung der Beispielsynthesen</u>**

**<u>Abkürzungen</u>**

| | |
|---|---|
| AcOH | Essigsäure |
| aq. | wässrig |
| Brine | ges. aq. NaCl- Lsg. |
| BuLi | n- Butyllithium |
| d | Tage |
| DCM | Dichlormethan |
| DIPEA | N, N- Diisopropylethylamin |
| DMF | N, N- Dimethylformamid |
| EE | Ethylacetat |
| ges. | gesättigt |
| h | Stunde (n) |
| konz. | konzentriert |
| Lsg. | Lösung |
| m/z | Verhältnis Masse zur Ladung |
| M | molar |
| MeCN | Acetonitril |
| MeOH | Methanol |
| min | Minuten |
| MS | Massenspektrometrie |
| N/A | nicht verfügbar |
| $NEt_3$ | Triethylamin |
| RG | Retigabine |
| RT | Raumtemperatur 23 ± 7 °C |
| SC | Säulenchromatographie auf Kieselgel |
| THF | Tetrahydrofuran |
| vv | Volumenverhältnis |

Alle nicht explizit beschriebenen Ausgangsstoffe waren entweder kommerziell verfügbar (Anbieter können beispielsweise in der Symyx® Available Chemicals Database der Firma MDL, San Ramon, US recherchiert werden) oder deren Synthese ist in der Fachliteratur bereits exakt beschrieben (Experimentelle Vorschriften können beispielweise in der Reaxys® Datenbank der Firma Elsevier, Amsterdam, NL recherchiert werden) oder können nach den dem Fachmann bekannten Methoden hergestellt werden.

Als stationäre Phase für die Säulenchromatographie (SC) wurde Kieselgel 60 (0.040 - 0.063 mm) eingesetzt.

Die analytische Charakterisierung aller Zwischenprodukte und Beispielverbindungen erfolgte mittels $^1$H-NMR-Spektroskopie. Zusätzlich wurden für alle Beispielverbindungen und ausgewählte Zwischenprodukte massenspektrometrische Untersuchungen (MS, Angabe m/z für $[M+H]^+$) durchgeführt.

## Synthese von Zwischenprodukten

### Synthese des Zwischenprodukts VA01: 2-(Phenylsulfonyl)ethanthiol

a) Synthese von S-2-(Phenylsulfonyl)ethyl ethanthiolat

Eine Lösung von 10.0 g (48.9 mmol) (2- Chlorethylsulfonyl) benzol in Benzol (180 ml) wurde mit (3.6 ml, 25.8 mmol) $NEt_3$ und 3.5 ml (49.0 mmol) Thioessigsäure versetzt und anschließend 3 h auf 80 °C erhitzt. Danach wurde mit EE verdünnt und mit Wasser und Brine gewaschen. Die organische Phase wurde über $MgSO_4$ getrocknet, filtriert und im Vakuum eingeengt. Als Rückstand wurden 11, 6 g (47, 5 mmol, 97%) S- 2- (Phenylsulfonyl)- ethyl ethanthiolat erhalten, das ohne zusätzliche Aufreinigung weiter umgesetzt wurde.

b) Synthese von 2-(Phenylsulfonyl)ethanthiol

Eine Lösung von 11, 6 g (47, 5 mmol) S- 2- (Phenylsulfonyl)- ethyl ethanthiolat in 10% methanolischer Salzsäure wurde 24 h auf 80 °C erhitzt. Anschließend wurde im Vakuum eingeengt. Der Rückstand wurde mit EE aufgenommen und es wurde mit Wasser und Brine gewaschen. Die organische Phase wurde über $MgSO_4$ getrocknet, filtriert und im Vakuum eingeengt. Als Rückstand wurden 9, 0 g (44, 5 mmol, 94%) 2- (Phenylsulfonyl) ethanthiol erhalten, das ohne Aufreinigung weiter umgesetzt wurde.

### Synthese des Zwischenprodukts VB01: 3-Nitro-2-(2-(phenylsulfonyl)ethylthio)chinolin

Eine Lösung von 1, 21 g (5, 8 mmol) 2- Chlor- 3- nitrochinolin in THF (30 ml) wurde nacheinander mit 1, 76 g (8.7 mmoi) Zwischenprodukt VA01 und 0, 98 g (8.7 mmol) Kalium- tert.- Butylat versetzt und 16 h bei RT gerührt. Danach wurde die Reaktionslösung mit EE verdünnt und mit Wasser und Brine gewaschen. Die organische Phase wurde über $Na_2SO_4$ getrocknet, filtriert und im Vakuum eingeengt. Durch Kristallisation (DCM / Hexan) wurden 539 mg (1, 4 mmol, 25%) 3- Nitro- 2- (2- (phenylsulfonyl) ethylthio) chinolin erhalten.

### Synthese des Zwischenprodukts VC01: 2-(2-(Phenylsulfonyl)ethylthio)chinolin-3-amin

Eine Lösung von 700 mg (1, 87 mmol) Zwischenprodukt VB01 in MeOH (10 ml) wurden mit 290 mg (5, 2 mmol) Eisenpulver versetzt und auf 0 °C abgekühlt. Anschließend wurden 3, 7 ml konz. Salzsäure zugetropft, wobei die Temperatur bei 0- 5 °C gehalten wurde. Danach wurde 3 h auf 70 °C erhitzt und nach Abkühlen der Reaktionslösung auf RT wurde durch Kieselgur filtriert. Das Filtrat wurde im Vakuum aufkonzentriert und mit einer aq. $NaHCO_3$- Lsg basisch gestellt. Danach wurde mit EE extrahiert und die organische Phase wurde über $Na_2SO_4$ getrocknet, filtriert und im Vakuum eingeengt. Durch SC (EE / Hexan 4: 1) wurden 341 mg (0, 99 mmol, 53%) 2- (2- (Phenylsulfonyl) ethylthio) chinolin- 3- amin erhalten.

### Synthese des Zwischenprodukts VC05: 2-Chlor-7-(trifluormethyl)chinolin-3-amin

a) Synthese von 2-Chlor-7-(trifluormethyl)chinolin-3-carbonsäure

Zu einer Lösung von 1, 9 ml (11 mmol) DIPEA in THF (77 ml) wurden bei- 78 °C 11 ml BuLi (1 M in Hexan) getropft. Nach 30 min Rühren bei- 78 °C wurde eine Lösung von 2, 31 g (10 mmol) 2- Chlor- 7- (trifluormethyl) chinolin in THF (30 ml) zugegeben und es wurde weitere 30 min bei- 78 °C gerührt. Danach wurde die Reaktionslösung auf fein verteiltes, festes $CO_2$ gegossen. Nach Erwärmen auf RT wurde das THF größtenteils im Vakuum entfernt. Dann wurde mit einer 1 M aq. NaOH- Lsg versetzt und die Phasen wurden getrennt. Die wässrige Phase wurde mit 10%- Salzsäure angesäuert und mit EE extrahiert. Die organische Phase wurde mit Wasser und Brine gewaschen, über $Na_2SO_4$ getrocknet, filtriert und im Vakuum eingeengt. Als Rückstand wurden 2, 12 g (7, 7 mmol, 71%) 2- Chlor- 7- (trifluormethyl) chinolin- 3- carbonsäure erhalten, die ohne zusätzliche Aufreinigung weiter umgesetzt wurden.

b) Synthese von 2-Chlor-7-(trifluormethyl)chinolin-3-amin

Eine Lösung von 1, 4 g (5 mmol) 2- Chlor- 7- (trifluormethyl) chinolin- 3- carbonsäure in Benzol (500 ml) wurde bei RT mit 20, 6 g (75 mmol) Diphenylphophorylazid versetzt und anschließend 5 h auf 90 °C erhitzt. Dann wurde im Vakuum eingeengt und der Rückstand wurde mit THF (80 ml) aufgenommen. Diese Lösung wurde mit 4N aq. LiOH-Lsg (30 ml) versetzt und 1 h bei RT gerührt. Anschließend wurde mit Wasser verdünnt und mit EE extrahiert. Die organische Phase wurde mit Wasser und Brine gewaschen, über $Na_2SO_4$ getrocknet, filtriert und im Vakuum eingeengt.

Durch SC (Hexan / EE 9: 1) des Rückstand wurden 310 mg (1, 3 mmol, 26%) 2- Chlor- 7- (trifluormethyl) chinolin- 3-amin erhalten.

**Synthese des Zwischenprodukts VC06: 2-(Ethylthio)-4-methyl-7-(trifluormethyl)chinolin-3-amin**

a) Synthese von 2-(2-Chloro-4-methylchinolin-3-yl)isoindolin-1,3-dion

Eine Lösung von 2, 7 g (6, 9 mmol) N- (2- acetylphenyl)- 2- (1, 3- dioxoisoindolin- 2- yl)- acetamid in DMF (27 ml) wurde mit 1, 4 g (10, 4 mmol) $K_2CO_3$ versetzt und 16 h auf 60 °C erhitzt. Nach Abkühlen auf RT wurde mit 2N Salzsäure auf pH 2- 3 eingestellt. Der enstandene Niederschlag wurde abgesaugt und im Vakuum bei 70°C getrocknet. Der Rückstand wurde mit 9 ml (97 mmol) $POCl_3$ versetzt. Diese Lösung wurde 2 h auf 100 °C erhitzt und anschließend noch 16 h bei RT gerührt. Danach wurde mit Toluol versetzt und im Vakuum eingeengt. Diese Vorgand wurde wiederholt wobei als Rückstand 2, 13 g (5, 5 mmol, 79%) 2- (2- Chloro- 4- methylchinolin- 3- yl) isoindolin- 1, 3- dion erhalten wurden, die ohne zusätzliche Aufreinigung weiter umgesetzt wurden.

b) Synthese von 2-(2-(Ethylthio)-4-methylchinolin-3-yl)isoindolin-1,3-dion

Eine Lösung von 2, 1 g (5, 1 mmol) 2- (2- Chloro- 4- methylchinolin- 3- yl) isoindolin- 1, 3- dion in DMF (36 ml) wurde nacheinander mit 2, 1 g (15, 4 mmol) $K_2CO_3$ und 760 $\mu$l (10, 3 mmol) Ethanthiol versetzt. Anschließend wurde 16 h auf 60 °C erhitzt. Danach wurden erneut 2, 1 g (15, 4 mmol) $K_2CO_3$ und 760 $\mu$l (10, 3 mmol) Ethanthiol zugegeben und es wurde nochmals 16 h auf 50 °C erhitzt. Nach Abkühlen auf RT wurde mit Wasser verdünnt und mit zweimal mit EE extrahiert. Die vereinten organischen Phasen wurden mit Brine gewaschen, über $MgSO_4$ getrocknet, filtriert und im Vakuum eingeengt. Als Rückstand wurden 1, 7 g (4, 0 mmol, 78%) 2- (2- (Ethylthio)- 4- methylchinolin- 3- yl) isoindolin-1, 3- dion erhalten, die ohne zusätzliche Aufreinigung weiter umgesetzt wurden.

c) Synthese von 2-(Ethylthio)-4-methyl-7-(trifluormethyl)chinolin-3-amin

Eine Lösung von 1, 67 g (4, 0 mmol) 2- (2- (Ethylthio)- 4- methylchinolin- 3- yl) isoindolin- 1, 3- dion und 0, 5 g (8, 0 mmol) Hydrazinhydrat in EtOH (60 ml) wurde 4 h auf 70 °C erhitzt und anschließend 16 h bei RT gerührt. Danach wurde die Reaktionslösung mit EE (2 x 50 ml) extrahiert und die vereinten organischen Phasen wurden mit Brine gewaschen, über $MgSO_4$ getrocknet, filtriert und im Vakuum eingeengt. Nach SC (Hexan / EE 3: 1) mit dem Rückstand wurden 656 mg (2, 3 mmol, 57%) 2- (Ethylthio)- 4- methyl- 7- (trifluormethyl) chinolin- 3- amin erhalten.

**Synthese weiterer Zwischenprodukte**

Die Synthese weiterer Zwischenprodukte erfolgte nach den bereits beschriebenen Verfahren. In Tabelle 1 ist angegeben, welche Verbindung nach welchem Verfahren hergestellt wurde. Dem Fachmann ist dabei ersichtlich, welche Edukte und Reagenzien jeweils eingesetzt wurden.

**Tabelle 1:**

| Zwischenprodukt | Chemische Bezeichnung | Herstellung analog Zwischenprodukt | Ausbeute [%] |
|---|---|---|---|
| **VB02** | 3-Nitro-2-(pentylthio)chinolin | VB01 | 75 |
| **VC02** | 2-(Pentylthio)chinolin-3-amin | VC01 | 78 |
| **VC03** | 2-(2-(Phenylthio)ethylthio)chinolin-3-amin | VC01 | 63 |
| **VC04** | 2-(Ethylthio)chinolin-3-amin | VC01 | 55 |

**Synthese der Beispielverbindungen**

**Synthese der Beispielverbindung 3: 3-Cyclohexyl-N-(2-(2-(phenylsulfonyl)-ethylthio)chinolin-3-yl)propanamid**

Eine Lösung von 250 mg (0, 73 mmol) Zwischenprodukts VC01 in DCM (4 ml) wurde mit 255 $\mu$l (2, 6 mmol) DIPEA versetzt und auf 0 °C abgekühlt. Anschließend wurden 128 mg (0, 73 mmol) 3- Cyclohexyl- propansäurechlorid zugegeben. Danach wurde die Reaktionslösung 16 h bei RT gerührt und anschließend wurde mit DCM verdünnt und

mit einer ges. aq. $Na_2CO_3$- Lsg und Brine gewaschen. Die organische Phase wurde über $Na_2SO_4$ getrocknet, filtriert und im Vakuum eingeengt. Durch SC (EE / Hexan 4: 1) wurden 162 mg (0, 34 mmol, 46%) 3- Cyclohexyl- N- (2- (2- (phenylsulfonyl)- ethylthio) chinolin- 3- yl) propanamid erhalten. MS: m/z 483, 2 $[M+H]^+$.

**Synthese der Beispielverbindung 10: N-[2-Ethylsulfanyl-7-(trifluormethyl)-chinolin-3-yl]-2-thiophen-2-yl-acetamid**

a) Synthese von N-(2-Chlor-7-(trifluormethyl)chinolin-3-yl)-2-(thiophen-2-yl)acetamid

Eine Lösung von 493 mg (2 mmol) 2- Chlor- 7- (trifluormethyl) chinolin- 3- amin (VC05) in DCM (14 ml) wurde bei 0 °C nacheinander mit 373 µl (4 mmol) DIPEA und 385 mg (2, 4 mmol) 2- (thiophen- 2- yl) acetylchlorid versetzt. Nach 16 h Rühren bei RT wurde mit DCM verdünnt. Dann wurde mit Wasser und Brine gewaschen, über $Na_2SO_4$ getrocknet, filtriert und im Vakuum eingeengt. Nach SC (Hexan / EE 19: 1) des Rückstands wurden 321 mg (0, 87 mmol, 44%) N- (2- Chlor- 7- (trifluormethyl) chinolin- 3- yl)- 2- (thiophen- 2- yl) acetamid erhalten.

b) Synthese von N-[2-Ethylsulfanyl-7-(trifluormethyl)-chinolin-3-yl]-2-thiophen-2-yl-acetamid

Eine Lösung von 300 mg (0, 81 mmol) %) N- (2- Chlor- 7- (trifluormethyl) chinolin- 3- yl)- 2- (thiophen- 2- yl) acetamid in DMF (6 ml) wurde mit 331 mg (2, 4 mmol) $K_2CO_3$ und 40 mg (1, 6 mmoi) Ethanthiol versetzt und 16 h auf 60 °C erhitzt. Danch wurde mit Wasser verdünnt und mit EE extrahiert. Die organische Phase wurde mit Wasser und Brine gewaschen, über $Na_2SO_4$ getrocknet, filtriert und im Vakuum eingeengt. Durch SC (Hexan / EE 9: 1) des Rückstands wurden 242 mg (0, 6 mmol, 74%) N- [2- Ethylsulfanyl- 7- (trifluormethyl)- chinolin- 3- yl]- 2- thiophen- 2- yl- acetamid erhalten. MS: m/z 397, 1 $[M+H]^+$.

**Synthese der Beispielverbindung 13: 2-Cyclohexyl-N-(2-ethylsulfanyl-chinolin-3-yl)-acetamid**

Eine Suspension von 427 mg (3 mmol) 2- Cyclohexylessigsäure in DCM (45 ml) wurde bei 0 °C mit 760 mg (6 mmol) Oxalylchlorid und einer katalytischen Menge DMF (100 µl) versetzt. Nach 4 h Rühren bei RT wurde im Vakuum eingeengt. Der Rückstand wurde mit Dioxan (30 ml) aufgenommen und mit 670 mg (8 mmol) $NaHCO_3$ versetzt. Nach 5 min Rühren bei RT wurden 408 mg (2 mmol) 2- (Ethylthio) chinolin- 3- amin (VC04) zugegeben. Nach 16 h Rühren bei RT wurde die Reaktionslösung mit EE verdünnt und mit einer ges. aq. $NaHCO_3$- Lsg und Brine gewaschen, über $Na_2SO_4$ getrocknet, filtriert und im Vakuum eingeengt. Durch SC (Hexan / EE 19: 1) des Rückstands wurden 397 mg (1, 0 mmol, 51%) 2- Cyclohexyl- N- (2- ethylsulfanyl- chinolin- 3- yl)- acetamid erhalten. MS: m/z 329, 2 $[M+H]^+$.

**Synthese weiterer Beispielverbindungen**

Die Synthese weiterer Beispielverbindungen erfolgte nach den bereits beschriebenen Verfahren. In Tabelle 2 ist angegeben, welche Verbindung nach welchem Verfahren hergestellt wurden. Dem Fachmann ist dabei ersichtlich, welche Edukte und Reagenzien jeweils eingesetzt wurden.

**Tabelle 2:**

| Beispiel | Chemische Bezeichnung | Herstellung analog Beispiel | Ausbeute [%] | MS m/z $[M+H]^+$ |
|---|---|---|---|---|
| **1** | 2-Cyclohexyl-N-(2-(2-(phenylsulfonyl) ethylthio)chinolin-3-yl) acetamid | 3 | 51 | 469,2 |
| **2** | N-(2-(2-(Phenylsulfonyl) ethylthio)chinolin-3-yl)-2-(thiophen-2-yl)acetamid | 3 | 73 | 469,1 |
| **4** | N-(2-(Pentylthio)chinolin-3-yl)-2-(thiophen-2-yl)acetamid | 3 | 20 | 371,1 |
| **6** | N-(2-(2-(Phenylthio)ethylthio) chinolin-3-yl)-2-(thiophen-2-yl)acetamid | 3 | 71 | 437,1 |

(fortgesetzt)

| Beispiel | Chemische Bezeichnung | Herstellung analog Beispiel | Ausbeute [%] | MS m/z $[M+H]^+$ |
|---|---|---|---|---|
| **7** | N-(2-(Ethylthio)chinolin-3-yl)-2-(thiophen-2-yl)acetamid | 3 | 60 | 329,1 |
| **9** | N-(2-(Ethylthio)chinolin-3-yl)-3,3-dimethylbutanamid | 3 | 60 | 303,1 |
| **11** | N-[2-Ethylsulfanyl-7-(trifluormethyl)- chinolin-3-yl]-3,3-dimethyl-butyramid | 10 | 31 (über 2 Stufen) | 371,1 |
| **12** | 3-Cyclopentyl-N-[2-ethylsulfanyl-7-(trifluormethyl)- chinolin-3-yl]-propionamid | 10 | 38 (über 2 Stufen) | 397,1 |
| **14** | 2-(5-Bicyclo[2.2.1] heptanyl)-N-(2-ethylsulfanyl-chinolin-3-yl)-acetamid | 13 | 28 | 341,2 |
| **15** | 3-Cyclopentyl-N-(2-ethylsulfanyl-chinolin-3-yl)-propionamid | 13 | 56 | 329,2 |
| **16** | 2-(5-Bicyclo[2.2.1] heptanyl)-N-[2-ethylsulfanyl-7-(trifluormethyl)- chinolin-3-yl]-acetamid | 10 | 6 (über 2 Stufen) | 409,1 |
| **17** | 3-Cyclopentyl-N-[2-ethylsulfanyl-4-methyl-7-(trifluormethyl)- chinolin-3-yl]-propionamid | 3 | 18 | 411,2 |
| **18** | N-[2-Ethylsulfanyl-4-methyl-7-(trifluormethyl)- chinolin-3-yl]-2-thiophen-2-yl-acetamid | 3 | 24 | 411,1 |

## Pharmakologische Experimente

### Fluoreszenzassay unter Verwendung eines ’voltage sensitive dyes’

Humane, KCNQ2/3- Kanäle exprimierende CHO- K1- Zellen werden in Zellkulturflaschen (z.B. 80 $cm^2$ TC flasks, Nunc) mit DMEM- high glucose (Sigma Aldrich, D7777) inklusive 10% FCS (PAN Biotech, z.B. 3302- P270521) oder alternativ MEM Alpha Medium (1x, flüssig, Invitrogen, #22571) , 10 % Fetal Calf Serum (FCS) (Invitrogen, #10270-106, hitzeinaktiviert) und den notwendigen Selektionsantibiotika bei 37°C, 5 % $CO_2$ und 95 % Luftfeuchtigkeit adhärent kultiviert.

Vor Aussaat für die Messungen werden die Zellen mit einem 1 x DPBS-Puffer ohne $Ca^{2+}/Mg^{2+}$ (z.B. Invitrogen, #14190-094) gewaschen und mittels Accutase (PAA Laboratories, #L11-007) vom Boden des Kulturgefäßes abgelöst (Inkubation mit Accutase für 15 min bei 37°C). Die Bestimmung der dann vorliegenden Zellzahl wird mit einem CASY™ cell counter (Modell TCC, Schärfe System) durchgeführt, um anschließend je nach Dichteoptimierung für die individuelle Zelllinie 20.000 - 30.000 Zellen/well/100 μl des beschriebenen Nährmediums auf die 96 well-Messplatten des Typs Corning™ CellBIND™ (Flat Clear Bottom Black Polystyrene Microplates, #3340) auszubringen. Danach erfolgt eine einstündige Inkubation bei Raumtemperatur ohne Begasung oder Regelung der Luftfeuchtigkeit, gefolgt von einer 24stündigen Inkubation bei 37°C, 5 % $CO_2$ und 95 % Luftfeuchtigkeit.

Der spannungssensitive Fluoreszenzfarbstoff aus dem Membrane Potential Assay Kit (Red™ Bulk format part R8123 for FLIPR, MDS Analytical Technologies™) wird vorbereitet, indem der Inhalt eines Gefäßes *Membrane Potential Assay Kit Red Component A* in 200 ml Extrazellulären Puffers (ES- Puffer, 120 mM NaCl, 1 mM KCl, 10 mM HEPES, 2 mM $CaCm_2$, 2 mM $MgCl_2$, 10 mM Glucose; pH 7, 4) gelöst wird. Nach Abnahme des Nährmediums werden die Zellen mit 200 μl ES- Puffer gewaschen, anschließend mit 100 μl der oben angesetzten Farbstofflösung überschichtet und 45

min bei Raumtemperatur unter Lichtverschluss inkubiert.

Die Fluoreszenzmessungen werden mit einem BMG Labtech FLUOstar™-, BMG Labtech NOVOstar™- oder BMG Labtech POLARstar™-Instrument durchgeführt (525 nm Exication, 560 nm Emission, Bottom Read mode). Nach der Farbstoff-Inkubation werden 50 μl der zu testenden Substanzen in den gewünschten Konzentrationen oder 50 μl ES-Puffer zwecks Kontrolle in separate Cavitäten der Messplatte gegeben und 30 min bei Raumtemperatur unter Abschirmung von Licht inkubiert. Anschließend misst man für 5 min die Fluoreszenzintensität des Farbstoffs und ermittelt so zu einem festgelegten und gleichbleibenden Zeitpunkt den Fluoreszenzwert $F_1$ eines jeden wells. Daraufhin erfolgt Zugabe von 15 μl einer 100 mM KCl-Lösung (Endkonzentration 92 mM) in jedes well. Die Veränderung der Fluoreszenz wird anschließend so lange gemessen, bis alle relevanten Messwerte erhalten sind (vornehmlich 5-30 min). Zu einem festgelegten Zeitpunkt nach KCl-Applikation wird ein Fluoreszenzwert $F_2$ ermittelt, in diesem Falle zum Zeitpunkt des Fluoreszenzpeaks.

Zur Berechnung wird die Fluoreszenzintensität $F_2$ mit der Fluoreszenzintensität $F_1$ verglichen und daraus die agonistische Aktivität der Zielverbindung auf den Kaliumkanal ermittelt. $F_2$ und $F_1$ werden hierfür wie folgt verrechnet:

$$\left(\frac{F_2 - F_1}{F_1}\right) \times 100 = \frac{\Delta F}{F}(\%)$$

Um zu ermitteln, ob eine Substanz eine agonistische Aktivität besitzt, kann z.B. $\frac{\Delta F}{F}$ F mit $\left(\frac{\Delta F}{F}\right)_K$ von Kontrollzellen verglichen werden. $\left(\frac{\Delta F}{F}\right)_K$ wird ermittelt, indem man dem Reaktionsansatz anstatt der zu testenden Substanz lediglich die Pufferlösung zusetzt, den Wert $F_{1K}$ der Fluoreszenzintensität bestimmt, die Kaliumionen wie oben beschrieben zugibt und einen Wert $F_{2K}$ der Fluoreszenzintensität misst. Dann werden $F_{2K}$ und $F_{1K}$ wie folgt verrechnet:

$$\left(\frac{F_{2K} - F_{1K}}{F_{1K}}\right) \times 100 = \left(\frac{\Delta F}{F}\right)_K (\%)$$

Eine Substanz besitzt eine agonistische Aktivität auf den Kaliumkanal, wenn $\frac{\Delta F}{F}$ größer als $\left(\frac{\Delta F}{F}\right)_K$ ist:

$$\frac{\Delta F}{F} \rangle \left(\frac{\Delta F}{F}\right)_K$$

Unabhängig vom Vergleich von $\frac{\Delta F}{F}$ mit $\left(\frac{\Delta F}{F}\right)_K$ lässt sich auch auf eine agonistische Aktivität einer Zielverbindung schließen, wenn mit steigender Dosierung der Zielverbindung eine Zunahme von $\frac{\Delta F}{F}$ zu beobachten ist. Kalkulationen von $EC_{50}$-Werten werden mit Hilfe der Software 'Prism v4.0' (GraphPad Software™) durchgeführt.

**Formalintest Maus**

Der Formalin Test (Dubuisson, D. and Dennis, S.G., 1977, Pain, 4, 161 - 174) stellt ein Modell für den akuten sowie den chronischen Schmerz dar. Durch eine einmalige Formalin-Injektion in die dorsale Seite einer Hinterpfote wird bei freibeweglichen Versuchstieren eine biphasische nozizeptive Reaktion induziert, die durch Beobachtung von drei

deutlich voneinander unterscheidbaren Verhaltensmustern erfasst wird. Die Reaktion ist zweiphasisch: Phase 1 = Sofortreaktion (Dauer bis 10 min; Pfotenschütteln, Lecken), Phase 2 = Spätreaktion (nach einer Ruhephase; ebenfalls Pfotenschütteln, Lecken; Dauer bis 60 min). Die 1. Phase reflektiert eine direkte Stimulation der peripheren Nozisensoren mit hohem spinalen nozizeptiven Input (akute Schmerzphase); die 2. Phase reflektiert eine spinale und periphere Hypersensibilisierung (chronische Schmerzphase). In den hier vorgestellten Untersuchungen wurde die chronische Schmerzkomponente (Phase 2) ausgewertet.

Formalin wird mit dem Volumen von 20 $\mu$l und einer Konzentration von 1 % subkutan in die dorsale Seite der rechten Hinterpfote jedes Tieres appliziert. Die spezifischen Verhaltensänderungen, wie Anheben, Schütteln oder Lecken der Pfote (score 3, Dubuisson & Dennis, 1977), werden im Beobachtungszeitraum von 21 bis 24 min nach Formalininjektion beobachtet und registriert. Das Verhalten der Tiere nach Substanzgabe (n = 10 pro Substanzdosierung) wurde mit einer Kontrollgruppe, welcher Vehikel appliziert wurde (n=10) verglichen.

Basierend auf der Quantifizierung des Schmerzverhaltens wurde die Substanzwirkung im Formalin-Test als Änderung gegenüber der Kontrolle in Prozent ermittelt. Die Berechnungen der $ED_{50}$ ($ED_{50}$ = mittlere effektive Dosis) erfolgten mittels Regressionsanalyse nach der Methode von Litchfield and Wilcoxon (Litchfield, J.T., Wilcoxon, J.J., 1949, J. Pharmacol. Exp. Ther. 96, 99 - 113). Der Applikationszeitpunkt der Verbindung vor der Formalin-Injektion war bei intravenöser Applikation 5 min und bei oraler Applikation 30 min.

**Pharmakologische Daten**

In Tabelle 3 sind die Ergebnisse aus den zuvor beschriebenen pharmakologischen Modellen zusammengefasst.

Tabelle 3

| Bsp-Nr. | Fluorimetrie EC50 [nM] | Fluorimetrie % Efficacy (Retigabine = 100%) | Formalin Test Maus IV Effekt @ Dosis [mg/kg] |
|---|---|---|---|
| **1** | 253 | 80 | |
| **2** | 67 | 80 | |
| **3** | 174 | 97 | |
| **4** | 107 | 60 | |
| **6** | | 25 | |
| **7** | 156 | 93 | 24% @ 1 |
| **9** | 414 | 142 | |
| **10** | 1153 | 57 | |
| **11** | | 35 | |
| **12** | 5024 | 62 | |
| **13** | 150 | 101 | |
| **14** | 204 | 179 | |
| **15** | 82 | 143 | 17% @ 1 |
| **16** | 647 | 66 | |
| **17** | 66 | 107 | 90% @ 0,68 |
| **18** | 94 | 140 | 43% @ 1 |

**Patentansprüche**

1. Substituierte 3-Amino-2-mercaptochinoline der allgemeinen Formel (1)

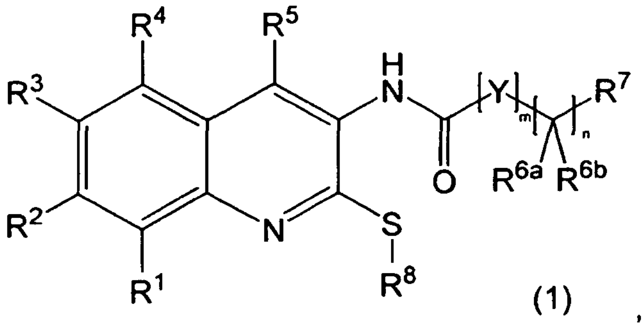

(1) ,

worin

m für 0 oder 1 und
n für eine ganze Zahl von 0 bis 4 steht,
$R^1$ $R^2$, $R^3$, $R^4$ $R^{6a}$, $R^{6b}$ jeweils unabhängig voneinander für H; F; Cl; Br; I; $NO_2$; $CF_3$; CN; OH; $OCF_3$; SH; $SCF_3$; $NH_2$; $C_{1-10}$-Alkyl, O-$C_{1-10}$-Alkyl, O-C(=O)-$C_{1-10}$-Alkyl, S-$C_{110}$-Alkyl, NH($C_{1-10}$-Alkyl), N($C_{1-10}$-Alkyl)$_2$, NH-C(=O)-$C_{1-10}$-Alkyl, N(C(=O)-$C_{1-10}$-Alkyl)$_2$ oder C(=O)-$C_{1-10}$-Alkyl, jeweils gesättigt oder ungesättigt, verzweigt oder unverzweigt, unsubstituiert oder einfach oder mehrfach substituiert; $C_{3-8}$-Cycloalkyl oder -Heterocyclyl, jeweils gesättigt oder ungesättigt, unsubstituiert oder einfach oder mehrfach substituiert; stehen,
$R^5$ für H; F; Cl; Br; I; $NO_2$; $CF_3$; CN; $OCF_3$; SH; $SCF_3$; $NH_2$; $C_{1-10}$-Alkyl, O-$C_{1-10}$-Alkyl, O-C(=O)-$C_{1-10}$-Alkyl, S-$C_{1-10}$-Alkyl, NH($C_{1-10}$-Alkyl), N($C_{1-10}$-Alkyl)$_2$, NH-C(=O)-$C_{1-10}$-Alkyl, N(C(=O)-$C_{1-10}$-Alkyl)$_2$ oder C(=O)-$C_{1-10}$-Alkyl, jeweils gesättigt oder ungesättigt, verzweigt oder unverzweigt, unsubstituiert oder einfach oder mehrfach substituiert; $C_{3-8}$-Cycloalkyl oder -Heterocyclyl, jeweils gesättigt oder ungesättigt, unsubstituiert oder einfach oder mehrfach substituiert; steht,
Y für O oder $NR^9$ steht,
wobei $R^9$ für H oder $C_{1-4}$-Alkyl gesättigt oder ungesättigt, verzweigt oder unverzweigt, unsubstituiert oder einfach oder mehrfach substituiert steht,
$R^7$ für $C_{1-10}$-Alkyl oder $C_{2-10}$-Heteroalkyl, gesättigt oder ungesättigt, verzweigt oder unverzweigt, unsubstituiert oder einfach oder mehrfach substituiert; $C_{3-10}$-Cycloalkyl oder- Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert steht;
mit der Maßgabe, dass, wenn $R^7$ Heterocyclyl bedeutet, die Bindung des Heterocyclyls an die übergeordnete allgemeine Struktur über ein Kohlenstoffatom des Heterocyclyls erfolgen kann und
mit der Maßgabe, dass, wenn $R^7$ Aryl oder Heteroaryl bedeutet, die Summe von n und m größer oder gleich 1 beträgt;
$R^8$ ausgewählt ist aus der Gruppe bestehend aus $C_{1-10}$-Alkyl oder $C_{2-10}$-Heteroalkyl, jeweils gesättigt oder ungesättigt, verzweigt oder unverzweigt, unsubstituiert oder einfach oder mehrfach substituiert; $C_{3-10}$-Cycloalkyl oder -Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$-Alkyl oder $C_{2-8}$-Heteroalkyl verbrücktes $C_{3-10}$-Cycloalkyl oder -Heterocyclyl, jeweils gesättigt oder ungesättigt, unsubstituiert oder einfach oder mehrfach substituiert; oder über $C_{1-8}$-Alkyl oder $C_{2-8}$-Heteroalkyl verbrücktes Aryl oder Heteroaryl, jeweils unsubstituiert oder einfach oder mehrfach substituiert, wobei die Alkyl- oder Heteroalkylkette jeweils verzweigt oder unverzweigt, gesättigt oder ungesättigt, unsubstituiert sein kann;
worin "Alkyl substituiert", "Heteroalkyl substituiert", "Heterocyclyl substituiert" und "Cycloalkyl substituiert" für die Substitution eines oder mehrerer Wasserstoffatome jeweils unabhängig voneinander durch F; Cl; Br; I; CN; $CF_3$; =O; =NH; =C($NH_2$)$_2$; $NO_2$; $R^0$; C(=O)H; C(=O)$R^0$; $CO_2$H; C(=O)O$R^0$; CON$H_2$; C(=O)NH$R^0$; C(=O)N($R^0$)$_2$; OH; O$R^0$; -O-($C_{1-8}$-Alkyl)-O-; O-C(=O)-$R^0$; O-C(=O)-O-$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2$N$H_2$; O-S(=O)$_2$NH$R^0$; O-S(=O)$_2$N($R^0$)$_2$; $NH_2$; NH-$R^0$; N($R^0$)$_2$; NH-C(=O)-$R^0$; NH-C(=O)-O-$R^0$; NH-C(=O)-N$H_2$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-N$H_2$; N$R^0$-C(=O)-NH-$R^0$; N$R^0$-C(=O)-N($R^0$)$_2$; NH-S(=O)$_2$OH; NH-S(=O)$_2$$R^0$; NH-S(=O)$_2$O$R^0$; NH-S(=O)$_2$N$H_2$; NH-S(=O)$_2$NH$R^0$; NH-S(=O)$_2$N($R^0$)$_2$; N$R^0$-S(=O)$_2$OH; N$R^0$-S(=O)$_2$$R^0$; N$R^0$-S(=O)$_2$O$R^0$; N$R^0$-S(=O)$_2$N$H_2$; N$R^0$-S(=O)$_2$NH$R^0$; N$R^0$-S(=O)$_2$N($R^0$)$_2$; SH; S$R^0$; S(=O)$R^0$; S(=O)$_2$$R^0$; S(=O)$_2$H; S(=O)$_2$OH; S(=O)$_2$O$R^0$; S(=O)$_2$N$H_2$; S(=O)$_2$NH$R^0$; S(=O)$_2$N($R^0$)$_2$; steht;
worin "Aryl substituiert" und "Heteroaryl substituiert" für die Substitution eines oder mehrerer Wasserstoffatome jeweils unabhängig voneinander durch F; Cl; Br; I; $NO_2$; $CF_3$; CN; $R^0$; C(=O)H; C(=O)$R^0$; $CO_2$H; C(=O)O$R^0$; CON$H_2$; C(=O)NH$R^0$; C(=O)N($R^0$)$_2$; OH; O$R^0$; -O-($C_{1-8}$-Alkyl)-O-; O-C(=O)-$R^0$; O-C(=O)-O-$R^0$; O-(C=O)-NH-

$R^0$; O-C(=O)-N($R^0$)$_2$; O-S(=O)$_2$-$R^0$; O-S(=O)$_2$OH; O-S(=O)$_2$O$R^0$; O-S(=O)$_2$N$H_2$; 0-S(=O)$_2$NH$R^0$; O-S(=O)$_2$N ($R^0$)$_2$; N$H_2$; NH-$R^0$; N($R^0$)$_2$; NH-C(=O)-$R^0$; NH-C(=O)-O-$R^0$; NH-C(=O)-N$H_2$; NH-C(=O)-NH-$R^0$; NH-C(=O)-N ($R^0$)$_2$; N$R^0$-C(=O)-$R^0$; N$R^0$-C(=O)-O-$R^0$; N$R^0$-C(=O)-N$H_2$; N$R^0$-C(=O)-NH-$R^0$; N$R^0$-C(=O)-N($R^0$)$_2$; NH-S (=O)$_2$OH; NH-S(=O)$_2$$R^0$; NH-S(=O)$_2$O$R^0$; NH-S(=O)$_2$N$H_2$; NH-S(=O)$_2$NH$R^0$; NH-S(=O)$_2$N($R^0$)$_2$; N$R^0$-S (=O)$_2$OH; N$R^0$-S(=O)$_2$$R^0$; N$R^0$-S(=O)$_2$O$R^0$; N$R^0$-S(=O)$_2$N$H_2$; N$R^0$-S(=O)$_2$NH$R^0$; N$R^0$-S(=O)$_2$N($R^0$)$_2$; SH; S$R^0$; S(=O)$R^0$; S(=O)$_2$$R^0$; S(=O)$_2$OH; S(=O)$_2$O$R^0$; S(=O)$_2$N$H_2$; S(=O)$_2$NH$R^0$; S(=O)$_2$N($R^0$)$_2$; steht; und
$R^0$ für $C_{1-10}$-Alkyl oder $C_{2-10}$-Heteroalkyl, jeweils gesättigt oder ungesättigt, verzweigt oder unverzweigt, unsubstituiert oder einfach oder mehrfach substituiert; $C_{3-10}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$-Alkyl oder $C_{2-8}$-Heteroalkyl verbrücktes $C_{3-10}$-Cycloalkyl oder Heterocyclyl, jeweils gesättigt oder ungesättigt, unsubstituiert oder einfach oder mehrfach substituiert, wobei die Alkyl- oder Heteroalkylkette jeweils verzweigt oder unverzweigt, gesättigt oder ungesättigt, unsubstituiert, einfach oder mehrfach substituiert sein kann; oder über $C_{1-8}$-Alkyl oder $C_{2-8}$-Heteroalkyl verbrücktes Aryl oder Heteroaryl, jeweils unsubstituiert oder einfach oder mehrfach substituiert, wobei die Alkyl- oder Heteroalkylkette jeweils verzweigt oder unverzweigt, gesättigt oder ungesättigt, unsubstituiert, einfach oder mehrfach substituiert sein kann; steht;
in Form der freien Verbindungen oder Salze physiologisch verträglicher Säuren oder Basen.

2. Mercaptochinoline nach Anspruch 1, **dadurch gekennzeichnet, dass** wenn $R^7$ Heterocyclyl bedeutet die Bindung des Heterocyclyls an die übergeordnete allgemeine Struktur über ein Kohlenstoffatom des Heterocyclyls erfolgt.

3. Mercaptochinoline nach Anspruch 1 oder 2, **dadurch gekennzeichnet, dass** im Fall von
"Alkyl", "Heteroalkyl-", "Heterocyclyl-" oder "Cycloalkyl-" Substituenten diese ausgewählt sind aus der Gruppe bestehend aus F; Cl; Br; I; $NO_2$; $CF_3$; CN; =O; $C_{1-8}$-Alkyl; Aryl; Heteroaryl; $C_{3-10}$-Cycloalkyl; Heterocyclyl; über $C_{1-8}$-Alkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2$H; C(=O)O-$C_{1-8}$-Alkyl; C(=O)O-Aryl; C(=O)O-Heteroaryl; CON$H_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N ($C_{1-8}$-Alkyl)(Heteroaryl); C(=O)N(Heteroaryl)(Aryl); OH; O-$C_{1-8}$-Alkyl; OC$F_3$; -O-($C_{1-8}$-Alkyl)-0-; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$ ; NH-$C_{1-8}$-Alkyl; N($C_{1-8}$-Alkyl)$_2$; NH-C(=O)$C_{1-8}$-Alkyl; NH-C(=O)-Aryl; NH-C(=O)-Heteroaryl; SH; S-$C_{1-8}$-Alkyl; SC$F_3$; S-Benzyl; S-Aryl; S-Heteroaryl; S(=O)$_2$$C_{1-8}$-Alkyl; S(=O)$_2$Aryl; S(=O)$_2$Heteroaryl; S(=O)$_2$OH; S(=O)$_2$O-$C_{1-8}$-Alkyl; S(=O)$_2$O-Aryl; S(=O)$_2$OHeteroaryl; S(=O)$_2$-NH-$C_{1-8}$-Alkyl; S(=O)$_2$-NH-Aryl; und S(=O)$_2$-NH-$C_{1-8}$-Heteroaryl und
"Aryl" oder "Heteroaryl-" Substituenten diese ausgewählt sind aus der Gruppe bestehend aus F; Cl; Br; I; $NO_2$; $CF_3$; CN; $C_{1-8}$-Alkyl; Aryl; Heteroaryl; $C_{3-10}$-Cycloalkyl; Heterocyclyl; $C_{1-8}$-Alkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2$H; C(=O)O-$C_{1-8}$-Alkyl; C(=O) O-Aryl; C(=O)O-Heteroaryl; CON$H_2$; C(=O)NH-$C_{1-8}$-Alkyl-, C(=O)N($C_{1-8}$-Alkyl)$_2$; C(=O)NH-Aryl; C(=O)N(Aryl)$_2$; C (=O)NH-Heteroaryl; C(=O)N(Heteroaryl)2; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; OC$F_3$; -O-($C_{1-8}$-Alkyl)-O-; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$ ; NH-$C_{1-8}$-Alkyl; N($C_{1-8}$-Alkyl)$_2$; NH-C(=O)$C_{1-8}$-Alkyl; NH-C(=O)-Aryl; NH-C(=O)-Heteroaryl; SH; S-$C_{1-8}$-Alkyl-, SC$F_3$; S-Benzyl; S-Aryl; S-Heteroaryl; S(=O)$_2$$C_{1-8}$-Alkyl; S(=O)$_2$Aryl; S(=O)$_2$Heteroaryl; S(=O)$_2$OH; S(=O)$_2$O-$C_{1-8}$-Alkyl; S(=O)$_2$O-Aryl; S(=O)$_2$O-Heteroaryl; S(=O)$_2$-NH-$C_{1-8}$-Alkyl-, S(=O)$_2$-NH-Aryl; S(=O)$_2$-NH-$C_{1-8}$-Heteroaryl.

4. Mercaptochinoline nach einem der vorstehenden Ansprüche **dadurch gekennzeichnet, dass** im Fall von
$R^1$, $R^2$, $R^3$ und $R^4$ jeweils unabhängig voneinander ausgewählt sind aus der Gruppe bestehend aus H; F; Cl; Br; I; $NO_2$; $CF_3$; CN; OH; OC$F_3$; SH; SC$F_3$; N$H_2$; $C_{1-6}$-Alkyl, O-$C_{1-6}$-Alkyl, O-C(=O)-$C_{1-6}$-Alkyl, S-$C_{1-6}$-Alkyl, NH($C_{1-6}$-Alkyl), N($C_{1-6}$-Alkyl)$_2$, NH-C(=O)-$C_{1-6}$-Alkyl, N(C(=O)-$C_{1-6}$-Alkyl)$_2$ und C(=O)-$C_{1-6}$-Alkyl, jeweils gesättigt oder ungesättigt, verzweigt oder unverzweigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; OC$F_3$; SH; SC$F_3$; N$H_2$; $C_{1-6}$-Alkyl, O-$C_{1-6}$-Alkyl, O-C(=O)-$C_{1-6}$-Alkyl, S-$C_{1-6}$-Alkyl, NH($C_{1-6}$-Alkyl), N($C_{1-6}$-Alkyl)$_2$, NH-C(=O)-$C_{1-6}$-Alkyl, N(C(=O)-$C_{1-6}$-Alkyl)$_2$ und C(=O)-$C_{1-6}$-Alkyl; $C_{3-6}$-Cycloalkyl oder -Heterocyclyl jeweils gesättigt oder ungesättigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; OC$F_3$; SH; SC$F_3$; N$H_2$; $C_{1-6}$-Alkyl, O-$C_{1-6}$-Alkyl, O-C(=O)-$C_{1-6}$-Alkyl, S-$C_{1-6}$-Alkyl, NH($C_{1-6}$-Alkyl), N($C_{1-6}$-Alkyl)$_2$, NH-C (=O)-$C_{1-6}$-Alkyl, N(C(=O)-$C_{1-6}$-Alkyl)$_2$ und C(=O)-$C_{1-6}$-Alkyl,
und

$R^5$ ausgewählt ist aus der Gruppe bestehend aus H; F; Cl; Br; I; $NO_2$; $CF_3$; CN; $OCF_3$; SH; $SCF_3$; $NH_2$; $C_{1-6}$-Alkyl, O-$C_{1-6}$-Alkyl, O-C(=O)-$C_{1-6}$-Alkyl, S-$C_{1-6}$-Alkyl, NH($C_{1-6}$-Alkyl), N($C_{1-6}$-Alkyl)$_2$, NH-C(=O)-$C_{1-6}$-Alkyl, N(C (=O)-$C_{1-6}$-Alkyl)$_2$ und C(=O)-$C_{1-6}$-Alkyl, jeweils gesättigt oder ungesättigt, verzweigt oder unverzweigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{1-6}$-Alkyl, O-$C_{1-6}$-Alkyl, O-C(=O)-$C_{1-6}$-Alkyl, S-$C_{1-6}$-Alkyl, NH($C_{1-6}$-Alkyl), N($C_{1-6}$-Alkyl)$_2$, NH-C(=O)-$C_{1-6}$-Alkyl, N(C(=O)-$C_{1-6}$-Alkyl)$_2$ und C (=O)-$C_{1-6}$-Alkyl; $C_{3-6}$-Cycloalkyl oder -Heterocyclyl jeweils gesättigt oder ungesättigt, unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_3$; CN; OH; $OCF_3$; SH; $SCF_3$; $NH_2$; $C_{16}$-Alkyl, O-$C_{1-6}$-Alkyl, O-C (O)-$C_{1-6}$-Alkyl, S-$C_{1-6}$-Alkyl, NH($C_{1-6}$-Alkyl), N($C_{1-6}$-Alkyl)$_2$, NH-C(=O)-$C_{1-6}$-Alkyl, N(C(=O)-$C_{1-6}$-Alkyl)$_2$ und C (=O)-$C_{1-6}$-Alkyl.

5. Mercaptochinoline nach einem der vorstehenden Ansprüche, **dadurch gekennzeichnet, dass**
$R^1$, $R^2$, $R^3$ und $R^4$ jeweils unabhängig voneinander für H; F; Cl; CN; $OCF_3$; $SCF_3$; $CF_3$; $CH_3$ oder $OCH_3$ stehen und $R^5$ H, F, Cl, $OCF_3$, $SCF_3$, $C_{1-6}$-Alkyl, O-$C_{1-6}$-Alkyl oder S-$C_{1-6}$-Alkyl bedeutet.

6. Mercaptochinoline nach einem der vorstehenden Ansprüche, **dadurch gekennzeichnet, dass**
$R^{6a}$ und $R^{6b}$ jeweils unabhängig voneinander für H; F; Cl; Br; I; Methyl; Ethyl; n-Propyl; iso-Propyl; n-Butyl; sec.-Butyl; tert.-Butyl; OH; O-Methyl oder O-Ethyl stehen.

7. Mercaptochinoline nach einem der vorstehenden Ansprüche, **dadurch gekennzeichnet, dass**
$R^7$ für $C_{2-6}$-Alkyl oder $C_{2-6}$-Heteroalkyl, gesättigt oder ungesättigt; verzweigt oder unverzweigt, unsubstituiert; $C_{3-8}$-Cycloalkyl oder -Heterocyclyl gesättigt oder ungesättigt, unsubstituiert; Phenyl, Furyl, Thienyl oder Pyridyl, jeweils unsubstituiert oder einfach oder mehrfach substituiert mit einem oder mehreren Substituenten jeweils unabhängig voneinander ausgewählt aus der Gruppe bestehend aus F, Cl, Br, I, CN; $CF_3$, $OCF_3$, $SCF_3$, $CH_3$ und $OCH_3$, steht.

8. Mercaptochinoline nach einem der vorstehenden Ansprüche, **dadurch gekennzeichnet, dass**
m für 0, n für 1 und $R^7$ für Aryl oder Heteroaryl, jeweils unsubstituiert oder einfach oder mehrfach substituiert steht.

9. Mercaptochinoline nach einem der vorstehenden Ansprüche, **dadurch gekennzeichnet, dass**
m für 0, n für 1 oder 2 und $R^7$ für $C_{1-10}$-Alkyl oder $C_{2-10}$-Heteroalkyl, gesättigt oder ungesättigt; verzweigt oder unverzweigt, unsubstituiert oder einfach oder mehrfach substituiert; $C_{3-10}$-Cycloalkyl oder -Heterocyclyl gesättigt oder ungesättigt, unsubstituiert oder einfach oder mehrfach substituiert steht.

10. Mercaptochinoline nach einem der vorstehenden Ansprüche, **dadurch gekennzeichnet, dass**
$R^8$ ausgewählt ist aus der Gruppe bestehend aus $C_{1-8}$-Alkyl oder $C_{2-8}$-Heteroalkyl, jeweils gesättigt oder ungesättigt, verzweigt oder unverzweigt, unsubstituiert oder einfach oder mehrfach substituiert mit einem oder mehreren Substituenten unabhängig voneinander ausgewählt aus der Gruppe bestehend aus F, Cl, Br, I, OH, =O, $OCF_3$, $SCF_3$, $CF_3$, $C_{1-4}$-Alkyl und O$C_{1-4}$-Alkyl; $C_{3-10}$-Cycloalkyl oder -Heterocyclyl, jeweils gesättigt oder ungesättigt, unsubstituiert oder einfach oder mehrfach substituiert mit einem oder mehreren Substituenten unabhängig voneinander ausgewählt aus der Gruppe bestehend aus F, Cl, Br, I, OH, =O, $OCF_3$, $SCF_3$, $CF_3$, $C_{1-4}$-Alkyl und O$C_{1-4}$-Alkyl; Aryl oder Heteroaryl, jeweils unsubstituiert oder einfach oder mehrfach substituiert mit einem oder mehreren Substituenten unabhängig voneinander ausgewählt aus der Gruppe bestehend aus H; F; Cl; Br; I; $NO_2$; $CF_{3-}$ CN; OH; $OCF_3$; SH; $SCF_3$; $NH_2$; $C_{1-6}$-Alkyl, O-$C_{1-6}$-Alkyl, O-C(=O)-$C_{1-6}$-Alkyl, S-$C_{1-6}$-Alkyl, NH($C_{1-6}$-Alkyl), N($C_{1-6}$-Alkyl)$_2$, NH-C(=O)-$C_{1-6}$-Alkyl, N(C(=O)-$C_{1-6}$-Alkyl)$_2$ oder C(=O)-$C_{1-6}$-Alkyl; Aryl oder Heteroaryl, jeweils unsubstituiert oder einfach oder mehrfach substituiert mit einem oder mehreren Substituenten unabhängig voneinander ausgewählt aus der Gruppe bestehend aus H; F; Cl; Br; I; $CF_{3-}$ CN; OH; $OCF_3$; SH; $SCF_3$; $NH_2$; $C_{1-6}$-Alkyl, O-$C_{1-6}$-Alkyl, O-C(=O)-$C_{1-6}$-Alkyl, S-$C_{1}$-$_6$-Alkyl, NH($C_{1-6}$-Alkyl), N($C_{1-6}$-Alkyl)$_2$, NH-C(=O)-$C_{1-6}$-Alkyl, N(C(=O)-$C_{1-6}$-Alkyl)$_2$ oder C(=O)-$C_{1-6}$-Alkyl; über $C_{1-8}$-Alkyl oder $C_{2-8}$-Heteroalkyl verbrücktes $C_{3-10}$-Cycloalkyl, gesättigt oder ungesättigt, unsubstituiert oder einfach oder mehrfach substituiert mit einem oder mehreren Substituenten unabhängig voneinander ausgewählt aus der Gruppe bestehend aus F, Cl, Br, I, OH, =O, $OCF_3$, $SCF_3$, $CF_3$, $C_{1-8}$-Alkyl und O$C_{1-8}$-Alkyl, wobei die Alkyl- oder Heteroalkylkette jeweils verzweigt oder unverzweigt, gesättigt oder ungesättigt, unsubstituiert sein kann; oder über $C_{1-8}$-Alkyl oder $C_{2-8}$-Heteroalkyl verbrücktes Aryl oder Heteroaryl, jeweils unsubstituiert oder einfach oder mehrfach substituiert mit einem oder mehreren Substituenten unabhängig voneinander ausgewählt aus der Gruppe bestehend aus F, Cl, Br, I, OH, $NH_2$, $OCF_3$, $SCF_3$, $CF_3$, $C_{1-8}$-Alkyl und O$C_{1-8}$-Alkyl, wobei die Alkyl- oder Heteroalkylkette jeweils verzweigt oder unverzweigt, gesättigt oder ungesättigt, unsubstituiert sein kann.

**11.** Mercaptochinoline nach Anspruch 1 ausgewählt aus der Gruppe

**1** 2-Cyclohexyl-N-(2-(2-(phenylsulfonyl)ethylthio)chinolin-3-yl)acetamid;
**2** N-(2-(2-(Phenylsulfonyl)ethylthio)chinolin-3-yl)-2-(thiophen-2-yl)acetamid;
**4** N-(2-(Pentylthio)chinolin-3-yl)-2-(thiophen-2-yl)acetamid;
**6** N-(2-(2-(Phenylthio)ethylthio)chinolin-3-yl)-2-(thiophen-2-yl)acetamid;
**7** N-(2-(Ethylthio)chinolin-3-yl)-2-(thiophen-2-yl)acetamid;
**9** N-(2-(Ethylthio)chinolin-3-yl)-3,3-dimethylbutanamid;
**11** N-[2-Ethylsulfanyl-7-(trifluormethyl)-chinolin-3-yl]-3,3-dimethyl-butyramid
**12** 3-Cyclopentyl-N-[2-ethylsulfanyl-7-(trifluormethyl)-chinolin-3-yl]-propionamid
**14** 2-(5-Bicyclo[2.2.1]heptanyl)-N-(2-ethylsulfanyl-chinolin-3-yl)-acetamid
**15** 3-Cyclopentyl-N-(2-ethylsulfanyl-chinolin-3-yl)-propionamid
**16** 2-(5-Bicyclo[2.2.1]heptanyl)-N-[2-ethylsulfanyl-7-(trifluormethyl)-chinolin-3-yl]-acetamid
**17** 3-Cyclopentyl-N-[2-ethylsulfanyl-4-methyl-7-(trifluormethyl)-chinolin-3-yl]-propionamid
**18** N-[2-Ethylsulfanyl-4-methyl-7-(trifluormethyl)-chinolin-3-yl]-2-thiophen-2-yl-acetamid

oder deren physiologisch verträgliche Salze.

**12.** Arzneimittel enthaltend wenigstens ein 3-Amino-2-mercaptochinolin nach einem der Ansprüche 1 bis 11 in Form eines einzelnen Stereoisomers oder deren Mischung, der freien Verbindungen und/oder ihrer physiologisch verträglichen Salze, sowie ggf. geeignete Zusatz- und/ oder Hilfsstoffe und/oder gegebenenfalls weiterer Wirkstoffe.

**13.** Verwendung wenigstens eines 3-Amino-2-mercaptochinolins nach einem der Ansprüche 1 bis 11. in Form eines einzelnen Stereoisomers oder deren Mischung, der freien Verbindung und/oder ihrer physiologisch verträglichen Salze, zur Herstellung eines Arzneimittels zur Behandlung von Schmerz, Epilepsie, Angstzuständen, Abhängigkeit, Manie, bipolaren Störungen, Migräne, kognitiven Erkrankungen, Dystonie-assoziierten Dyskinesien und/oder Harninkontinenz.

**14.** Ein substituiertes 3-Amino-2-mercaptochinolin nach einem der Ansprüche 1 bis 11 in Form eines einzelnen Stereoisomers oder deren Mischung, der freien Verbindung und/oder ihrer physiologisch verträglichen Salze zur Verwendung in der Behandlung von Schmerz, Epilepsie, Angstzuständen, Abhängigkeit, Manie, bipolaren Störungen, Migräne, kognitiven Erkrankungen, Dystonie-assoziierten Dyskinesien und/oder Harninkontinenz.

## Claims

**1.** Substituted 3-amino-2-mercaptoquinolines of the general formula (1)

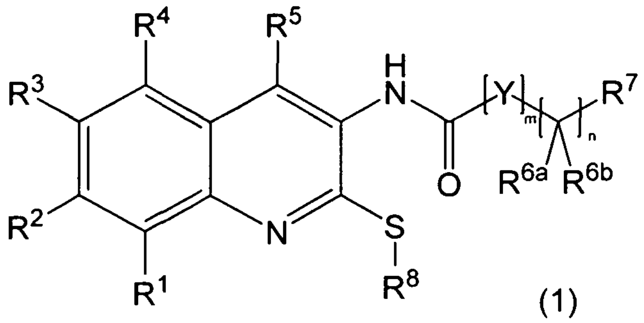

(1) ,

wherein

m represents 0 or 1 and
n represents an integer from 0 to 4,
$R^1$, $R^2$, $R^3$, $R^4$, $R^{6a}$ , $R^{6b}$ each independently of the others represents H; F; Cl; Br; I; $NO_2$; $CF_3$; CN; OH; $OCF_3$; SH; $SCF_3$; $NH_2$; $C_{1-10}$-alkyl, O-$C_{1-10}$-alkyl, O-C(=O)-$C_{1-10}$-alkyl, S-$C_{1-10}$-alkyl, NH($C_{1-10}$-alkyl), N($C_{1-10}$-alkyl)$_2$, NH-C(=O)-$C_{1-10}$-alkyl, N(C(=O)-$C_{1-10}$-alkyl)$_2$ or C(=O)-$C_{1-10}$-alkyl, in each case saturated or unsaturated, branched or unbranched, unsubstituted or mono- or poly-substituted; $C_{3-8}$-cycloalkyl or heterocyclyl, in each

case saturated or unsaturated, unsubstituted or mono- or poly-substituted;
$R^5$ represents H; F; Cl; Br; 1; $NO_2$; $CF_3$; CN; $OCF_3$; SH; $SCF_3$; $NH_2$; $C_{1-10}$-alkyl, O-$C_{1-10}$-alkyl, O-C(=O)-$C_{1-10}$-alkyl, S-$C_{1-10}$-alkyl, NH($C_{1-10}$-alkyl), N($C_{1-10}$-alkyl)$_2$, NH-C(=O)-$C_{1-10}$-alkyl, N(C(=O)-$C_{1-10}$-alkyl)$_2$ or C(=O)-$C_{1-10}$-alkyl, in each case saturated or unsaturated, branched or unbranched, unsubstituted or mono- or poly-substituted; $C_{3-8}$-cycloalkyl or heterocyclyl, in each case saturated or unsaturated, unsubstituted or mono- or poly-substituted;
Y represents O or $NR^9$,
wherein $R^9$ represents H or $C_{1-4}$-alkyl, saturated or unsaturated, branched or unbranched, unsubstituted or mono- or poly-substituted;
$R^7$ represents $C_{1-10}$-alkyl or $C_{2-10}$-heteroalkyl, saturated or unsaturated, branched or unbranched, unsubstituted or mono- or poly-substituted; $C_{3-10}$-cycloalkyl or heterocyclyl, in each case saturated or unsaturated, unsubstituted or mono- or poly-substituted; aryl or heteroaryl, in each case unsubstituted or mono- or poly-substituted; with the proviso that, when $R^7$ denotes heterocyclyl, the bonding of the heterocyclyl to the general structure of higher order can take place *via* a carbon atom of the heterocyclyl, and
with the proviso that, when $R^7$ denotes aryl or heteroaryl, the sum of n and m is greater than or equal to 1;
$R^8$ is selected from the group consisting of $C_{1-10}$-alkyl or $C_{2-10}$-heteroalkyl, in each case saturated or unsaturated, branched or unbranched, unsubstituted or mono- or poly-substituted; $C_{3-10}$-cycloalkyl or heterocyclyl, in each case saturated or unsaturated, unsubstituted or mono- or poly-substituted; aryl or heteroaryl, in each case unsubstituted or mono- or poly-substituted; $C_{1-8}$-alkyl- or $C_{2-8}$-heteroalkyl-bridged $C_{3-10}$-cycloalkyl or heterocyclyl, in each case saturated or unsaturated, unsubstituted or mono- or poly-substituted; or $C_{1-8}$-alkyl- or $C_{2-8}$-heteroalkyl-bridged aryl or heteroaryl, in each case unsubstituted or mono- or poly-substituted, wherein the alkyl or heteroalkyl chain in each case can be branched or unbranched, saturated or unsaturated, unsubstituted;
wherein "alkyl substituted", "heteroalkyl substituted", "heterocyclyl substituted" and "cycloalkyl substituted" denote the substitution of one or more hydrogen atoms, in each case independently of one another, by F; Cl; Br; I; CN; $CF_3$; =O; =NH; =C($NH_2$)$_2$; $NO_2$; $R^0$; C(=O)H; C(=O)$R^0$; $CO_2$H; C(=O)O$R^0$; CON$H_2$; C(=O)NH$R^0$; C(=O)N($R^0$)$_2$; OH; O$R^0$; -O-($C_{1-8}$-alkyl)-O-; O-C(=O)-$R^0$; O-C(=O)-O-$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2$N$H_2$; O-S(=O)$_2$NH$R^0$; O-S(=O)$_2$N($R^0$)$_2$; N$H_2$; NH-$R^0$; N($R^0$)$_2$; NH-C(=O)-$R^0$; NH-C(=O)-O-$R^0$; NH-C(=O)-N$H_2$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-N$H_2$; N$R^0$-C(=O)-NH-$R^0$; N$R^0$-C(=O)-N($R^0$)$_2$; NH-S(=O)$_2$OH; NH-S(=O)$_2$$R^0$; NH-S(=O)$_2$O$R^0$; NH-S(=O)$_2$N$H_2$; NH-S(=O)$_2$NH$R^0$; NH-S(=O)$_2$N($R^0$)$_2$; N$R^0$-S(=O)$_2$OH; N$R^0$-S(=O)$_2$$R^0$; N$R^0$-S(=O)$_2$O$R^0$; N$R^0$-S(=O)$_2$N$H_2$; N$R^0$-S(=O)$_2$NH$R^0$; N$R^0$-S(=O)$_2$N($R^0$)$_2$; SH; S$R^0$; S(=O)$R^0$; S(=O)$_2$$R^0$; S(=O)$_2$H; S(=O)$_2$OH; S(=O)$_2$O$R^0$; S(=O)$_2$N$H_2$; S(=O)$_2$NH$R^0$; S(=O)$_2$N($R^0$)$_2$;
wherein "aryl substituted" and "heteroaryl substituted" denote the substitution of one or more hydrogen atoms, in each case independently of one another, by F; Cl; Br; I; $NO_2$; $CF_3$; CN; $R^0$; C(=O)H; C(=O)$R^0$; $CO_2$H; C(=O)O$R^0$; CON$H_2$; C(=O)NH$R^0$; C(=O)N($R^0$)$_2$; OH; O$R^0$; -O-($C_{1-8}$-alkyl)-O-; O-C(=O)-$R^0$; O-C(=O)-O-$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2$N$H_2$; O-S(=O)$_2$NH$R^0$; O-S(=O)$_2$N($R^0$)$_2$; N$H_2$; NH-$R^0$; N($R^0$)$_2$; NH-C(=O)-$R^0$; NH-C(=O)-O-$R^0$-, NH-C(=O)-N$H_2$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-N$H_2$; N$R^0$-C(=O)-NH-$R^0$; N$R^0$-C(=O)-N($R^0$)$_2$; NH-S(=O)$_2$OH; NH-S(=O)$_2$$R^0$; NH-S(=O)$_2$O$R^0$; NH-S(=O)$_2$N$H_2$; NH-S(=O)$_2$NH$R^0$; NH-S(=O)$_2$N($R^0$)$_2$; N$R^0$-S(=O)$_2$OH; N$R^0$-S(=O)$_2$$R^0$; N$R^0$-S(=O)$_2$O$R^0$; N$R^0$-S(=O)$_2$N$H_2$; N$R^0$-S(=O)$_2$NH$R^0$; N$R^0$-S(=O)$_2$N($R^0$)$_2$; SH; S$R^0$; S(=O)$R^0$; S(=O)$_2$$R^0$; S(=O)$_2$OH; S(=O)$_2$O$R^0$; S(=O)$_2$N$H_2$; S(=O)$_2$NH$R^0$; S(=O)$_2$N($R^0$)$_2$; and
$R^0$ represents $C_{1-10}$-alkyl or $C_{2-10}$-heteroalkyl, in each case saturated or unsaturated, branched or unbranched, unsubstituted or mono- or poly-substituted; $C_{3-10}$-cycloalkyl or heterocyclyl, in each case saturated or unsaturated, unsubstituted or mono- or poly-substituted; aryl or heteroaryl, in each case unsubstituted or mono- or poly-substituted; $C_{1-8}$-alkyl- or $C_{2-8}$-heteroalkyl-bridged $C_{3-10}$-cycloalkyl or heterocyclyl, in each case saturated or unsaturated, unsubstituted or mono- or poly-substituted, wherein the alkyl or heteroalkyl chain in each case can be branched or unbranched, saturated or unsaturated, unsubstituted, mono- or poly-substituted; or $C_{1-8}$-alkyl- or $C_{2-8}$-heteroalkyl-bridged aryl or heteroaryl, in each case unsubstituted or mono- or poly-substituted, wherein the alkyl or heteroalkyl chain in each case can be branched or unbranched, saturated or unsaturated, unsubstituted, mono- or poly-substituted;
in the form of the free compounds or salts of physiologically acceptable acids or bases.

2. Mercaptoquinolines according to claim 1, **characterised in that** when $R^7$ denotes heterocyclyl, the bonding of the heterocyclyl to the general structure of higher order is carried out *via* a carbon atom of the heterocyclyl.

3. Mercaptoquinolines according to claim 1 or 2, **characterised in that** in the case of

"alkyl", "heteroalkyl", "heterocyclyl" or "cycloalkyl" substituents, these are selected from the group consisting of F; Cl; Br; I; $NO_2$; $CF_3$; CN; =O; $C_{1-8}$-alkyl; aryl; heteroaryl; $C_{3-10}$-cycloalkyl; heterocyclyl; $C_{1-8}$-alkyl-bridged aryl, heteroaryl, $C_{3-10}$-cycloalkyl or heterocyclyl; CHO; C(=O)$C_{1-8}$-alkyl; C(=O)aryl; C(=O)heteroaryl; $CO_2H$; C(=O)O-$C_{1-8}$-alkyl; C(=O)O-aryl; C(=O)O-heteroaryl; $CONH_2$; C(=O)NH-$C_{1-8}$-alkyl; C(=O)N($C_{1-8}$-alkyl)$_2$; C(=O)NH-aryl; C(=O)N(aryl)$_2$; C(=O)NH-heteroaryl; C(=O)N(heteroaryl)$_2$; C(=O)N($C_{1-8}$-alkyl)(aryl); C(=O)N($C_{1-8}$-alkyl)(heteroaryl); C(=O)N(heteroaryl)(aryl); OH; O-$C_{1-8}$-alkyl; $OCF_3$; -O-($C_{1-8}$-alkyl)-O-; O-($C_{1-8}$-alkyl)-OH; O-($C_{1-8}$-alkyl)-O-$C_{1-8}$-alkyl; O-benzyl; O-aryl; O-heteroaryl; O-C(=O)$C_{1-8}$-alkyl; O-C(=O)aryl; O-C(=O)heteroaryl; $NH_2$; NH-$C_{1-8}$-alkyl; N($C_{1-8}$-alkyl)$_2$; NH-C(=O)$C_{1-8}$-alkyl; NH-C(=O)-aryl; NH-C(=O)-heteroaryl; SH; S-$C_{1-8}$-alkyl; $SCF_3$; S-benzyl; S-aryl; S-heteroaryl; S(=O)$_2$$C_{1-8}$-alkyl; S(=O)$_2$aryl; S(=O)$_2$heteroaryl; S(=O)$_2$OH; S(=O)$_2$O-$C_{1-8}$-alkyl; S(=O)$_2$O-aryl; S(=O)$_2$O-heteroaryl; S(=O)$_2$-NH-$C_{1-8}$-alkyl; S(=O)$_2$-NH-aryl; and S(=O)$_2$-NH-$C_{1-8}$-heteroaryl and
"aryl" or "heteroaryl" substituents, these are selected from the group consisting of F; Cl; Br; I; $NO_2$; $CF_3$; CN; $C_{1-8}$-alkyl; aryl; heteroaryl; $C_{3-10}$-cycloalkyl; heterocyclyl; $C_{1-8}$-alkyl-bridged aryl, heteroaryl, $C_{3-10}$-cycloalkyl or heterocyclyl; CHO; C(=O)$C_{1-8}$-alkyl; C(=O)aryl; C(=O)heteroaryl; $CO_2H$; C(=O)O-$C_{1-8}$-alkyl; C(=O)O-aryl; C(=O)O-heteroaryl; $CONH_2$; C(=O)NH-$C_{1-8}$-alkyl; C(=O)N($C_{1-8}$-alkyl)$_2$; C(=O)NH-aryl; C(=O)N(aryl)$_2$; C(=O)NH-heteroaryl; C(=O)N(heteroaryl)$_2$; C(=O)N($C_{1-8}$-alkyl)(aryl); C(=O)N($C_{1-8}$-alkyl)(heteroaryl); C(=O)N(heteroaryl)(aryl); OH; O-$C_{1-8}$-alkyl; $OCF_3$; -O-($C_{1-8}$-alkyl)-O-; O-($C_{1-8}$-alkyl)-OH; O-($C_{1-8}$-alkyl)-O-$C_{1-8}$-alkyl; O-benzyl; O-aryl; O-heteroaryl; O-C(=O)$C_{1-8}$-alkyl; O-C(=O)aryl; O-C(=O)heteroaryl; $NH_2$; NH-$C_{1-8}$-alkyl; N($C_{1-8}$-alkyl)$_2$; NH-C(=O)$C_{1-8}$-alkyl; NH-C(=O)-aryl; NH-C(=O)-heteroaryl; SH; S-$C_{1-8}$-alkyl; $SCF_3$; S-benzyl; S-aryl; S-heteroaryl; S(=O)$_2$$C_{1-8}$-alkyl; S(=O)$_2$aryl; S(=O)$_2$heteroaryl; S(=O)$_2$OH; S(=O)$_2$O-$C_{1-8}$-alkyl; S(=O)$_2$O-aryl; S(=O)$_2$O-heteroaryl; S(=O)$_2$-NH-$C_{1-8}$-alkyl; S(=O)$_2$-NH-aryl; S(=O)$_2$-NH-$C_{1-8}$-heteroaryl.

4. Mercaptoquinolines according to any one of the preceding claims, **characterised in that**
$R^1$, $R^2$, $R^3$ and $R^4$ each independently of the others are selected from the group consisting of H; F; Cl; Br; I; $NO_2$; $CF_3$; CN; OH; $OCF_3$; SH; $SCF_3$; $NH_2$; $C_{1-6}$-alkyl, O-$C_{1-6}$-alkyl, O-C(=O)-$C_{1-6}$-alkyl, S-$C_{1-6}$-alkyl, NH($C_{1-6}$-alkyl), N($C_{1-6}$-alkyl)$_2$, NH-C(=O)-$C_{1-6}$-alkyl, N(C(=O)-$C_{1-6}$-alkyl)$_2$ and C(=O)-$C_{1-6}$-alkyl, in each case saturated or unsaturated, branched or unbranched, unsubstituted or mono- or poly-substituted by one or more substituents selected independently of one another from the group consisting of H; F; Cl; Br; I; $NO_2$; $CF_3$; CN; OH; $OCF_3$; SH; $SCF_3$; $NH_2$; $C_{1-6}$-alkyl, O-$C_{1-6}$-alkyl, O-C(=O)-$C_{1-6}$-alkyl, S-$C_{1-6}$-alkyl, NH($C_{1-6}$-alkyl), N($C_{1-6}$-alkyl)$_2$, NH-C(=O)-$C_{1-6}$-alkyl, N(C(=O)-$C_{1-6}$-alkyl)$_2$ and C(=O)-$C_{1-6}$-alkyl; $C_{3-6}$-cycloalkyl or heterocyclyl, in each case saturated or unsaturated, unsubstituted or mono- or poly-substituted by one or more substituents selected independently of one another from the group consisting of H; F; Cl; Br; I; $NO_2$; $CF_3$; CN; OH; $OCF_3$; SH; $SCF_3$; $NH_2$; $C_{1-6}$-alkyl, O-$C_{1-6}$-alkyl, O-C(=O)-$C_{1-6}$-alkyl, S-$C_{1-6}$-alkyl, NH($C_{1-6}$-alkyl), N($C_{1-6}$-alkyl)$_2$, NH-C(=O)-$C_{1-6}$-alkyl, N(C(=O)-$C_{1-6}$-alkyl)$_2$ and C(=O)-$C_{1-6}$-alkyl,
and
$R^5$ is selected from the group consisting of H; F; Cl; Br; I; $NO_2$; $CF_3$; CN; $OCF_3$; SH; $SCF_3$; $NH_2$; $C_{1-6}$-alkyl, O-$C_{1-6}$-alkyl, O-C(=O)-$C_{1-6}$-alkyl, S-$C_{1-6}$-alkyl, NH($C_{1-6}$-alkyl), N($C_{1-6}$-alkyl)$_2$, NH-C(=O)-$C_{1-6}$-alkyl, N(C(=O)-$C_{1-6}$-alkyl)$_2$ and C(=O)-$C_{1-6}$-alkyl, in each case saturated or unsaturated, branched or unbranched, unsubstituted or mono- or poly-substituted by one or more substituents selected independently of one another from the group consisting of H; F; Cl; Br; I; $NO_2$; $CF_3$; CN; OH; $OCF_3$; SH; $SCF_3$; $NH_2$; $C_{1-6}$-alkyl, O-$C_{1-6}$-alkyl, O-C(=O)-$C_{1-6}$-alkyl, S-$C_{1-6}$-alkyl, NH($C_{1-6}$-alkyl), N($C_{1-6}$-alkyl)$_2$, NH-C(=O)-$C_{1-6}$-alkyl, N(C(=O)-$C_{1-6}$-alkyl)$_2$ and C(=O)-$C_{1-6}$-alkyl; $C_{3-6}$-cycloalkyl or heterocyclyl, in each case saturated or unsaturated, unsubstituted or mono- or poly-substituted by one or more substituents selected independently of one another from the group consisting of H; F; Cl; Br; I; $NO_2$; $CF_3$; CN; OH; $OCF_3$; SH; $SCF_3$; $NH_2$; $C_{1-6}$-alkyl, O-$C_{1-6}$-alkyl, O-C(=O)-$C_{1-6}$-alkyl, S-$C_{1-6}$-alkyl, NH($C_{1-6}$-alkyl), N($C_{1-6}$-alkyl)$_2$, NH-C(=O)-$C_{1-6}$-alkyl, N(C(=O)-$C_{1-6}$-alkyl)$_2$ and C(=O)-$C_{1-6}$-alkyl.

5. Mercaptoquinolines according to any one of the preceding claims, **characterised in that**
$R^1$, $R^2$, $R^3$ and $R^4$ each independently of the others represents H; F; Cl; CN; $OCF_3$; $SCF_3$; $CF_3$; $CH_3$ or $OCH_3$ and $R^5$ denotes H, F, Cl, $OCF_3$, $SCF_3$, $C_{1-6}$-alkyl, O-$C_{1-6}$-alkyl or S-$C_{1-6}$-alkyl.

6. Mercaptoquinolines according to any one of the preceding claims, **characterised in that**
$R^{6a}$ and $R^{6b}$ each independently of the other represents H; F; Cl; Br; I; methyl; ethyl; n-propyl; isopropyl; n-butyl; sec-butyl; tert-butyl; OH; O-methyl or O-ethyl.

7. Mercaptoquinolines according to any one of the preceding claims, **characterised in that**
$R^7$ represents $C_{2-6}$-alkyl or $C_{2-6}$-heteroalkyl, saturated or unsaturated, branched or unbranched, unsubstituted; $C_{3-8}$-cycloalkyl or heterocyclyl, saturated or unsaturated, unsubstituted; phenyl, furyl, thienyl or pyridyl, in each case unsubstituted or mono- or poly-substituted by one or more substituents selected independently of one another from the group consisting of F, Cl, Br, I, CN; $CF_3$, $OCF_3$, $SCF_3$, $CH_3$ and $OCH_3$.

8. Mercaptoquinolines according to any one of the preceding claims, **characterised in that** m represents 0, n represents 1 and $R^7$ represents aryl or heteroaryl, in each case unsubstituted or mono- or poly-substituted.

9. Mercaptoquinolines according to any one of the preceding claims, **characterised in that** m represents 0, n represents 1 or 2 and $R^7$ represents $C_{1-10}$-alkyl or $C_{2-10}$-heteroalkyl, saturated or unsaturated; branched or unbranched, unsubstituted or mono- or poly-substituted; $C_{3-10}$-cycloalkyl or heterocyclyl, saturated or unsaturated, unsubstituted or mono- or poly-substituted.

10. Mercaptoquinolines according to any one of the preceding claims, **characterised in that** $R^8$ is selected from the group consisting of $C_{1-8}$-alkyl or $C_{2-8}$-heteroalkyl, in each case saturated or unsaturated, branched or unbranched, unsubstituted or mono- or poly-substituted by one or more substituents selected independently of one another from the group consisting of F, Cl, Br, I, OH, =O, $OCF_3$, $SCF_3$, $CF_3$, $C_{1-4}$-alkyl and $OC_{1-4}$-alkyl; $C_{3-10}$-cycloalkyl or heterocyclyl, in each case saturated or unsaturated, unsubstituted or mono- or poly-substituted by one or more substituents selected independently of one another from the group consisting of F, Cl, Br, I, OH, =O, $OCF_3$, $SCF_3$, $CF_3$, $C_{1-4}$-alkyl and $OC_{1-4}$-alkyl; aryl or heteroaryl, in each case unsubstituted or mono- or poly-substituted by one or more substituents selected independently of one another from the group consisting of H; F; Cl; Br; I; $NO_2$; $CF_3$; CN; OH; $OCF_3$; SH; $SCF_3$; $NH_2$; $C_{1-6}$-alkyl, O-$C_{1-6}$-alkyl, O-C(=O)-$C_{1-6}$-alkyl, S-$C_{1-6}$-alkyl, NH($C_{1-6}$-alkyl), N($C_{1-6}$-alkyl)$_2$, NH-C(=O)-$C_{1-6}$-alkyl, N(C(=O)-$C_{1-6}$-alkyl)$_2$ or C(=O)-$C_{1-6}$-alkyl; aryl or heteroaryl, in each case unsubstituted or mono- or poly-substituted by one or more substituents selected independently of one another from the group consisting of H; F; Cl; Br; I; $CF_3$; CN; OH; $OCF_3$; SH; $SCF_3$; $NH_2$; $C_{1-6}$-alkyl, O-$C_{1-6}$-alkyl, O-C(=O)-$C_{1-6}$-alkyl, S-$C_{1-6}$-alkyl, NH($C_{1-6}$-alkyl), N($C_{1-6}$-alkyl)$_2$, NH-C(=O)-$C_{1-6}$-alkyl, N(C(=O)-$C_{1-6}$-alkyl)$_2$ or C(=O)-$C_{1-6}$-alkyl; $C_{1-8}$-alkyl- or $C_{2-8}$-heteroalkyl-bridged $C_{3-10}$-cycloalkyl, saturated or unsaturated, unsubstituted or mono- or poly-substituted by one or more substituents selected independently of one another from the group consisting of F, Cl, Br, I, OH, =O, $OCF_3$, $SCF_3$, $CF_3$, $C_{1-8}$-alkyl and $OC_{1-8}$-alkyl, wherein the alkyl or heteroalkyl chain in each case can be branched or unbranched, saturated or unsaturated, unsubstituted; or $C_{1-8}$-alkyl- or $C_{2-8}$-heteroalkyl-bridged aryl or heteroaryl, in each case unsubstituted or mono- or poly-substituted by one or more substituents selected independently of one another from the group consisting of F, Cl, Br, I, OH, $NH_2$, $OCF_3$, $SCF_3$, $CF_3$, $C_{1-8}$-alkyl and $OC_{1-8}$-alkyl, wherein the alkyl or heteroalkyl chain in each case can be branched or unbranched, saturated or unsaturated, unsubstituted.

11. Mercaptoquinolines according to claim 1, selected from the group

**1** 2-cyclohexyl-N-(2-(2-(phenylsulfonyl)ethylthio)quinolin-3-yl)acetamide;
**2** N-(2-(2-(phenylsulfonyl)ethylthio)quinolin-3-yl)-2-(thiophen-2-yl)acetamide;
**4** N-(2-(pentylthio)quinolin-3-yl)-2-(thiophen-2-yl)acetamide;
**6** N-(2-(2-(phenylthio)ethylthio)quinolin-3-yl)-2-(thiophen-2-yl)acetamide;
**7** N-(2-(ethylthio)quinolin-3-yl)-2-(thiophen-2-yl)acetamide;
**9** N-(2-(ethylthio)quinolin-3-yl)-3,3-dimethylbutanamide;
**11** N-[2-ethylsulfanyl-7-(trifluoromethyl)-quinolin-3-yl]-3,3-dimethyl-butyramide
**12** 3-cyclopentyl-N-[2-ethylsulfanyl-7-(trifluoromethyl)-quinolin-3-yl]-propionamide
**14** 2-(5-bicyclo[2.2.1]heptanyl)-N-(2-ethylsulfanyl-quinolin-3-yl)-acetamide
**15** 3-cyclopentyl-N-(2-ethylsulfanyl-quinolin-3-yl)-propionamide
**16** 2-(5-bicyclo[2.2.1]heptanyl)-N-[2-ethylsulfanyl-7-(trifluoromethyl)-quinolin-3-yl]-acetamide
**17** 3-cyclopentyl-N-[2-ethylsulfanyl-4-methyl-7-(trifluoromethyl)-quinolin-3-yl]-propionamide
**18** N-[2-ethylsulfanyl-4-methyl-7-(trifluoromethyl)-quinolin-3-yl]-2-thiophen-2-yl-acetamide

or physiologically acceptable salts thereof.

12. Medicament comprising at least one 3-amino-2-mercaptoquinoline according to any one of claims 1 to 11, in the form of an individual stereoisomer or a mixture thereof, of the free compounds and/or physiologically acceptable salts thereof, as well as optionally suitable additives and/or auxiliary substances and/or optionally further active ingredients.

13. Use of at least one 3-amino-2-mercaptoquinoline according to any one of claims 1 to 11, in the form of an individual stereoisomer or a mixture thereof, of the free compound and/or physiologically acceptable salts thereof, in the preparation of a medicament for the treatment of pain, epilepsy, anxiety, dependency, mania, bipolar disorders, migraine, cognitive diseases, dystonia-associated dyskinesias and/or urinary incontinence.

**14.** A substituted 3-amino-2-mercaptoquinoline according to any one of claims 1 to 11, in the form of an individual stereoisomer or a mixture thereof, of the free compound and/or physiologically acceptable salts thereof, for use in the treatment of pain, epilepsy, anxiety, dependency, mania, bipolar disorders, migraine, cognitive diseases, dystonia-associated dyskinesias and/or urinary incontinence.

## Revendications

**1.** 3-amino-2-mercaptoquinoléines de formule générale (1)

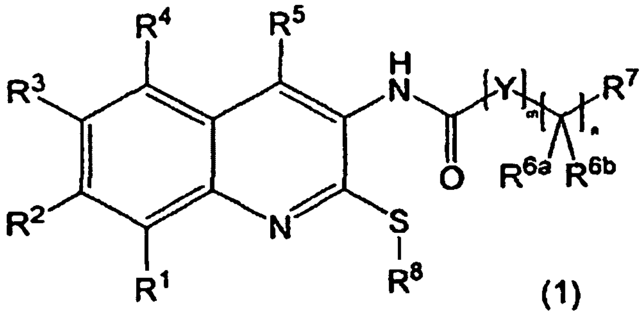

(1) ,

dans laquelle :

m est égal à 0 ou 1, et

n représente un nombre entier de 0 à 4, $R^1$, $R^2$, $R^3$, $R^4$, $R^{6a}$, $R^{6b}$ représentent, respectivement indépendamment les uns des autres, H ; F ; Cl ; Br ; I ; $NO_2$ ; $CF_3$ ; CN ; OH ; $OCF_3$ ; SH ; $SCF_3$ ; $NH_2$ ; un groupe alkyle en $C_{1-10}$, O-alkyle en $C_{1-10}$, O-C(=O)alkyle en $C_{1-10}$, S-alkyle en $C_{1-10}$, NH(alkyle en $C_{1-10}$), N(alkyle en $C_{1-10})_2$, NH-C (=O)alkyle en $C_{1-10}$, N(C(=O) alkyle en $C_{1-10})_2$ où C(=O)alkyle en $C_{1-10}$, respectivement saturé ou insaturé, ramifié ou non ramifié, non substitué ou mono- ou polysubstitué ; cycloalkyle ou hétérocyclyle en $C_{3-8}$, respectivement saturé ou insaturé, non substitué ou mono- ou polysubstitué ;

$R^5$ représente H ; F ; Cl ; Br ; I ; $NO_2$ ; $CF_3$ ; CN ; $OCF_3$ ; SH ; $SCF_3$ ; $NH_2$ ; un groupe alkyle en $C_{1-10}$, O-alkyle en $C_{1-10}$, O-C(=O)alkyle en $C_{1-10}$, S-alkyle en $C_{1-10}$, NH(alkyle en $C_{1-10}$), N (alkyle en $C_{1-10})_2$, NH-C(=O) alkyle en $C_{1-10}$, N(C(=O) alkyle en $C_{1-10})_2$ ou C(=O)alkyle en $C_{1-10;}$ respectivement saturé ou insaturé, ramifié ou non ramifié, non substitué ou mono- ou polysubstitué ; cycloalkyle ou hétérocyclyle en $C_{3-8}$, respectivement saturé ou insaturé, non substitué ou mono- ou polysubstitué ;

Y représente O ou $NR^9$,

$R^9$ représentant H ou un groupe alkyle en $C_{1-4}$ saturé ou insaturé, ramifié ou non ramifié, non substitué ou mono- ou polysubstitué,

$R^7$ représente un groupe alkyle en $C_{1-10}$ ou hétéroalkyle en $C_{2-10}$, saturé ou insaturé, ramifié ou non ramifié, non substitué ou mono- ou polysubstitué ; cycloalkyle ou hétérocyclyle en $C_{3-10}$, respectivement saturé ou insaturé, non substitué ou mono- ou polysubstitué ; aryle ou hétéroaryle, respectivement non substitué ou mono- ou polysubstitué ;

à la condition que, si $R^7$ représente un groupe hétérocyclyle, la liaison du groupe hétérocyclyle à la structure générale subordonnée se fait par le biais d'un atome de carbone du groupe hétérocyclyle ; et

à la condition que, si $R^7$ représente aryle ou hétéroaryle, la somme de n et m est supérieure ou égale à 1 ;

$R^8$ est choisi dans le groupe constitué par les groupes alkyle en $C_{1-10}$ ou hétéroalkyle en $C_{2-10}$, respectivement saturé ou insaturé, ramifié ou non ramifié, non substitué ou mono- ou polysubstitué ; cycloalkyle ou hétérocyclyle en $C_{3-10}$, respectivement saturé ou insaturé, non substitué ou mono- ou polysubstitué ; aryle ou hétéroaryle, respectivement non substitué ou mono- ou polysubstitué ; cycloalkyle ou hétérocyclyle en $C_{3-10}$ ponté par le biais d'un groupe alkyle en $C_{1-8}$ ou hétéroalkyle en $C_{2-8}$, respectivement saturé ou insaturé, non substitué ou mono-ou polysubstitué ; ou aryle ou hétéroaryle ponté par le biais d'un groupe alkyle en $C_{1-8}$ ou hétéroalkyle en $C_{2-8}$, respectivement non substitué ou mono- ou polysubstitué, la chaîne alkyle ou hétéroalkyle pouvant être respectivement ramifiée ou non ramifiée, saturée ou insaturée, non substituée ;

les expressions « alkyle substitué », « hétéroalkyle substitué », « hétérocyclyle substitué » et « cycloalkyle substitué » représentant la substitution d'un ou de plusieurs atomes d'hydrogène, respectivement indépendamment les uns des autres, par F ; Cl ; Br ; I ; CN ; $CF_3$ ; =O ; =NH ; =$C(NH_2)_2$ ; $NO_2$ ; $R^0$ ; C(=O)H ; C(=O)$R^0$ ; $CO_2H$ ; C(=O)O$R^0$ ; $CONH_2$ ; C(=O)NH$R^0$ ; C(=O)N$(R^0)_2$ ; OH ; O$R^0$ ; -O-(alkyle en $C_{1-8}$)-O- ; O-C(=O)-$R^0$ ; O-

$C(=O)-O-R^0$ ; $O-(C=O)-NH-R^0$ ; $O-C(=O)-N(R^0)_2$ ; $O-S(=O)_2-R^0$ ; $O-S(=O)_2OH$ ; $OS(=O)_2OR^0$ ; $O-S(=O)_2NH_2$ ; $O-S(=O)_2NHR^0$ ; $O-S(=O)_2N(R^0)_2$ ; $NH_2$ ; $NH-R^0$ ; $N(R^0)_2$ ; $NH-C(=O)-R^0$ ; $NH-C(=O)-O-R^0$ ; $NH-C(=O)-NH_2$ ; $NH-C(=O)-NH-R^0$ ; $NH-C(=O)-N(R^0)_2$ ; $NR^0-C(=O)-R^0$ ; $NR^0-C(=O)-O-R^0$ ; $NR^0-C(=O)-NH_2$ ; $NR^0-C(=O)-NH-R^0$ ; $NR^0-C(=O)-N(R^0)_2$ ; $NH-S(=O)_2OH$ ; $NH-S(=O)_2R^0$ ; $NH-S(=O)_2OR^0$ ; $NH-S(=O)_2NH_2$ ; $NH-S(=O)_2NHR^0$ ; $NH-S(=O)_2N(R^0)_2$ ; $NR^0-S(=O)_2OH$ ; $NR^0-S(=O)_2R^0$ ; $NR^0-S(=O)_2OR^0$ ; $NR^0-S(=O)_2NH_2$ ; $NR^0-S(=O)_2NHR^0$ ; $NR^0-S(=O)_2N(R^0)_2$ ; SH ; $SR^0$ ; $S(=O)R^0$ ; $S(=O)_2R^0$ ; $S(=O)_2H$ ; $S(=O)_2OH$ ; $S(=O)_2OR^0$ ; $S(=O)_2NH_2$ ; $S(=O)_2NHR^0$ ; $S(=O)_2N(R^0)_2$ ;

les expressions « aryle substitué » et « hétéroaryle substitué » représentant la substitution d'un ou de plusieurs atomes d'hydrogène, respectivement indépendamment les uns des autres par F ; Cl ; Br ; I ; $NO_2$ ; $CF_3$ ; CN ; $R^0$ ; C(=O)H ; $C(=O)R^0$ ; $CO_2H$ ; $C(=O)OR^0$ ; $CONH_2$ ; $C(=O)NHR^0$ ; $C(=O)N(R^0)_2$ ; OH ; $OR^0$ ; -O-(alkyle en $C_{1-8}$)-O- ; $O-C(=O)-R^0$ ; $O-C(=O)-O-R^0$ ; $O-(C=O)-NH-R^0$ ; $O-C(=O)-N(R^0)_2$ ; $OS(=O)_2-R^0$ ; $O-S(=O)_2OH$ ; $O-S(=O)_2OR^0$ ; $O-S(=O)_2NH_2$ ; $OS(=O)_2NHR^0$ ; $O-S(=O)_2-N(R^0)_2$ ; $NH_2$ ; $NH-R^0$ ; $N(R^0)_2$ ; $NH-C(=O)-R^0$ ; $NH-C(=O)-O-R^0$ ; $NH-C(=O)-NH_2$ ; $NH-C(=O)-NH-R^0$ ; $NH-C(=O)-N(R^0)_2$ ; $NR^0-C(=O)-R^0$ ; $NR^0-C(=O)-O-R^0$ ; $NR^0-C(=O)-NH_2$ ; $NR^0-C(=O)-NH-R^0$ ; $NR^0-C(=O)-N(R^0)_2$ ; $NH-S(=O)_2OH$ ; $NH-S(=O)_2R^0$ ; $NH-S(=O)_2OR^0$ ; $NH-S(=O)_2NH_2$ ; $NH-S(=O)_2NHR^0$ ; $NH-S(=O)_2N(R^0)_2$ ; $NR^0-S(=O)_2OH$ ; $NR^0-S(=O)_2R^0$ ; $NR^0-S(=O)_2OR^0$ ; $NR^0-S(=O)_2NH_2$ ; $NR^0-S(=O)_2NHR^0$ ; $NR^0-S(=O)_2N(R^0)_2$ ; SH ; $SR^0$ ; $S(=O)R^0$ ; $S(=O)_2R^0$ ; $S(=O)_2OH$ ; $S(=O)_2OR^0$ ; $S(=O)_2NH_2$ ; $S(=O)_2NHR^0$ ; $S(=O)_2N(R^0)_2$ ; et

$R^0$ est choisi dans le groupe constitué par les groupes alkyle en $C_{1-10}$ ou hétéroalkyle en $C_{2-10}$, respectivement saturé ou insaturé, ramifié ou non ramifié, non substitué ou mono- ou polysubstitué ; cycloalkyle ou hétérocyclyle en $C_{3-10}$, respectivement saturé ou insaturé, non substitué ou mono- ou polysubstitué ; aryle ou hétéroaryle, respectivement non substitué ou mono- ou polysubstitué ; cycloalkyle ou hétérocyclyle en $C_{3-10}$ ponté par le biais d'un groupe alkyle en $C_{1-8}$ ou hétéroalkyle en $C_{2-8}$, respectivement saturé ou insaturé, non substitué ou mono-ou polysubstitué ; la chaîne alkyle ou hétéroalkyle pouvant être respectivement ramifiée ou non ramifiée, saturée ou insaturée, non substituée ou mono- ou polysubstituée ; ou aryle ou hétéroaryle ponté par le biais d'un groupe alkyle en $C_{1-8}$ ou hétéroalkyle en $C_{2-8}$, respectivement non substitué ou mono- ou polysubstitué, la chaîne alkyle ou hétéroalkyle pouvant être respectivement ramifiée ou non ramifiée,
saturée ou insaturée, non substituée ou mono- ou polysubstituée ;
sous la forme de composés libres ou de sels d'acides ou de bases physiologiquement acceptables.

2. Mercaptoquinoléines selon la revendication 1, **caractérisées en ce que** :

si $R^7$ représente un groupe hétérocyclyle, la liaison du groupe hétérocyclyle à la structure générale subordonnée se fait par le biais d'un atome de carbone du groupe hétérocyclyle.

3. Mercaptoquinoléines selon la revendication 1 ou 2, **caractérisées en ce que**, dans le cas de :

substituants « alkyle », « hétéroalkyle », « hétérocyclyle » et « cycloalkyle », ceux-ci sont choisis dans le groupe constitué par F ; Cl ; Br ; I ; $NO_2$ ; $CF_3$ ; CN ; =O ; alkyle en $C_{1-8}$ ; aryle ; hétéroaryle ; cycloalkyle en $C_{3-10}$ ; hétérocyclyle ; aryle, hétéroaryle, cycloalkyle ou hétérocyclyle en $C_{3-10}$ ponté par le biais d'un groupe alkyle en $C_{1-8}$ ; CHO ; C(=O)alkyle en $C_{1-8}$ ; C(=O)aryle ; C(=O)-hétéroaryle ; $CO_2H$ ; C(=O)O-alkyle en $C_{1-8}$ ; C(=O)O-aryle ; C(=O)O-hétéroaryle ; $CONH_2$ ; C(=O)NH-(alkyle en $C_{1-8}$) ; C(=O)N(alkyle en $C_{1-8}$)$_2$ ; C(=O)NH-aryle ; C(=O)N(aryle)$_2$ ; C(=O)NH-hétéroaryle ; C(=O)N(hétéroaryle)$_2$ ; C(=O)N(alkyle en $C_{1-8}$) (aryle) ; C(=O)N(alkyle en $C_{1-8}$) (hétéroaryle) ; C(=O)N(hétéroaryle)(aryle) ; OH ; O-alkyle en $C_{1-8}$ ; $OCF_3$ ; -O-(alkyle en $C_{1-8}$)-O- ; -O-(alkyl en $C_{1-8}$)-OH ; -O-(alkyl en $C_{-8}$)-O-alkyle en $C_{1-8}$ ; O-benzyle ; O-aryle ; O-hétéroaryle ; -O-C(=O)(alkyle en $C_{1-8}$) ; O-C(=O)aryle ; O-C(=O)hétéroaryle ; $NH_2$ ; NH-alkyle en $C_{1-8}$ ; N(alkyle en $C_{1-8}$)$_2$ ; NH-C(=O)alkyle en $C_{1-8}$ ; NH-C(=O)aryle ; NH-C(=O)-hétéroaryle ; SH ; S-alkyle en $C_{1-8}$ ; $SCF_3$ ; S-benzyle, S-aryle, S-hétéroaryle ; $S(=O)_2$alkyle en $C_{1-8}$ ; $S(=O)_2$aryle ; $S(=O)_2$hétéroaryle ; $S(=O)_2OH$ ; $S(=O)_2O$-alkyle en $C_{1-8}$ ; $S(=O)_2O$-aryle ; $S(=O)_2O$-hétéroaryle ; $S(=O)_2$-NH-alkyle en $C_{1-8}$ ; $S(=O)_2$-NH-aryle ; et $S(=O)_2$-NH-hétéroaryle en $C_{1-8}$, et substituants « aryle » et « hétéroaryle », ceux-ci sont choisis dans le groupe constitué par F ; Cl ; Br ; I ; $NO_2$ ; $CF_3$ ; CN ; alkyle en $C_{1-8}$ ; aryle ; hétéroaryle ; cycloalkyle en $C_{310}$ ; hétérocyclyle ; aryle, hétéroaryle, cycloalkyle en $C_{3-10}$ ou hétérocyclyle ponté par le biais d'un groupe alkyle en $C_{1-8}$ ; CHO ; C(=O)alkyle en $C_{1-8}$ ; C(=O)aryle ; C(=O)hétéroaryle ; $CO_2H$ ; C(=O)O-alkyle en $C_{18}$ ; C(=O)O-aryle ; C(=O)O-hétéroaryle ; $CONH_2$ ; C(=O)NH-(alkyle en $C_{1-8}$) ; C(=O)N(alkyle en $C_{1-8}$)$_2$ ; C(=O)NH-aryle ; C(=O)N(aryle)$_2$ ; C(=O)NH-hétéroaryle ; C(=O)N(hétéroazyle)$_2$ ; C(=O)N(alkyle en $C_{1-8}$)(aryle) ; C(=O)N(alkyle en $C_{1-8}$)-(hétéroaryle) ; C(=O)N(hétéroaryle)(aryle) ; OH ; O-alkyle en $C_{1-8}$ ; $OCF_3$ ; -O-(alkyl en $C_{1-8}$)-O- ; -O-(alkyl en $C_{1-8}$)-OH ; -O-(alkyle en $C_{1-8}$)-O-alkyle en $C_{1-8}$ ; O-benzyle ; O-aryle ; O-hétéroaryle ; -O-C(=O)(alkyle en $C_{1-8}$) ; OC(=O)aryle ; O-C(=O)hétéroaryle ; $NH_2$ ; NH-alkyle en $C_{1-8}$ ; N(alkyle en $C_{1-8}$)$_2$ ; NH-C(=O)alkyle en $C_{1-8}$ ; NH-C(=O)aryle ; NH-C(=O)-hétéroaryle ; SH ; S-alkyle en $C_{1-8}$ ; $SCF_3$ ; S-benzyle, S-aryle, S-hétéroaryle ; $S(=O)_2$alkyle en $C_{1-8}$ ;

$S(=O)_2$aryle ; $S(=O)_2$hétéroaryle ; $S(=O)_2OH$ ; $S(=O)_2$O-alkyle en $C_{1-8}$ ; $S(=O)_2$O-aryle ; $S(=O)_2$O-hétéroaryle ; $S(=O)_2$-NH-alkyle en $C_{1-8}$ ; $S(=O)_2$-NH-aryle ; $S(=O)_2$-NH-hétéroaryle en $C_{1-8}$.

**4.** Mercaptoquinoléines selon l'une des revendications précédentes, **caractérisées en ce que** :

$R^1$, $R^2$, $R^3$ et $R^4$ sont choisis respectivement indépendamment les uns des autres dans le groupe constitué par H ; F ; Cl ; Br ; I ; $NO_2$ ; $CF_3$ ; CN ; OH ; $OCF_3$ ; SH ; $SCF_3$ ; $NH_2$ ; alkyle en $C_{1-6}$, O-alkyle en $C_{1-6}$, O-C(=O) alkyle en $C_{1-6}$, S-alkyle en $C_{1-6}$, NH (alkyle en $C_{1-6}$), N (alkyle en $C_{1-6})_2$, NH-C(=O)alkyle en $C_{1-6}$, N(C(=O)alkyle en $C_{1-6})_2$ ou C(=O)alkyle en $C_{1-6}$, respectivement saturé ou insaturé, ramifié ou non ramifié, non substitué ou mono- ou polysubstitué par un ou plusieurs substituants choisis indépendamment les uns des autres dans le groupe constitué par H ; F ; Cl ; Br ; I ; $NO_2$ ; $CF_3$ ; CN ; OH ; $OCF_3$ ; SH ; $SCF_3$ ; $NH_2$ ; alkyle en $C_{1-6}$, O-alkyle en $C_{1-6}$, O-C(=O)alkyle en $C_{1-6}$, S-alkyle en $C_{1-6}$, NH (alkyle en $C_{1-6}$), N (alkyle en $C_{1-6})_2$, NH-C(=O)alkyle en $C_{1-6}$, N(C(=O)alkyle en $C_{1-6})_2$ ou C(=O)alkyle en $C_{1-6}$ ; cycloalkyle ou hétérocyclyle en $C_{3-6}$, respectivement saturé ou insaturé, non substitué ou mono-ou polysubstitué par un ou plusieurs substituants choisis indépendamment les uns des autres dans le groupe constitué par H ; F ; Cl ; Br ; I ; $NO_2$ ; $CF_3$ ; CN ; OH ; $OCF_3$ ; SH ; $SCF_3$ ; $NH_2$ ; alkyle en $C_{1-6}$, O-alkyle en $C_{1-6}$, O-C(=O)alkyle en $C_{1-6}$, S-alkyle en $C_{1-6}$, NH(alkyle en $C_{1-6}$), N (alkyle en $C_{1-6})_2$, NH-C(=O)alkyle en $C_{1-6}$, N(C(=O)alkyle en $C_{1-6})_2$ ou C(=O)alkyle en $C_{1-6}$ ;
et
$R^5$ est choisi dans le groupe constitué par H ; F ; Cl ; Br ; I ; $NO_2$ ; $CF_3$ ; CN ; $OCF_3$ ; SH ; $SCF_3$ ; $NH_2$ ; alkyle en $C_{1-6}$, O-alkyle en $C_{1-6}$, O-C(=O)alkyle en $C_{1-6}$, S-alkyle en $C_{1-6}$, NH(alkyle en $C_{1-6}$), N(alkyle en $C_{1-6})_2$, NH-C(=O)alkyle en $C_{1-6}$, N(C(=O)alkyle en $C_{1-6})_2$ et C(=O)alkyle en $C_{1-6}$, respectivement saturé ou insaturé, ramifié ou non ramifié, non substitué ou mono- ou polysubstitué par un ou plusieurs substituants choisis indépendamment les uns des autres dans le groupe constitué par H ; F ; Cl ; Br ; I ; $NO_2$ ; $CF_3$ ; CN ; OH ; $OCF_3$ ; SH ; $SCF_3$ ; $NH_2$ ; alkyle en $C_{1-6}$, O-alkyle en $C_{1-6}$, O-C(=O)alkyle en $C_{1-6}$, S-alkyle en $C_{1-6}$, NH(alkyle en $C_{1-6}$), N (alkyle en $C_{1-6})_2$, NH-C(=O)alkyle en $C_{1-6}$, N(C(=O)alkyle en $C_{1-6})_2$ et C(=O)alkyle en $C_{1-6}$ ; cycloalkyle ou hétérocyclyle en $C_{3-6}$, respectivement saturé ou insaturé, ramifié ou non ramifié, non substitué ou mono-ou polysubstitué par un ou plusieurs substituants choisis indépendamment les uns des autres dans le groupe constitué par H ; F ; Cl ; Br ; I ; $NO_2$ ; $CF_3$ ; CN ; OH ; $OCF_3$ ; SH ; $SCF_3$ ; $NH_2$ ; alkyle en $C_{1-6}$, O-alkyle en $C_{1-6}$, O-C(=O)alkyle en $C_{1-6}$, S-alkyle en $C_{1-6}$, NH(alkyle en $C_{1-6}$), N(alkyle en $C_{1-6})_2$, NH-C(=O)alkyle en $C_{1-6}$, N(C(=O)alkyle en $C_{1-6})_2$ et C(=O)alkyle en $C_{1-6}$.

**5.** Mercaptoquinoléines selon l'une des revendications précédentes, **caractérisées en ce que** :

$R^1$, $R^2$, $R^3$ et $R^4$ représentent respectivement indépendamment les uns des autres H ; F ; Cl ; CN ; $OCF_3$ ; $SCF_3$ ; $CF_3$ ; $CH_3$ ou $OCH_3$ ; et
$R^5$ représente H, F, Cl, $OCF_3$, $SCF_3$, un groupe alkyle en $C_{1-6}$, O-alkyle en $C_{1-6}$ ou S-alkyle en $C_{1-6}$.

**6.** Mercaptoquinoléines selon l'une des revendications précédentes, **caractérisées en ce que** :

$R^{6a}$ et $R^{6b}$ représentent, respectivement indépendamment les uns des autres, H ; F ; Cl ; Br ; I ; un groupe méthyle ; éthyle ; n-propyle ; isopropyle ; n-butyle ; sec.-butyle ; tertiobutyle ; OH ; O-méthyle ou O-éthyle.

**7.** Mercaptoquinoléines selon l'une des revendications précédentes, **caractérisées en ce que** :

$R^7$ représente un groupe alkyle en $C_{2-6}$ ou hétéroalkyle en $C_{2-6}$, saturé ou insaturé ; ramifié ou non ramifié, non substitué ; cycloalkyle ou hétérocyclyle en $C_{3-8}$, saturé ou insaturé, non substitué ; phényle, furyle, thiényle ou pyridyle, respectivement non substitué ou mono- ou polysubstitué par un ou plusieurs substituants choisis indépendamment les uns des autres dans le groupe constitué par F, Cl, Br, I, CN ; $CF_3$, $OCF_3$, $SCF_3$, $CH_3$ et $OCH_3$.

**8.** Mercaptoquinoléines selon l'une des revendications précédentes, **caractérisées en ce que** :

m représente 0, n représente 1, et $R^7$ représente un groupe aryle ou hétéroaryle, respectivement non substitué ou mono- ou polysubstitué.

**9.** Mercaptoquinoléines selon l'une des revendications précédentes, **caractérisées en ce que** :

m représente 0, n représente 1 ou 2, et $R^7$ représente un groupe alkyle en $C_{1-10}$ ou hétéroalkyle en $C_{2-10}$, saturé

ou insaturé ; ramifié ou non ramifié, non substitué ou mono-ou polysubstitué ; cycloalkyle ou hétérocyclyle en $C_{3-10}$, saturé ou insaturé, non substitué ou mono- ou polysubstitué.

**10.** Mercaptoquinoléines selon l'une des revendications précédentes, **caractérisées en ce que** :

$R^8$ est choisi dans le groupe constitué par les groupes alkyle en $C_{1-8}$ ou hétéroalkyle en $C_{2-8}$, respectivement saturé ou insaturé, ramifié ou non ramifié, non substitué ou mono-ou polysubstitué par un ou plusieurs substituants choisis indépendamment les uns des autres dans le groupe constitué par F, Cl, Br, I, OH, =O, $OCF_3$, $SCF_3$, $CF_3$, alkyle en $C_{1-4}$ et O-alkyle en $C_{1-4}$ ; cycloalkyle ou hétérocyclyle en $C_{3-10}$, respectivement saturé ou insaturé, non substitué ou mono-ou polysubstitué par un ou plusieurs substituants choisis indépendamment les uns des autres dans le groupe constitué par F, Cl, Br, I, OH, =O, $OCF_3$, $SCF_3$, $CF_3$, alkyle en $C_{1-4}$ et O-alkyle en $C_{1-4}$ ; aryle ou hétéroaryle, respectivement non substitué ou mono- ou polysubstitué par un ou plusieurs substituants choisis indépendamment les uns des autres dans le groupe constitué par H ; F ; Cl ; Br ; I ; $NO_2$ ; $CF_3$ ; CN ; OH ; $OCF_3$ ; SH ; $SCF_3$ ; $NH_2$ ; alkyle en $C_{1-6}$, O-alkyle en $C_{1-6}$, O-C(=O)alkyle en $C_{1-6}$, S-alkyle en $C_{1-6}$, NH(alkyle en $C_{1-6}$), N(alkyle en $C_{1-6}$)$_2$, NH-C(=O)alkyle en $C_{1-6}$, N(C(=O)-alkyle en $C_{1-6}$)$_2$ ou C(=O)alkyle en $C_{1-6}$; aryle ou hétéroaryle, respectivement non substitué ou mono- ou polysubstitué par un ou plusieurs substituants choisis indépendamment les uns des autres dans le groupe constitué par H ; F ; Cl ; Br ; I ; $NO_2$ ; $CF_3$ ; CN ; OH ; $OCF_3$ ; SH ; $SCF_3$ ; $NH_2$ ; alkyle en $C_{1-6}$, O-alkyle en $C_{1-6}$, O-C(=O)alkyle en $C_{1-6}$, S-alkyle en $C_{1-6}$, NH (alkyle en $C_{1-6}$), N (alkyle en $C_{1-6}$)$_2$, NH-C(=O)alkyle en $C_{1-6}$, N(C(=O)alkyle en $C_{1-6}$)$_2$ ou C(=O)alkyle en $C_{1-6}$ ; cycloalkyle ou hétérocyclyle en $C_{3-10}$ ponté par un groupe alkyle en $C_{1-8}$ ou hétéroalkyle en $C_{2-8}$, respectivement saturé ou insaturé, non substitué ou mono-ou polysubstitué par un ou plusieurs substituants choisis indépendamment les uns des autres dans le groupe constitué par F, Cl, Br, I, OH, =O, $OCF_3$, $SCF_3$, $CF_3$, alkyle en $C_{1-8}$ et O-alkyle en $C_{1-8}$, la chaîne alkyle ou hétéroalkyle pouvant être respectivement ramifiée ou non ramifiée, saturé ou insaturée, non substituée ; ou aryle ou hétéroaryle ponté par un groupe alkyle en $C_{1-8}$ ou hétéroalkyle en $C_{2-8}$, respectivement saturé ou insaturé, non substitué ou mono-ou polysubstitué par un ou plusieurs substituants choisis indépendamment les uns des autres dans le groupe constitué par F, Cl, Br, I, OH, =O, $OCF_3$, $SCF_3$, $CF_3$, alkyle en $C_{1-8}$ et O-alkyle en $C_{1-8}$, la chaîne alkyle ou hétéroalkyle pouvant être respectivement ramifiée ou non ramifiée, saturé ou insaturée, non substituée.

**11.** Mercaptoquinoléines selon la revendication 1, choisies dans le groupe constitué par

1 2-cyclohexyl-N-(2-(2-(phénylsulfonyl)éthylthio)-quinoléine-3-yl)acétamide ;
2 N-(2-(2-(phénylsulfonyl)éthylthio)quinoléine-3-yl)-2-(thiophène-2-yl)acétamide ;
4 N-(2-(pentylthio)-quinoléine-3-yl)-2-(thiophène-2-yl)acétamide ;
6 N-(2-(2-(phénylthio)éthylthio)quinoléine-3-yl)-2-(thiophène-2-yl)acétamide ;
7 N-(2-(éthylthio)quinoléine-3-yl)-2-(thiophène-2-yl)acétamide ;
9 N-(2-(éthylthio)quinoléine-3-yl)-3,3-diméthyl-butanamide ;
11 N-[2-éthylsulfanyl-7-(trifluorométhyl)quinoléine-3-yl]-3,3-diméthylbutyramide ;
12 3-cyclopentyl-N-[2-éthylsulfanyl-7-(trifluorométhyl)-quinoléine-3-yl]propionamide
14 2-(5-bicyclo[2.2.1]heptanyl)-N-(2-éthylsulfanyl-quinoléine-3-yl)acétamide
15 3-cyclopentyl-N-(2-éthylsulfanyl-quinoléine-3-yl)-propionamide
16 2-(5-bicyclo[2.2.1]heptanyl)-N-[2-éthylsulfanyl-7-(trifluorométhyl)quinoléine-3-yl)acétamide
17 3-cyclopentyl-N-[2-éthylsulfanyl-4-méthyl-7-(trifluorométhyl)-quinoléine-3-yl]propionamide
18 N-[2-éthylsulfanyl-4-méthyl-7-(trifluorométhyl)-quinoléine-3-yl]-2-thiophène-2-yl-acétamide ;

ou leurs sels physiologiquement acceptables.

**12.** Médicament contenant au moins une 3-amino-2-mercaptoquinoléine selon l'une des revendications 1 à 11, sous la forme d'un stéréoisomère individuel ou de leur mélange, des composés libres et/ou de leurs sels physiologiquement acceptables, ainsi que les additifs et/ou auxiliaires le cas échéant appropriés et/ou éventuellement d'autres principes actifs.

**13.** Utilisation d'au moins une 3-amino-2-mercapto-quinoléine selon l'une des revendications 1 à 11, sous la forme d'un stéréoisomère individuel ou de leur mélange, du composé libre et/ou de leurs sels physiologiquement acceptables, pour la préparation d'un médicament pour le traitement de la douleur, de l'épilepsie, des états anxieux, de la dépendance, de la manie, des troubles bipolaires, de la migraine, des maladies cognitives, des dyskinésies associées à la dystonie et/ou de l'incontinence urinaire.

**14.** 3-amino-2-mercaptoquinoléine substituée selon l'une des revendications 1 à 11, sous la forme d'un stéréoisomère individuel ou de leur mélange, du composé libre et/ou de leurs sels physiologiquement acceptables, pour l'utilisation dans le traitement de la douleur, de l'épilepsie, des états anxieux, de la dépendance, de la manie, des troubles bipolaires, de la migraine, des maladies cognitives, des dyskinésies associées à la dystonie et/ou de l'incontinence urinaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20020128277 A
- WO 2008046582 A
- WO 2007057447 A


### In der Beschreibung aufgeführte Nicht-Patentliteratur


- **PASSMORE et al.** *J Neurosci.,* 2003, vol. 23 (18), 7227-36
- **BLACKBURN-MUNRO ; JENSEN.** *Eur J Pharmacol.,* 2003, vol. 460 (2-3), 109-16
- **DOST et al.** *Naunyn Schmiedebergs Arch Pharmacol,* 2004, vol. 369 (4), 382-390
- **NIELSEN et al.** *Eur J Pharmacol.,* 2004, vol. 487 (1-3), 93-103
- **GRIBKOFF.** *Expert Opin Ther Targets,* 2003, vol. 7 (6), 737-748
- **KORSGAARD et al.** *J Pharmacol Exp Ther.,* 2005, vol. 14 (1), 282-92
- **WICKENDEN et al.** *Expert Opin Ther Pat,* 2004, vol. 14 (4), 457-469
- **GRIBKOFF.** *Expert Opin Ther Targets,* 2008, vol. 12 (5), 565-81
- **MICELI et al.** *Curr Opin Pharmacol,* 2008, vol. 8 (1), 65-74
- **STRENG et al.** *J Urol,* 2004, vol. 172, 2054-2058
- **HANSEN et al.** *Eur J Pharmacol,* 2007, vol. 570 (1-3), 77-88
- **DENCKER et al.** *Epilepsy Behav,* 2008, vol. 12 (1), 49-53
- **RICHTER et al.** *Br J Pharmacol,* 2006, vol. 149 (6), 747-53
- **BENNETT, G.J. ; XIE, Y.K.** A peripheral mononeuropathy in rat that produces disorders of pain sensation like those seen in man. *Pain,* 1988, vol. 33 (1), 87-107
- **KIM, S.H. ; CHUNG, J.M.** An experimental model for peripheral neuropathy produced by segmental spinal nerve ligation in the rat. *Pain,* 1992, vol. 50 (3), 355-363
- **DE SARRO et al.** *Naunyn-Schmiedeberg's Arch. Pharmacoi.,* 2001, vol. 363, 330-336
- **J. MARCH.** Advanced Organic Chemistry. Wiley & Sons, 2007
- **F. A. CAREY ; R. J. SUNDBERG.** Advanced Organic Chemistry, Parts A and B. Springer, 2007
- Autorenkollektiv. Compendium of Organic Synthetic Methods. Wiley & Sons
- **DUBUISSON, D. ; DENNIS, S.G.** *Pain,* 1977, vol. 4, 161-174
- **LITCHFIELD, J.T. ; WILCOXON, J.J.** *J. Pharmacol. Exp. Ther.,* 1949, vol. 96, 99-113